# EUROPEAN PATENT APPLICATION

(11) **EP 3 045 540 A1**
(43) Date of publication of application: **20.07.2016**
(21) Application number: 15000132.9
(22) Date of filing: 19.01.2015
(51) Int. Cl.: C12P 19/56, C07H 15/252

(54) **Enzymatic process for the regioselective manufacturing of N-Fmoc-doxorubicin-14-O-dicarboxylic acid mono esters**

(71) Applicant: Æterna Zentaris GmbH, 60314 Frankfurt am Main (DE)
(72) Inventor: Schuster, Tilmann, D-63762 Großostheim (DE); Gerlach, Matthias, D-63636 Brachttal (DE); Schuch, Falk, D-61194 Niddatal (DE)

(57) **Abstract**

The present invention describes a process for the selective synthesis of *N*-Fmoc-doxorubicin-14-*O*-dicarboxylic acid mono ester derivatives starting from *N*-Fmoc-doxorubicin using lipase enzymes and bis-acyl donor compounds such as dicarboxylic acids or anhydrides.

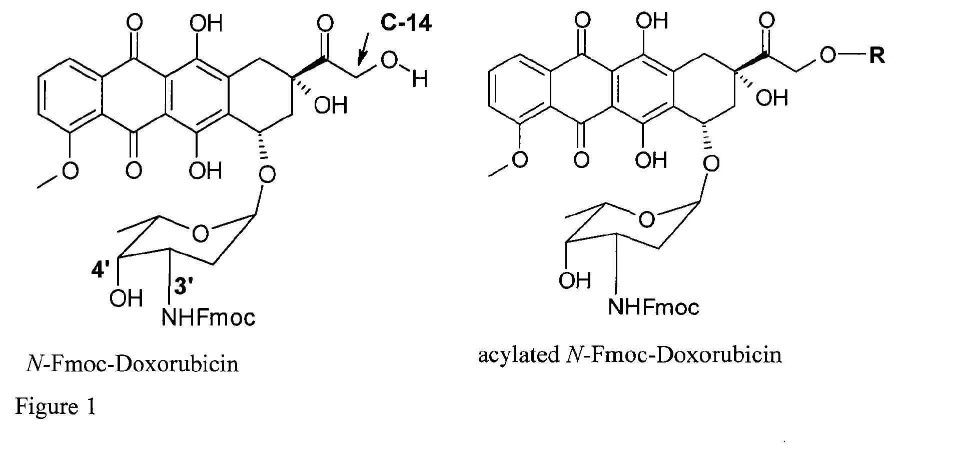

## Description

### Technical field

The present invention describes a process for the use of enzymes for the selective synthesis of *N*-Fmoc-doxorubicin-14-*O*-dicarboxylic acid mono ester derivatives using bis-acyl donor compounds such as dicarboxylic acids or anhydrides.

### Prior Art

Starting from *N*-Fmoc-doxorubicin, the chemical acylation to form *N*-Fmoc-doxorubicin-14-O-hemiglutarate as intermediate in the synthesis of LHRH analogs containing doxorubicin is described by Nagy et al. (J. Proc. Natl. Acad. Sci. USA 1996, 93, 7269).

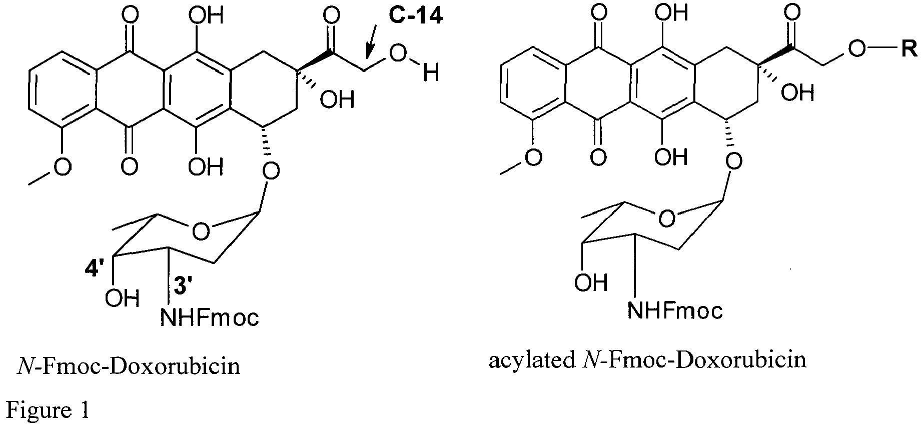

Addition of glutaric anhydride to *N*-Fmoc-doxorubicin (obtained from C3'-NH₂-doxorubicin) in anhydrous DMF in the presence of DIPEA overnight yielded a crude product, which by HPLC analyses showed ∼75% of the desired hemiglutarate, 18% of unreacted *N*-Fmoc-doxorubicin and 7% other impurities, including 4% attributed to the 4'-OH-hemiglutarate. HPLC purification resulted in a yield of 64% hemiglutarate.

The low solubility of *N*-Fmoc-doxorubicin hemiglutarate was identified as a problem and used as explanation, why the hemiglutarate was usually not isolated, but directly converted to the final product.
Corresponding data are disclosed in US5843903, filed by Schally, Nagy and Cai.

In an overall judgment, these results were experimentally confirmed as shown by reference experiment 1.

### Reference experiment 1: Synthesis of N-Fmoc-doxorubicin-O-hemiglutarate using glutaric anhydride catalyzed by DIPEA as described by Nagy et al. (J. Proc. Natl. Acad. Sci. USA 1996,93,7269)

*N*-Fmoc-doxorubicin (13.46 g), DIPEA (4.54 g) and glutaric anhydride (8.02g) were dissolved in DMF (128 mL). The reaction mixture was stirred for 7 h at room temperature. The progress of the reaction was monitored by HPLC. The reaction mixture was cooled to 15 °C and added into 662 mL of a aqueous acetic acid solution (w/w = 5 %). The product is filtered, washed with aqueous acetic acid solution (w/w = 5 %) (142 mL) and dried under vacuum to constant weight at 50 °C to give crude *N*-Fmoc-doxorubicin-14-*O*-hemiglutarate.

Yield: 15.2 g (95 %)
Purity: HPLC: 51.8 % (Impurity profile - see Appendix 1).

HPLC analysis of isolated, crude hemiglutarate showed 51.8% *N*-Fmoc-doxorubicin-14-O-hemiglutarate, 1.3% of unreacted *N*-Fmoc-doxorubicin and 11.1% of the 4'-*O*-, 14-*O-*dihemiglutarate.

While not surprising, an analogous experiment without base resulted in nearly no conversion.

### Reference experiment 2: Synthesis of N-Fmoc-doxorubicin-O-hemiglutarate using glutaric anhydride without base

The same reaction conditions as used in Example 1 were applied for the acylation without DIPEA. The reaction was slow and 64 % *N*-Fmoc-doxorubicin remained unreacted after overnight stirring. Therefore no work-up was performed.
Yield: not isolated, Purity: n.a.

### Selective acylation of the C14-position of doxorubicin derivatives using enzymes

The utility of enzymes achieving the selective modification of structurally complex substrates under mild conditions is also well documented (Wong et al., Enzymes in Synthetic Organic Chemistry, 1st ed. Pergamon: Tarrytown, NY, Oxford, U.K., 1994).

Cotterill et al. (Organic Process Research & Development 2005, 9, 818) reported the use of lipases for the regioselective esterification of the C14-hydroxy group of *N*-trifluoroacetyl-doxorubicin with a series of (mono) carboxylic acids.
Furthermore, it is reported that,
- it has been necessary to protect the C3'-NH₂ group in order to regioselectively acylate the C 14-OH group, since the C3'-NH₂ moiety and the C4'-OH group of doxorubicin are known to be chemical more reactive than the C14-OH group
- the removal of water is essential in order to allow the reaction to complete
- the selectivity of acylation (C14-OH vs C4'-OH) is clearly dependent upon the reaction solvent
- the solubility of *N*-trifluoroacetyl-doxorubicin in the reaction solvents is a key prerequisite for production of gram quantities
- the best conditions to conduct the reaction were found to be at 58°C in 8h in a 3:1 MTBE/2-butanone mixture to give the desired *N-*trifluoroacetyl-doxorubicin valerate in 94% yield, including 4% byproducts.

These teachings are confirmed by reference experiments 3 and 4.
Reference experiment 3 shows, that the lipase is essential for the process of adding glutaric acid to *N*-Fmoc-doxorubicin, as otherwise *N*-Fmoc-doxorubicin remains unreacted. Reference experiment 4 shows, that protection of the C3'-NH₂ group is essential as otherwise the yield of the desired doxorubicin hemiglutarate is only 14% (as found by HPLC analysis), while starting material doxorubicin remains to 7%. The main product (47%) was not structurally identified.

### Reference experiment 3: Synthesis of N-Fmoc-doxorubicin-O-hemiglutarate using glutaric acid without lipase

*N*-Fmoc-doxorubicin (50 mg) was suspended in MTBE/MEK 3:1 (v/v) (10 mL) and to this was added glutaric acid (82 mg). The reaction flask was fitted with a dropping funnel and condenser assembly in which the funnel was filled with 3 Å molecular sieves (ca. 10 g). The reaction mixture was stirred vigorously and heated for 25 h at reflux temperature. The progress of the reaction was monitored by HPLC. No product could be detected.
Yield: not isolated
Purity: product not detectable (HPLC - see Appendix 2)

### Reference experiment 4: Synthesis of doxorubicin-O-hemiglutarate using glutaric acid as bis-acyl donor under lipase conditions

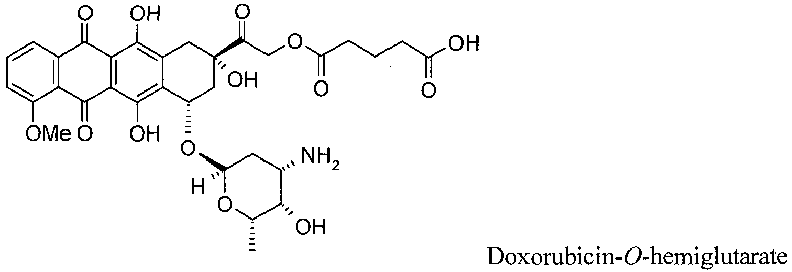

Doxorubicin (50 mg) was suspended in MTBE/MEK 3:1 (v/v) (10 mL) and to this was added lipase acrylic resin from *Candida Antarctica B* (233 mg, 10500 units/g resin) and glutaric acid (109 mg). The reaction flask was fitted with a dropping funnel and condenser assembly in which the dropping funnel (but not the pressure equalizing tube) was filled with 3 Å molecular sieves (ca. 10 g). The reaction mixture was stirred vigorously and heated for 25 h at reflux temperature. The progress of the reaction was monitored by HPLC. Only less than 15 % of product (doxorubicin-*O*-hemiglutarate) beside other reaction products could be detected via HPLC. Therefore no work-up was performed.
Yield: not isolated
Purity: 14.1 % (HPLC- see Appendix 3)

Michels, Rich, Meckler and Coterrill filed a patent application (WO03/057896) disclosing a process for preparation of doxorubicin derivatives, which may carry *N*-alkylated amino sugar or a non-basic sugar moieties, and which are acylated via non-chemically modified lipases on the C14-OH group. A broad range of solvents, non-activated and activated acyl donors as well as lipases are described to be applicable within the claimed process. Process examples either start from *N-*trifluoroacetyl-doxorubicin or from *N*-alloc-doxorubicin and use two lipases (PS30 and Chirazym L2, C3) to acylate to the C14-butyrate, -valerate and a PEP derivative, respectively.
To overcome the low solubility of anthracycline derivatives containing a free amine, conversion to *N*-alloc doxorubicin is proposed, which is disclosed as "highly soluble in less polar organic solvents suitable for synthesis".

Cotterill et al. (Biotech and Bioeng. 2008, 101, 435) reported a three step synthesis on unprotected doxorubicin-14-*O*-esters using lipases starting from doxorubicin HCl salt. The low solubility of unprotected doxorubicin is described as the key problem to be solved for efficient synthesis of doxorubicin-14-*O*-esters.
The alloc group was identified as a modifying agent for the synthesis of doxorubicin C14 esters due to the improved solubility of *N*-alloc doxorubicin in a variety of process solvents and the facile removal of the alloc group to afford the desired products.
With regard to regioselectivity, the introduction of small protecting groups, such as Boc or alloc, onto the doxorubicin-3'-NH₂ group did not provide sufficient steric hindrance to prevent acylation of the C4'-OH. The introduction of larger protection groups (e.g. *N-*Fmoc) is described to partially influence the acylation of the adjacent C4'-OH, but due to the poor solubility of *N*-Fmoc-doxorubicin it is stated to be an unpractical approach.
Since the chosen alloc group did not provide for specific C14 esterification, selectivity was reported to be achieved by carefully selecting the binary solvent system. While one solvent was chosen with respect to maximizing solubilization of *N*-alloc doxorubicin, the other solvent was selected in order to maintain catalytic activity and doxorubicin-C14 selectivity.

Summarizing the prior art, it was known, that for a C14-OH esterification of doxorubicin, its C3'-NH₂ group needs to be modified, as otherwise no clean esterification occurs. Said C3'- NH₂ group should be modified preferable by a bulky protection group that can shield the C4'-OH group to prevent its undesired esterification. The *N*-trifluoroacetyl- and the *N*-alloc groups were used to modify the C3'-NH₂ group. However, the *N*-Fmoc protecting group was clearly stated as unsuited due to the poor solubility of *N*-Fmoc-doxorubicin.

### Summary of the Invention

It has surprisingly been found, that *N*-Fmoc-doxorubicin can indeed be used as starting material in enzyme catalyzed acylation reactions with very good yield and high selectivity for C 14-OH esterification, despite its low solubility. The present inventions therefore provides a solution to a problem clearly identified in the prior art for esterification of *N*-Fmoc-doxorubicin, being specifically expressed by the statement "the solubility of *N*-Fmoc-doxorubicin was ... not synthetically useful" (see Cotterill et al. Biotech and Bioeng. 2008, p 438, left column, 1^{st} paragraph).

The use of the *N*-Fmoc protecting group to modify the doxorubicin C3'-NH₂ group is advantageous compared to other commonly used nitrogen modifying groups, because this bulky group shields the doxorubicin C4'-OH group and thus provides due to enhanced selectivity for better yields in shorter reaction times, combined with a better impurity profile.

The lipase catalyzed esterification of *N*-alloc-doxorubicin or *N*-trifluoroacetyl-doxorubicin with glutaric acid yielded 51 % and 70% of the corresponding products, respectively (see reference experiments 5 and 6). Under the same conditions, *N*-Fmoc-doxorubicin was converted to *N*-Fmoc-doxorubicin hemiglutarate in 92% yield (see example 1a).
Further optimization resulted in yields up to 95%, while reducing the reaction time to 4h (see example 1d). This was achieved by organic binary solvent systems, wherein one component provided the possibility to remove water from the reaction vessel in order to drive the reaction to completion, while the other component ensured lipase activity and a sufficient solubility of the starting materials. The need for a solvent to direct regioselective esterification was overcome due to the directing effects of the bulky Fmoc group.

### Description of the invention

The present invention relates to a process for preparation of an *N*-Fmoc-doxorabicin-14-*O-*dicarboxylic acid mono ester compound according to formula (I) or a pharmaceutically acceptable salt thereof, wherein in formula (I) R is a dicarboxylic group: 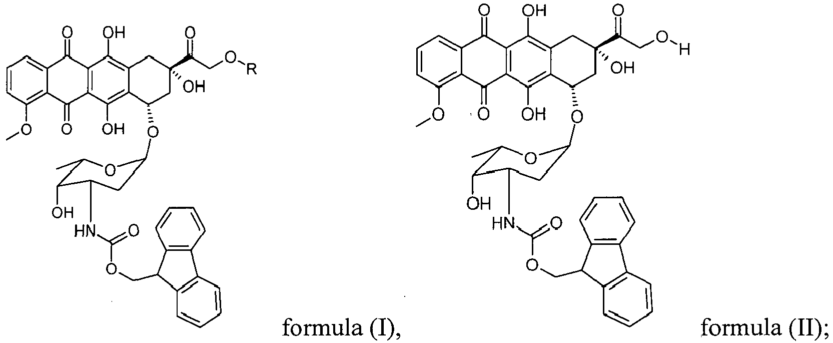 said process comprising reacting *N*-Fmoc-doxorubicin (formula II) with a bis-acyl donor compound in the presence of a lipase in liquid organic media to acylate said *N*-Fmoc-doxorubicin with high selectivity at its C14-OH group, under conditions effective to remove water from the reaction mixture.

The reaction of the present invention is carried out in liquid organic media. Possible liquid organic media include but are not limited to organic solvents of a group (A) selected from dichloromethane, chloroform, tetrachloromethane, benzene, toluene, diethyl ether, methyl-*tert*-butyl ether ("MTBE"), diisopropyl ether, cyclopentyl methyl ether ("CPME"), acetone, methyl-ethylketone ("MEK"), methyl-isobutylketone ("MIBK"), anisole (PhOMe), dimethylsulfoxide ("DMSO"), *N*-methyl-2-pyrrolidone ("NMP"), 1,4-dioxanee, pyridine, acetonitrile ("MeCN"), *N*,*N-*dimethyl formamide ("DMF"), tetrahydrofuran ("THF"), 2-methyltetrahydrofuran ("Me-THF"), ethyl acetate ("EtOAc"), *n*-pentane ("pent"), *n*-hexane ("hex"), *n*-heptane ("hept"), 2,2,4-trimethylpentane ("isooctane"), cyclopentane, cyclohexane, cycloheptane, *tert*-butanol ("tBuOH"), *tert*-amyl alcohol ("tAmyl-OH").

Liquid organic media include mixtures of two organic solvents, wherein one solvent is chosen to facilitate azeotropic distillation, i.e. the removal of water from the reaction vessel by azeotropic distillation in order to drive the reaction to completion. The other solvent is chosen to ensure lipase activity and a sufficient solubility of the starting materials. Solvents facilitating azeotropic distillation include, but are not limited to solvents from a group (B) selected from methyl-*tert*-butyl ether, toluene, benzene, cyclopentyl methyl ether, acetonitrile, tetrahydrofuran, 2-methyltetrahydrofuran, methyl-ethylketone, ethyl acetate, 1,4-dioxanee, *n*-hexane, *n*-heptane, *n*-pentane, or 2,2,4-trimethyl pentane.
The use of other solvents being also able to facilitate azeotropic distillation is considered analogous to the mentioned solvents and is therefore included into the process of the present application.
Solvents to ensure lipase activity and a sufficient solubility of the starting materials include, but are not limited to solvents from a group (C) selected from methyl-ethylketone, acetone, cyclopentyl methyl ether, anisole, tetrahydrofuran, 2-methyltetrahydrofuran, methyl-isobutylketone, *N*-methyl-2-pyrrolidone, *tert*-amyl alcohol, *tert*-butanol, cyclopentane or diethyl ether.
The use of other solvents being also able to ensure lipase activity and a sufficient solubility of the starting materials is considered analogous to the mentioned solvents and is therefore included into the process of the present application.
The ratio between said first solvent and said second solvent is covered by the range from 6:1 to 1:6, preferably from 4:1 to 1:4, more preferably from 3:1 to 1:3, further more preferably from 1:1 to 1:3, and most preferably the ratio is selected from the group consisting of 6:1, 5:1, 4:1, 3:1, 2:1, 1:1, 1:2, 1:3, 1:4, 1:5, or 1:6.

Mixtures of two organic solvents include:
a) mixtures with MTBE:
   - a1: MTBE/MEK in a ratio (v/v) covered by the range from 6:1 to 1:6, preferably from 4:1 to 1:4, more preferably from 3:1 to 1:3, further more preferably from 1:1 to 1:3, and most preferably the ratio is selected from the group consisting of 6:1, 5:1, 4:1, 3:1, 2:1, 1:1, 1:2, 1:3, 1:4, 1:5, or 1:6
   - a2: MTBE/acetone in a ratio (v/v) covered by the range from 6:1 to 1:6, preferably from 4:1 to 1:4, more preferably from 3:1 to 1:3, further more preferably from 1:1 to 1:3, and most preferably the ratio is selected from the group consisting of 6:1, 5:1, 4:1, 3:1, 2:1, 1:1, 1:2, 1:3, 1:4, 1:5, or 1:6
   - a3: MTBE/CPME in a ratio (v/v) covered by the range from 6:1 to 1:6, preferably from 4:1 to 1:4, more preferably from 3:1 to 1:3, further more preferably from 1:1 to 1:3, and most preferably the ratio is selected from the group consisting of 6:1, 5:1, 4:1, 3:1, 2:1, 1:1, 1:2, 1:3, 1:4, 1:5, or 1:6
   - a4: MTBE/anisole in a ratio (v/v) covered by the range from 6:1 to 1:6, preferably from 4:1 to 1:4, more preferably from 3:1 to 1:3, further more preferably from 1:1 to 1:3, and most preferably the ratio is selected from the group consisting of 6:1, 5:1, 4:1, 3:1, 2:1, 1:1, 1:2, 1:3, 1:4, 1:5, or 1:6
   - a5: MTBE/THF in a ratio (v/v) covered by the range from 6:1 to 1:6, preferably from 4:1 to 1:4, more preferably from 3:1 to 1:3, further more preferably from 1:1 to 1:3, and most preferably the ratio is selected from the group consisting of 6:1, 5:1, 4:1, 3:1, 2:1, 1:1, 1:2, 1:3, 1:4, 1:5, or 1:6
   - a6: MTBE/Me-THF in a ratio (v/v) covered by the range from 6:1 to 1:6, preferably from 4:1 to 1:4, more preferably from 3:1 to 1:3, further more preferably from 1:1 to 1:3, and most preferably the ratio is selected from the group consisting of 6:1, 5:1, 4:1, 3:1, 2:1, 1:1, 1:2, 1:3, 1:4, 1:5, or 1:6
   - a7: MTBE/MIBK in a ratio (v/v) covered by the range from 6:1 to 1:6, preferably from 4:1 to 1:4, more preferably from 3:1 to 1:3, further more preferably from 1:1 to 1:3, and most preferably the ratio is selected from the group consisting of 6:1, 5:1, 4:1, 3:1, 2:1, 1:1, 1:2, 1:3, 1:4, 1:5, or 1:6
   - a8: MTBE/NMP in a ratio (v/v) covered by the range from 6:1 to 1:6, preferably from 4:1 to 1:4, more preferably from 3:1 to 1:3, further more preferably from 1:1 to 1:3, and most preferably the ratio is selected from the group consisting of 6:1, 5:1, 4:1, 3:1, 2:1, 1:1, 1:2, 1:3, 1:4, 1:5, or 1:6
   - a9: MTBE/tAmylOH in a ratio (v/v) covered by the range from 6:1 to 1:6, preferably from 4:1 to 1:4, more preferably from 3:1 to 1:3, further more preferably from 1:1 to 1:3, and most preferably the ratio is selected from the group consisting of 6:1, 5:1, 4:1, 3:1, 2:1, 1:1, 1:2, 1:3, 1:4, 1:5, or 1:6
   - a10: MTBE/tBuOH in a ratio (v/v) covered by the range from 6:1 to 1:6, preferably from 4:1 to 1:4, more preferably from 3:1 to 1:3, further more preferably from 1:1 to 1:3, and most preferably the ratio is selected from the group consisting of 6:1, 5:1, 4:1, 3:1, 2:1, 1:1, 1:2, 1:3, 1:4, 1:5, or 1:6
   - a11: MTBE/cyclopentane in a ratio (v/v) covered by the range from 6:1 to 1:6, preferably from 4:1 to 1:4, more preferably from 3:1 to 1:3, further more preferably from 1:1 to 1:3, and most preferably the ratio is selected from the group consisting of 6:1, 5:1, 4:1, 3:1, 2:1, 1:1, 1:2, 1:3, 1:4, 1:5, or 1:6
   - a12: MTBE/diethyl ether in a ratio (v/v) covered by the range from 6:1 to 1:6, preferably from 4:1 to 1:4, more preferably from 3:1 to 1:3, further more preferably from 1:1 to 1:3, and most preferably the ratio is selected from the group consisting of 6:1, 5:1, 4:1, 3:1, 2:1, 1:1, 1:2, 1:3, 1:4, 1:5, or 1:6
b) mixtures with toluene:
   - b1: Toluene/MEK in a ratio (v/v) covered by the range from 6:1 to 1:6, preferably from 4:1 to 1:4, more preferably from 3:1 to 1:3, further more preferably from 1:1 to 1:3, and most preferably the ratio is selected from the group consisting of 6:1, 5:1, 4:1, 3:1, 2:1, 1:1, 1:2, 1:3, 1:4, 1:5, or 1:6
   - b2: Toluene/acetone in a ratio (v/v) covered by the range from 6:1 to 1:6, preferably from 4:1 to 1:4, more preferably from 3:1 to 1:3, further more preferably from 1:1 to 1:3, and most preferably the ratio is selected from the group consisting of 6:1, 5:1, 4:1, 3:1, 2:1, 1:1, 1:2, 1:3, 1:4, 1:5, or 1:6
   - b3: Toluene/CPME in a ratio (v/v) covered by the range from 6:1 to 1:6, preferably from 4:1 to 1:4, more preferably from 3:1 to 1:3, further more preferably from 1:1 to 1:3, and most preferably the ratio is selected from the group consisting of 6:1, 5:1, 4:1, 3:1, 2:1, 1:1, 1:2, 1:3, 1:4, 1:5, or 1:6
   - b4: Toluene/anisole in a ratio (v/v) covered by the range from 6:1 to 1:6, preferably from 4:1 to 1:4, more preferably from 3:1 to 1:3, further more preferably from 1:1 to 1:3, and most preferably the ratio is selected from the group consisting of 6:1, 5:1, 4:1, 3:1, 2:1, 1:1, 1:2, 1:3, 1:4, 1:5, or 1:6
   - b5: Toluene/THF in a ratio (v/v) covered by the range from 6:1 to 1:6, preferably from 4:1 to 1:4, more preferably from 3:1 to 1:3, further more preferably from 1:1 to 1:3, and most preferably the ratio is selected from the group consisting of 6:1, 5:1, 4:1, 3:1, 2:1, 1:1, 1:2, 1:3, 1:4, 1:5, or 1:6
   - b6: Toluene/Me-TE-IF in a ratio (v/v) covered by the range from 6:1 to 1:6, preferably from 4:1 to 1:4, more preferably from 3:1 to 1:3, further more preferably from 1:1 to 1:3, and most preferably the ratio is selected from the group consisting of 6:1, 5:1, 4:1, 3:1, 2:1, 1:1, 1:2, 1:3, 1:4, 1:5, or 1:6
   - b7: Toluene/MIBK in a ratio (v/v) covered by the range from 6:1 to 1:6, preferably from 4:1 to 1:4, more preferably from 3:1 to 1:3, further more preferably from 1:1 to 1:3, and most preferably the ratio is selected from the group consisting of 6:1, 5:1, 4:1, 3:1, 2:1, 1:1, 1:2, 1:3, 1:4, 1:5, or 1:6
   - b8: Toluene/NMP in a ratio (v/v) covered by the range from 6:1 to 1:6, preferably from 4:1 to 1:4, more preferably from 3:1 to 1:3, further more preferably from 1:1 to 1:3, and most preferably the ratio is selected from the group consisting of 6:1, 5:1, 4:1, 3:1, 2:1, 1:1, 1:2, 1:3, 1:4, 1:5, or 1:6
   - b9: Toluene/tAmylOH in a ratio (v/v) covered by the range from 6:1 to 1:6, preferably from 4:1 to 1:4, more preferably from 3:1 to 1:3, further more preferably from 1:1 to 1:3, and most preferably the ratio is selected from the group consisting of 6:1, 5:1, 4:1, 3:1, 2:1, 1:1, 1:2, 1:3, 1:4, 1:5, or 1:6
   - b10: Toluene/tBuOH in a ratio (v/v) covered by the range from 6:1 to 1:6, preferably from 4:1 to 1:4, more preferably from 3:1 to 1:3, further more preferably from 1:1 to 1:3, and most preferably the ratio is selected from the group consisting of 6:1, 5:1, 4:1, 3:1, 2:1, 1:1, 1:2, 1:3, 1:4, 1:5, or 1:6
   - b11: Toluene/cyclopentane in a ratio (v/v) covered by the range from 6:1 to 1:6, preferably from 4:1 to 1:4, more preferably from 3:1 to 1:3, further more preferably from 1:1 to 1:3, and most preferably the ratio is selected from the group consisting of 6:1, 5:1, 4:1, 3:1, 2:1, 1:1, 1:2, 1:3, 1:4, 1:5, or 1:6
   - b12: Toluene/diethyl ether in a ratio (v/v) covered by the range from 6:1 to 1:6, preferably from 4:1 to 1:4, more preferably from 3:1 to 1:3, further more preferably from 1:1 to 1:3, and most preferably the ratio is selected from the group consisting of 6:1, 5:1, 4:1, 3:1, 2:1, 1:1, 1:2, 1:3, 1:4, 1:5, or 1:6
c) mixtures with benzene:
   - c1: Benzene/MEK in a ratio (v/v) covered by the range from 6:1 to 1:6, preferably from 4:1 to 1:4, more preferably from 3:1 to 1:3, further more preferably from 1:1 to 1:3, and most preferably the ratio is selected from the group consisting of 6:1, 5:1, 4:1, 3:1, 2:1, 1:1, 1:2, 1:3, 1:4, 1:5, or 1:6
   - c2: Benzene/acetone in a ratio (v/v) covered by the range from 6:1 to 1:6, preferably from 4:1 to 1:4, more preferably from 3:1 to 1:3, further more preferably from 1:1 to 1:3, and most preferably the ratio is selected from the group consisting of 6:1, 5:1, 4:1, 3:1, 2:1, 1:1, 1:2, 1:3, 1:4, 1:5, or 1:6
   - c3: Benzene/CPME in a ratio (v/v) covered by the range from 6:1 to 1:6, preferably from 4:1 to 1:4, more preferably from 3:1 to 1:3, further more preferably from 1:1 to 1:3, and most preferably the ratio is selected from the group consisting of 6:1, 5:1, 4:1, 3:1, 2:1, 1:1, 1:2, 1:3, 1:4, 1:5, or 1:6
   - c4: Benzene/anisole in a ratio (v/v) covered by the range from 6:1 to 1:6, preferably from 4:1 to 1:4, more preferably from 3:1 to 1:3, further more preferably from 1:1 to 1:3, and most preferably the ratio is selected from the group consisting of 6:1, 5:1, 4:1, 3:1, 2:1, 1:1, 1:2, 1:3, 1:4, 1:5, or 1:6
   - c5: Benzene/THF in a ratio (v/v) covered by the range from 6:1 to 1:6, preferably from 4:1 to 1:4, more preferably from 3:1 to 1:3, further more preferably from 1:1 to 1:3, and most preferably the ratio is selected from the group consisting of 6:1, 5:1, 4:1, 3:1, 2:1, 1:1, 1:2, 1:3, 1:4, 1:5, or 1:6
   - c6: Benzene/Me-THF in a ratio (v/v) covered by the range from 6:1 to 1:6, preferably from 4:1 to 1:4, more preferably from 3:1 to 1:3, further more preferably from 1:1 to 1:3, and most preferably the ratio is selected from the group consisting of 6:1, 5:1, 4:1, 3:1, 2:1, 1:1, 1:2, 1:3, 1:4, 1:5, or 1:6
   - c7: Benzene/MIBK in a ratio (v/v) covered by the range from 6:1 to 1:6, preferably from 4:1 to 1:4, more preferably from 3:1 to 1:3, further more preferably from 1:1 to 1:3, and most preferably the ratio is selected from the group consisting of 6:1, 5:1, 4:1, 3:1, 2:1, 1:1, 1:2, 1:3, 1:4, 1:5, or 1:6
   - c8: Benzene/NMP in a ratio (v/v) covered by the range from 6:1 to 1:6, preferably from 4:1 to 1:4, more preferably from 3:1 to 1:3, further more preferably from 1:1 to 1:3, and most preferably the ratio is selected from the group consisting of 6:1, 5:1, 4:1, 3:1, 2:1, 1:1, 1:2, 1:3, 1:4, 1:5, or 1:6
   - c9: Benzene/tAmylOH in a ratio (v/v) covered by the range from 6:1 to 1:6, preferably from 4:1 to 1:4, more preferably from 3:1 to 1:3, further more preferably from 1:1 to 1:3, and most preferably the ratio is selected from the group consisting of 6:1, 5:1, 4:1, 3:1, 2:1, 1:1, 1:2, 1:3, 1:4, 1:5, or 1:6
   - c10: Benzene/tBuOH in a ratio (v/v) covered by the range from 6:1 to 1:6, preferably from 4:1 to 1:4, more preferably from 3:1 to 1:3, further more preferably from 1:1 to 1:3, and most preferably the ratio is selected from the group consisting of 6:1, 5:1, 4:1, 3:1, 2:1, 1:1, 1:2, 1:3, 1:4, 1:5, or 1:6
   - c11: Benzene/cyclopentane in a ratio (v/v) covered by the range from 6:1 to 1:6, preferably from 4:1 to 1:4, more preferably from 3:1 to 1:3, further more preferably from 1:1 to 1:3, and most preferably the ratio is selected from the group consisting of 6:1, 5:1, 4:1, 3:1, 2:1, 1:1, 1:2, 1:3, 1:4, 1:5, or 1:6
   - c12: Benzene/diethyl ether in a ratio (v/v) covered by the range from 6:1 to 1:6, preferably from 4:1 to 1:4, more preferably from 3:1 to 1:3, further more preferably from 1:1 to 1:3, and most preferably the ratio is selected from the group consisting of 6:1, 5:1, 4:1, 3:1, 2:1, 1:1, 1:2, 1:3, 1:4, 1:5, or 1:6
d) mixtures with CPME:
   - d1: CPME/MEK in a ratio (v/v) covered by the range from 6:1 to 1:6, preferably from 4:1 to 1:4, more preferably from 3:1 to 1:3, further more preferably from 1:1 to 1:3, and most preferably the ratio is selected from the group consisting of 6:1, 5:1, 4:1, 3:1, 2:1, 1:1, 1:2, 1:3, 1:4, 1:5, or 1:6
   - d2: CPME/acetone in a ratio (v/v) covered by the range from 6:1 to 1:6, preferably from 4:1 to 1:4, more preferably from 3:1 to 1:3, further more preferably from 1:1 to 1:3, and most preferably the ratio is selected from the group consisting of 6:1, 5:1, 4:1, 3:1, 2:1, 1:1, 1:2, 1:3, 1:4, 1:5, or 1:6
   - d3: CPME/anisole in a ratio (v/v) covered by the range from 6:1 to 1:6, preferably from 4:1 to 1:4, more preferably from 3:1 to 1:3, further more preferably from 1:1 to 1:3, and most preferably the ratio is selected from the group consisting of 6:1, 5:1, 4:1, 3:1, 2:1, 1:1, 1:2, 1:3, 1:4, 1:5, or 1:6
   - d4: CPME/THF in a ratio (v/v) covered by the range from 6:1 to 1:6, preferably from 4:1 to 1:4, more preferably from 3:1 to 1:3, further more preferably from 1:1 to 1:3, and most preferably the ratio is selected from the group consisting of 6:1, 5:1, 4:1, 3:1, 2:1, 1:1, 1:2, 1:3, 1:4, 1:5, or 1:6
   - d5: CPME/Me-THF in a ratio (v/v) covered by the range from 6:1 to 1:6, preferably from 4:1 to 1:4, more preferably from 3:1 to 1:3, further more preferably from 1:1 to 1:3, and most preferably the ratio is selected from the group consisting of 6:1, 5:1, 4:1, 3:1, 2:1, 1:1, 1:2, 1:3, 1:4, 1:5, or 1:6
   - d6: CPME/MIBK in a ratio (v/v) covered by the range from 6:1 to 1:6, preferably from 4:1 to 1:4, more preferably from 3:1 to 1:3, further more preferably from 1:1 to 1:3, and most preferably the ratio is selected from the group consisting of 6:1, 5:1, 4:1, 3:1, 2:1, 1:1, 1:2, 1:3, 1:4, 1:5, or 1:6
   - d7: CPME/NMP in a ratio (v/v) covered by the range from 6:1 to 1:6, preferably from 4:1 to 1:4, more preferably from 3:1 to 1:3, further more preferably from 1:1 to 1:3, and most preferably the ratio is selected from the group consisting of 6:1, 5:1, 4:1, 3:1, 2:1, 1:1, 1:2, 1:3, 1:4, 1:5, or 1:6
   - d8: CPME/tAmylOH in a ratio (v/v) covered by the range from 6:1 to 1:6, preferably from 4:1 to 1:4, more preferably from 3:1 to 1:3, further more preferably from 1:1 to 1:3, and most preferably the ratio is selected from the group consisting of 6:1, 5:1, 4:1, 3:1, 2:1, 1:1, 1:2, 1:3, 1:4, 1:5, or 1:6
   - d9: CPME/tBuOH in a ratio (v/v) covered by the range from 6:1 to 1:6, preferably from 4:1 to 1:4, more preferably from 3:1 to 1:3, further more preferably from 1:1 to 1:3, and most preferably the ratio is selected from the group consisting of 6:1, 5:1, 4:1, 3:1, 2:1, 1:1, 1:2, 1:3, 1:4, 1:5, or 1:6
   - d10: CPME/cyclopentane in a ratio (v/v) covered by the range from 6:1 to 1:6, preferably from 4:1 to 1:4, more preferably from 3:1 to 1:3, further more preferably from 1:1 to 1:3, and most preferably the ratio is selected from the group consisting of 6:1, 5:1, 4:1, 3:1, 2:1, 1:1, 1:2, 1:3, 1:4, 1:5, or 1:6
   - d11: CPME/diethyl ether in a ratio (v/v) covered by the range from 6:1 to 1:6, preferably from 4:1 to 1:4, more preferably from 3:1 to 1:3, further more preferably from 1:1 to 1:3, and most preferably the ratio is selected from the group consisting of 6:1, 5:1, 4:1, 3:1, 2:1, 1:1, 1:2, 1:3, 1:4, 1:5, or 1:6
e) mixtures with MeCN:
   - e1: MeCN/MEK in a ratio (v/v) covered by the range from 6:1 to 1:6, preferably from 4:1 to 1:4, more preferably from 3:1 to 1:3, further more preferably from 1:1 to 1:3, and most preferably the ratio is selected from the group consisting of 6:1, 5:1, 4:1, 3:1, 2:1, 1:1, 1:2, 1:3, 1:4, 1:5, or 1:6
   - e2: MeCN/acetone in a ratio (v/v) covered by the range from 6:1 to 1:6, preferably from 4:1 to 1:4, more preferably from 3:1 to 1:3, further more preferably from 1:1 to 1:3, and most preferably the ratio is selected from the group consisting of 6:1, 5:1, 4:1, 3:1, 2:1, 1:1, 1:2, 1:3, 1:4, 1:5, or 1:6
   - e3: MeCN/CPME in a ratio (v/v) covered by the range from 6:1 to 1:6, preferably from 4:1 to 1:4, more preferably from 3:1 to 1:3, further more preferably from 1:1 to 1:3, and most preferably the ratio is selected from the group consisting of 6:1, 5:1, 4:1, 3:1, 2:1, 1:1, 1:2, 1:3, 1:4, 1:5, or 1:6
   - e4: MeCN/anisole in a ratio (v/v) covered by the range from 6:1 to 1:6, preferably from 4:1 to 1:4, more preferably from 3:1 to 1:3, further more preferably from 1:1 to 1:3, and most preferably the ratio is selected from the group consisting of 6:1, 5:1, 4:1, 3:1, 2:1, 1:1, 1:2, 1:3, 1:4, 1:5, or 1:6
   - e5: MeCN/THF in a ratio (v/v) covered by the range from 6:1 to 1:6, preferably from 4:1 to 1:4, more preferably from 3:1 to 1:3, further more preferably from 1:1 to 1:3, and most preferably the ratio is selected from the group consisting of 6:1, 5:1, 4:1, 3:1, 2:1, 1:1, 1:2, 1:3, 1:4, 1:5, or 1:6
   - e6: MeCN/Me-THF in a ratio (v/v) covered by the range from 6:1 to 1:6, preferably from 4:1 to 1:4, more preferably from 3:1 to 1:3, further more preferably from 1:1 to 1:3, and most preferably the ratio is selected from the group consisting of 6:1, 5:1, 4:1, 3:1, 2:1, 1:1, 1:2, 1:3, 1:4, 1:5, or 1:6
   - e7: MeCN/MIBK in a ratio (v/v) covered by the range from 6:1 to 1:6, preferably from 4:1 to 1:4, more preferably from 3:1 to 1:3, further more preferably from 1:1 to 1:3, and most preferably the ratio is selected from the group consisting of 6:1, 5:1, 4:1, 3:1, 2:1, 1:1, 1:2, 1:3, 1:4, 1:5, or 1:6
   - e8: MeCN/NMP in a ratio (v/v) covered by the range from 6:1 to 1:6, preferably from 4:1 to 1:4, more preferably from 3:1 to 1:3, further more preferably from 1:1 to 1:3, and most preferably the ratio is selected from the group consisting of 6:1, 5:1, 4:1, 3:1, 2:1, 1:1, 1:2, 1:3, 1:4, 1:5, or 1:6
   - e9: MeCN/tAmylOH in a ratio (v/v) covered by the range from 6:1 to 1:6, preferably from 4:1 to 1:4, more preferably from 3:1 to 1:3, further more preferably from 1:1 to 1:3, and most preferably the ratio is selected from the group consisting of 6:1, 5:1, 4:1, 3:1, 2:1, 1:1, 1:2, 1:3, 1:4, 1:5, or 1:6
   - e10: MeCN/tBuOH in a ratio (v/v) covered by the range from 6:1 to 1:6, preferably from 4:1 to 1:4, more preferably from 3:1 to 1:3, further more preferably from 1:1 to 1:3, and most preferably the ratio is selected from the group consisting of 6:1, 5:1, 4:1, 3:1, 2:1, 1:1, 1:2, 1:3, 1:4, 1:5, or 1:6
   - e11: MeCN/cyclopentane in a ratio (v/v) covered by the range from 6:1 to 1:6, preferably from 4:1 to 1:4, more preferably from 3:1 to 1:3, further more preferably from 1:1 to 1:3, and most preferably the ratio is selected from the group consisting of 6:1, 5:1, 4:1, 3:1, 2:1, 1:1, 1:2, 1:3, 1:4, 1:5, or 1:6
   - e12: MeCN/diethyl ether in a ratio (v/v) covered by the range from 6:1 to 1:6, preferably from 4:1 to 1:4, more preferably from 3:1 to 1:3, further more preferably from 1:1 to 1:3, and most preferably the ratio is selected from the group consisting of 6:1, 5:1, 4:1, 3:1, 2:1, 1:1, 1:2, 1:3, 1:4, 1:5, or 1:6
f) mixtures with THF:
   - f1: THF/MEK in a ratio (v/v) covered by the range from 6:1 to 1:6, preferably from 4:1 to 1:4, more preferably from 3:1 to 1:3, further more preferably from 1:1 to 1:3, and most preferably the ratio is selected from the group consisting of 6:1, 5:1, 4:1, 3:1, 2:1, 1:1, 1:2, 1:3, 1:4, 1:5, or 1:6
   - f2: THF/acetone in a ratio (v/v) covered by the range from 6:1 to 1:6, preferably from 4:1 to 1:4, more preferably from 3:1 to 1:3, further more preferably from 1:1 to 1:3, and most preferably the ratio is selected from the group consisting of 6:1, 5:1, 4:1, 3:1, 2:1, 1:1, 1:2, 1:3, 1:4, 1:5, or 1:6
   - f3: THF/CPME in a ratio (v/v) covered by the range from 6:1 to 1:6, preferably from 4:1 to 1:4, more preferably from 3:1 to 1:3, further more preferably from 1:1 to 1:3, and most preferably the ratio is selected from the group consisting of 6:1, 5:1, 4:1, 3:1, 2:1, 1:1, 1:2, 1:3, 1:4, 1:5, or 1:6
   - f4: THF/anisole in a ratio (v/v) covered by the range from 6:1 to 1:6, preferably from 4:1 to 1:4, more preferably from 3:1 to 1:3, further more preferably from 1:1 to 1:3, and most preferably the ratio is selected from the group consisting of 6:1, 5:1, 4:1, 3:1, 2:1, 1:1, 1:2, 1:3, 1:4, 1:5, or 1:6
   - f5: THF/Me-THF in a ratio (v/v) covered by the range from 6:1 to 1:6, preferably from 4:1 to 1:4, more preferably from 3:1 to 1:3, further more preferably from 1:1 to 1:3, and most preferably the ratio is selected from the group consisting of 6:1, 5:1, 4:1, 3:1, 2:1, 1:1, 1:2, 1:3, 1:4, 1:5, or 1:6
   - f6: THF/MIBK in a ratio (v/v) covered by the range from 6:1 to 1:6, preferably from 4:1 to 1:4, more preferably from 3:1 to 1:3, further more preferably from 1:1 to 1:3, and most preferably the ratio is selected from the group consisting of 6:1, 5:1, 4:1, 3:1, 2:1, 1:1, 1:2, 1:3, 1:4, 1:5, or 1:6
   - f7: THF/NMP in a ratio (v/v) covered by the range from 6:1 to 1:6, preferably from 4:1 to 1:4, more preferably from 3:1 to 1:3, further more preferably from 1:1 to 1:3, and most preferably the ratio is selected from the group consisting of 6:1, 5:1, 4:1, 3:1, 2:1, 1:1, 1:2, 1:3, 1:4, 1:5, or 1:6
   - f8: THF/tAmylOH in a ratio (v/v) covered by the range from 6:1 to 1:6, preferably from 4:1 to 1:4, more preferably from 3:1 to 1:3, further more preferably from 1:1 to 1:3, and most preferably the ratio is selected from the group consisting of 6:1, 5:1, 4:1, 3:1, 2:1, 1:1, 1:2, 1:3, 1:4, 1:5, or 1:6
   - f9: THF/tBuOH in a ratio (v/v) covered by the range from 6:1 to 1:6, preferably from 4:1 to 1:4, more preferably from 3:1 to 1:3, further more preferably from 1:1 to 1:3, and most preferably the ratio is selected from the group consisting of 6:1, 5:1, 4:1, 3:1, 2:1, 1:1, 1:2, 1:3, 1:4, 1:5, or 1:6
   - f10: THF/cyclopentane in a ratio (v/v) covered by the range from 6:1 to 1:6, preferably from 4:1 to 1:4, more preferably from 3:1 to 1:3, further more preferably from 1:1 to 1:3, and most preferably the ratio is selected from the group consisting of 6:1, 5:1, 4:1, 3:1, 2:1, 1:1, 1:2, 1:3, 1:4, 1:5, or 1:6
   - f11: THF/diethyl ether in a ratio (v/v) covered by the range from 6:1 to 1:6, preferably from 4:1 to 1:4, more preferably from 3:1 to 1:3, further more preferably from 1:1 to 1:3, and most preferably the ratio is selected from the group consisting of 6:1, 5:1, 4:1, 3:1, 2:1, 1:1, 1:2, 1:3, 1:4, 1:5, or 1:6
g) mixtures with Me-THF:
   - g1: Me-THF/MEK in a ratio (v/v) covered by the range from 6:1 to 1:6, preferably from 4:1 to 1:4, more preferably from 3:1 to 1:3, further more preferably from 1:1 to 1:3, and most preferably the ratio is selected from the group consisting of 6:1, 5:1, 4:1, 3:1, 2:1, 1:1, 1:2, 1:3, 1:4, 1:5, or 1:6
   - g2: Me-THF/acetone in a ratio (v/v) covered by the range from 6:1 to 1:6, preferably from 4:1 to 1:4, more preferably from 3:1 to 1:3, further more preferably from 1:1 to 1:3, and most preferably the ratio is selected from the group consisting of 6:1, 5:1, 4:1, 3:1, 2:1, 1:1, 1:2, 1:3, 1:4, 1:5, or 1:6
   - g3: Me-THF/CPME in a ratio (v/v) covered by the range from 6:1 to 1:6, preferably from 4:1 to 1:4, more preferably from 3:1 to 1:3, further more preferably from 1:1 to 1:3, and most preferably the ratio is selected from the group consisting of 6:1, 5:1, 4:1, 3:1, 2:1, 1:1, 1:2, 1:3, 1:4, 1:5, or 1:6
   - g4: Me-THF/anisole in a ratio (v/v) covered by the range from 6:1 to 1:6, preferably from 4:1 to 1:4, more preferably from 3:1 to 1:3, further more preferably from 1:1 to 1:3, and most preferably the ratio is selected from the group consisting of 6:1, 5:1, 4:1, 3:1, 2:1, 1:1, 1:2, 1:3, 1:4, 1:5, or 1:6
   - g5: Me-THF/THF in a ratio (v/v) covered by the range from 6:1 to 1:6, preferably from 4:1 to 1:4, more preferably from 3:1 to 1:3, further more preferably from 1:1 to 1:3, and most preferably the ratio is selected from the group consisting of 6:1, 5:1, 4:1, 3:1, 2:1, 1:1, 1:2, 1:3, 1:4, 1:5, or 1:6
   - g6: Me-THF/MIBK in a ratio (v/v) covered by the range from 6:1 to 1:6, preferably from 4:1 to 1:4, more preferably from 3:1 to 1:3, further more preferably from 1:1 to 1:3, and most preferably the ratio is selected from the group consisting of 6:1, 5:1, 4:1, 3:1, 2:1, 1:1, 1:2, 1:3, 1:4, 1:5, or 1:6
   - g7: Me-THF/NMP in a ratio (v/v) covered by the range from 6:1 to 1:6, preferably from 4:1 to 1:4, more preferably from 3:1 to 1:3, further more preferably from 1:1 to 1:3, and most preferably the ratio is selected from the group consisting of 6:1, 5:1, 4:1, 3:1, 2:1, 1:1, 1:2, 1:3, 1:4, 1:5, or 1:6
   - g8: Me-THF/tAmylOH in a ratio (v/v) covered by the range from 6:1 to 1:6, preferably from 4:1 to 1:4, more preferably from 3:1 to 1:3, further more preferably from 1:1 to 1:3, and most preferably the ratio is selected from the group consisting of 6:1, 5:1, 4:1, 3:1, 2:1, 1:1, 1:2, 1:3, 1:4, 1:5, or 1:6
   - g9: Me-THF/tBuOH in a ratio (v/v) covered by the range from 6:1 to 1:6, preferably from 4:1 to 1:4, more preferably from 3:1 to 1:3, further more preferably from 1:1 to 1:3, and most preferably the ratio is selected from the group consisting of 6:1, 5:1, 4:1, 3:1, 2:1, 1:1, 1:2, 1:3, 1:4, 1:5, or 1:6
   - g10: Me-THF/cyclopentane in a ratio (v/v) covered by the range from 6:1 to 1:6, preferably from 4:1 to 1:4, more preferably from 3:1 to 1:3, further more preferably from 1:1 to 1:3, and most preferably the ratio is selected from the group consisting of 6:1, 5:1, 4:1, 3:1, 2:1, 1:1, 1:2, 1:3, 1:4, 1:5, or 1:6
   - g11: Me-THF/diethyl ether in a ratio (v/v) covered by the range from 6:1 to 1:6, preferably from 4:1 to 1:4, more preferably from 3:1 to 1:3, further more preferably from 1:1 to 1:3, and most preferably the ratio is selected from the group consisting of 6:1, 5:1, 4:1, 3:1, 2:1, 1:1, 1:2, 1:3, 1:4, 1:5, or 1:6
h) mixtures with MEK:
   - h1: MEK/acetone in a ratio (v/v) covered by the range from 6:1 to 1:6, preferably from 4:1 to 1:4, more preferably from 3:1 to 1:3, further more preferably from 1:1 to 1:3, and most preferably the ratio is selected from the group consisting of 6:1, 5:1, 4:1, 3:1, 2:1, 1:1, 1:2, 1:3, 1:4, 1:5, or 1:6
   - h2: MEK/CPME in a ratio (v/v) covered by the range from 6:1 to 1:6, preferably from 4:1 to 1:4, more preferably from 3:1 to 1:3, further more preferably from 1:1 to 1:3, and most preferably the ratio is selected from the group consisting of 6:1, 5:1, 4:1, 3:1, 2:1, 1:1, 1:2, 1:3, 1:4, 1:5, or 1:6
   - h3: MEK/anisole in a ratio (v/v) covered by the range from 6:1 to 1:6, preferably from 4:1 to 1:4, more preferably from 3:1 to 1:3, further more preferably from 1:1 to 1:3, and most preferably the ratio is selected from the group consisting of 6:1, 5:1, 4:1, 3:1, 2:1, 1:1, 1:2, 1:3, 1:4, 1:5, or 1:6
   - h4: MEK/THF in a ratio (v/v) covered by the range from 6:1 to 1:6, preferably from 4:1 to 1:4, more preferably from 3:1 to 1:3, further more preferably from 1:1 to 1:3, and most preferably the ratio is selected from the group consisting of 6:1, 5:1, 4:1, 3:1, 2:1, 1:1, 1:2, 1:3, 1:4, 1:5, or 1:6
   - h5: MEK/Me-THF in a ratio (v/v) covered by the range from 6:1 to 1:6, preferably from 4:1 to 1:4, more preferably from 3:1 to 1:3, further more preferably from 1:1 to 1:3, and most preferably the ratio is selected from the group consisting of 6:1, 5:1, 4:1, 3:1, 2:1, 1:1, 1:2, 1:3, 1:4, 1:5, or 1:6
   - h6: MEK/MIBK in a ratio (v/v) covered by the range from 6:1 to 1:6, preferably from 4:1 to 1:4, more preferably from 3:1 to 1:3, further more preferably from 1:1 to 1:3, and most preferably the ratio is selected from the group consisting of 6:1, 5:1, 4:1, 3:1, 2:1, 1:1, 1:2, 1:3, 1:4, 1:5, or 1:6
   - h7: MEK/NMP in a ratio (v/v) covered by the range from 6:1 to 1:6, preferably from 4:1 to 1:4, more preferably from 3:1 to 1:3, further more preferably from 1:1 to 1:3, and most preferably the ratio is selected from the group consisting of 6:1, 5:1, 4:1, 3:1, 2:1, 1:1, 1:2, 1:3, 1:4, 1:5, or 1:6
   - h8: MEK/tAmylOH in a ratio (v/v) covered by the range from 6:1 to 1:6, preferably from 4:1 to 1:4, more preferably from 3:1 to 1:3, further more preferably from 1:1 to 1:3, and most preferably the ratio is selected from the group consisting of 6:1, 5:1, 4:1, 3:1, 2:1, 1:1, 1:2, 1:3, 1:4, 1:5, or 1:6
   - h9: MEK/tBuOH in a ratio (v/v) covered by the range from 6:1 to 1:6, preferably from 4:1 to 1:4, more preferably from 3:1 to 1:3, further more preferably from 1:1 to 1:3, and most preferably the ratio is selected from the group consisting of 6:1, 5:1, 4:1, 3:1, 2:1, 1:1, 1:2, 1:3, 1:4, 1:5, or 1:6
   - h10: MEK/cyclopentane in a ratio (v/v) covered by the range from 6:1 to 1:6, preferably from 4:1 to 1:4, more preferably from 3:1 to 1:3, further more preferably from 1:1 to 1:3, and most preferably the ratio is selected from the group consisting of 6:1, 5:1, 4:1, 3:1, 2:1, 1:1, 1:2, 1:3, 1:4, 1:5, or 1:6
   - h11: MEK/diethyl ether in a ratio (v/v) covered by the range from 6:1 to 1:6, preferably from 4:1 to 1:4, more preferably from 3:1 to 1:3, further more preferably from 1:1 to 1:3, and most preferably the ratio is selected from the group consisting of 6:1, 5:1, 4:1, 3:1, 2:1, 1:1, 1:2, 1:3, 1:4, 1:5, or 1:6
i) mixtures with EtOAc:
   - i1: EtOAc/MEK in a ratio (v/v) covered by the range from 6:1 to 1:6, preferably from 4:1 to 1:4, more preferably from 3:1 to 1:3, further more preferably from 1:1 to 1:3, and most preferably the ratio is selected from the group consisting of 6:1, 5:1, 4:1, 3:1, 2:1, 1:1, 1:2, 1:3, 1:4, 1:5, or 1:6
   - i2: EtOAc/acetone in a ratio (v/v) covered by the range from 6:1 to 1:6, preferably from 4:1 to 1:4, more preferably from 3:1 to 1:3, further more preferably from 1:1 to 1:3, and most preferably the ratio is selected from the group consisting of 6:1, 5:1, 4:1, 3:1, 2:1, 1:1, 1:2, 1:3, 1:4, 1:5, or 1:6
   - i3: EtOAc/CPME in a ratio (v/v) covered by the range from 6:1 to 1:6, preferably from 4:1 to 1:4, more preferably from 3:1 to 1:3, further more preferably from 1:1 to 1:3, and most preferably the ratio is selected from the group consisting of 6:1, 5:1, 4:1, 3:1, 2:1, 1:1, 1:2, 1:3, 1:4, 1:5, or 1:6
   - i4: EtOAc/anisole in a ratio (v/v) covered by the range from 6:1 to 1:6, preferably from 4:1 to 1:4, more preferably from 3:1 to 1:3, further more preferably from 1:1 to 1:3, and most preferably the ratio is selected from the group consisting of 6:1, 5:1, 4:1, 3:1, 2:1, 1:1, 1:2, 1:3, 1:4, 1:5, or 1:6
   - i5: EtOAc/THF in a ratio (v/v) covered by the range from 6:1 to 1:6, preferably from 4:1 to 1:4, more preferably from 3:1 to 1:3, further more preferably from 1:1 to 1:3, and most preferably the ratio is selected from the group consisting of 6:1, 5:1, 4:1, 3:1, 2:1, 1:1, 1:2, 1:3, 1:4, 1:5, or 1:6
   - i6: EtOAc/Me-THF in a ratio (v/v) covered by the range from 6:1 to 1:6, preferably from 4:1 to 1:4, more preferably from 3:1 to 1:3, further more preferably from 1:1 to 1:3, and most preferably the ratio is selected from the group consisting of 6:1, 5:1, 4:1, 3:1, 2:1, 1:1, 1:2, 1:3, 1:4, 1:5, or 1:6
   - i7: EtOAc/MIBK in a ratio (v/v) covered by the range from 6:1 to 1:6, preferably from 4:1 to 1:4, more preferably from 3:1 to 1:3, further more preferably from 1:1 to 1:3, and most preferably the ratio is selected from the group consisting of 6:1, 5:1, 4:1, 3:1, 2:1, 1:1, 1:2, 1:3, 1:4, 1:5, or 1:6
   - i8: EtOAc/NMP in a ratio (v/v) covered by the range from 6:1 to 1:6, preferably from 4:1 to 1:4, more preferably from 3:1 to 1:3, further more preferably from 1:1 to 1:3, and most preferably the ratio is selected from the group consisting of 6:1, 5:1, 4:1, 3:1, 2:1, 1:1, 1:2, 1:3, 1:4, 1:5, or 1:6
   - i9: EtOAc/tAmylOH in a ratio (v/v) covered by the range from 6:1 to 1:6, preferably from 4:1 to 1:4, more preferably from 3:1 to 1:3, further more preferably from 1:1 to 1:3, and most preferably the ratio is selected from the group consisting of 6:1, 5:1, 4:1, 3:1, 2:1, 1:1, 1:2, 1:3, 1:4, 1:5, or 1:6
   - i10: EtOAc/tBuOH in a ratio (v/v) covered by the range from 6:1 to 1:6, preferably from 4:1 to 1:4, more preferably from 3:1 to 1:3, further more preferably from 1:1 to 1:3, and most preferably the ratio is selected from the group consisting of 6:1, 5:1, 4:1, 3:1, 2:1, 1:1, 1:2, 1:3, 1:4, 1:5, or 1:6
   - i11: EtOAc/cyclopentane in a ratio (v/v) covered by the range from 6:1 to 1:6, preferably from 4:1 to 1:4, more preferably from 3:1 to 1:3, further more preferably from 1:1 to 1:3, and most preferably the ratio is selected from the group consisting of 6:1, 5:1, 4:1, 3:1, 2:1, 1:1, 1:2, 1:3, 1:4, 1:5, or 1:6
   - i12: EtOAc/diethyl ether in a ratio (v/v) covered by the range from 6:1 to 1:6, preferably from 4:1 to 1:4, more preferably from 3:1 to 1:3, further more preferably from 1:1 to 1:3, and most preferably the ratio is selected from the group consisting of 6:1, 5:1, 4:1, 3:1, 2:1, 1:1, 1:2, 1:3, 1:4, 1:5, or 1:6
j) mixtures with 1,4-dioxane:
   - j1: 1,4-dioxane/MEK in a ratio (v/v) covered by the range from 6:1 to 1:6, preferably from 4:1 to 1:4, more preferably from 3:1 to 1:3, further more preferably from 1:1 to 1:3, and most preferably the ratio is selected from the group consisting of 6:1, 5:1, 4:1, 3:1, 2:1, 1:1, 1:2, 1:3, 1:4, 1:5, or 1:6
   - j2: 1,4-dioxane/acetone in a ratio (v/v) covered by the range from 6:1 to 1:6, preferably from 4:1 to 1:4, more preferably from 3:1 to 1:3, further more preferably from 1:1 to 1:3, and most preferably the ratio is selected from the group consisting of 6:1, 5:1, 4:1, 3:1, 2:1, 1:1, 1:2, 1:3, 1:4, 1:5, or 1:6
   - j3: 1,4-dioxane/CPME in a ratio (v/v) covered by the range from 6:1 to 1:6, preferably from 4:1 to 1:4, more preferably from 3:1 to 1:3, further more preferably from 1:1 to 1:3, and most preferably the ratio is selected from the group consisting of 6:1, 5:1, 4:1, 3:1, 2:1, 1:1, 1:2, 1:3, 1:4, 1:5, or 1:6
   - j4: 1,4-dioxane/anisole in a ratio (v/v) covered by the range from 6:1 to 1:6, preferably from 4:1 to 1:4, more preferably from 3:1 to 1:3, further more preferably from 1:1 to 1:3, and most preferably the ratio is selected from the group consisting of 6:1, 5:1, 4:1, 3:1, 2:1, 1:1, 1:2, 1:3, 1:4, 1:5, or 1:6
   - j5: 1,4-dioxane/THF in a ratio (v/v) covered by the range from 6:1 to 1:6, preferably from 4:1 to 1:4, more preferably from 3:1 to 1:3, further more preferably from 1:1 to 1:3, and most preferably the ratio is selected from the group consisting of 6:1, 5:1, 4:1, 3:1, 2:1, 1:1, 1:2, 1:3, 1:4, 1:5, or 1:6
   - j6: 1,4-dioxane/Me-THF in a ratio (v/v) covered by the range from 6:1 to 1:6, preferably from 4:1 to 1:4, more preferably from 3:1 to 1:3, further more preferably from 1:1 to 1:3, and most preferably the ratio is selected from the group consisting of 6:1, 5:1, 4:1, 3:1, 2:1, 1:1, 1:2, 1:3, 1:4, 1:5, or 1:6
   - j7: 1,4-dioxane/MIBK in a ratio (v/v) covered by the range from 6:1 to 1:6, preferably from 4:1 to 1:4, more preferably from 3:1 to 1:3, further more preferably from 1:1 to 1:3, and most preferably the ratio is selected from the group consisting of 6:1, 5:1, 4:1, 3:1, 2:1, 1:1, 1:2, 1:3, 1:4, 1:5, or 1:6
   - j8: 1,4-dioxane/NMP in a ratio (v/v) covered by the range from 6:1 to 1:6, preferably from 4:1 to 1:4, more preferably from 3:1 to 1:3, further more preferably from 1:1 to 1:3, and most preferably the ratio is selected from the group consisting of 6:1, 5:1, 4:1, 3:1, 2:1, 1:1, 1:2, 1:3, 1:4, 1:5, or 1:6
   - j9: 1,4-dioxane/tAmylOH in a ratio (v/v) covered by the range from 6:1 to 1:6, preferably from 4:1 to 1:4, more preferably from 3:1 to 1:3, further more preferably from 1:1 to 1:3, and most preferably the ratio is selected from the group consisting of 6:1, 5:1, 4:1, 3:1, 2:1, 1:1, 1:2, 1:3, 1:4, 1:5, or 1:6
   - j10: 1,4-dioxane/tBuOH in a ratio (v/v) covered by the range from 6:1 to 1:6, preferably from 4:1 to 1:4, more preferably from 3:1 to 1:3, further more preferably from 1:1 to 1:3, and most preferably the ratio is selected from the group consisting of 6:1, 5:1, 4:1, 3:1, 2:1, 1:1, 1:2, 1:3, 1:4, 1:5, or 1:6
   - j11: 1,4-dioxane/cyclopentane in a ratio (v/v) covered by the range from 6:1 to 1:6, preferably from 4:1 to 1:4, more preferably from 3:1 to 1:3, further more preferably from 1:1 to 1:3, and most preferably the ratio is selected from the group consisting of 6:1, 5:1, 4:1, 3:1, 2:1, 1:1, 1:2, 1:3, 1:4, 1:5, or 1:6
   - j12: 1,4-dioxane/diethyl ether in a ratio (v/v) covered by the range from 6:1 to 1:6, preferably from 4:1 to 1:4, more preferably from 3:1 to 1:3, further more preferably from 1:1 to 1:3, and most preferably the ratio is selected from the group consisting of 6:1, 5:1, 4:1, 3:1, 2:1, 1:1, 1:2, 1:3, 1:4, 1:5, or 1:6
k) mixtures with *n*-hexane (hex):
   - k1: Hex/MEK in a ratio (v/v) covered by the range from 6:1 to 1:6, preferably from 4:1 to 1:4, more preferably from 3:1 to 1:3, further more preferably from 1:1 to 1:3, and most preferably the ratio is selected from the group consisting of 6:1, 5:1, 4:1, 3:1, 2:1, 1:1, 1:2, 1:3, 1:4, 1:5, or 1:6
   - k2: Hex/acetone in a ratio (v/v) covered by the range from 6:1 to 1:6, preferably from 4:1 to 1:4, more preferably from 3:1 to 1:3, further more preferably from 1:1 to 1:3, and most preferably the ratio is selected from the group consisting of 6:1, 5:1, 4:1, 3:1, 2:1, 1:1, 1:2, 1:3, 1:4, 1:5, or 1:6
   - k3: Hex/CPME in a ratio (v/v) covered by the range from 6:1 to 1:6, preferably from 4:1 to 1:4, more preferably from 3:1 to 1:3, further more preferably from 1:1 to 1:3, and most preferably the ratio is selected from the group consisting of 6:1, 5:1, 4:1, 3:1, 2:1, 1:1, 1:2, 1:3, 1:4, 1:5, or 1:6
   - k4: Hex/anisole in a ratio (v/v) covered by the range from 6:1 to 1:6, preferably from 4:1 to 1:4, more preferably from 3:1 to 1:3, further more preferably from 1:1 to 1:3, and most preferably the ratio is selected from the group consisting of 6:1, 5:1, 4:1, 3:1, 2:1, 1:1, 1:2, 1:3, 1:4, 1:5, or 1:6
   - k5: Hex/THF in a ratio (v/v) covered by the range from 6:1 to 1:6, preferably from 4:1 to 1:4, more preferably from 3:1 to 1:3, further more preferably from 1:1 to 1:3, and most preferably the ratio is selected from the group consisting of 6:1, 5:1, 4:1, 3:1, 2:1, 1:1, 1:2, 1:3, 1:4, 1:5, or 1:6
   - k6: Hex/Me-THF in a ratio (v/v) covered by the range from 6:1 to 1:6, preferably from 4:1 to 1:4, more preferably from 3:1 to 1:3, further more preferably from 1:1 to 1:3, and most preferably the ratio is selected from the group consisting of 6:1, 5:1, 4:1, 3:1, 2:1, 1:1, 1:2, 1:3, 1:4, 1:5, or 1:6
   - k7: Hex/MIBK in a ratio (v/v) covered by the range from 6:1 to 1:6, preferably from 4:1 to 1:4, more preferably from 3:1 to 1:3, further more preferably from 1:1 to 1:3, and most preferably the ratio is selected from the group consisting of 6:1, 5:1, 4:1, 3:1, 2:1, 1:1, 1:2, 1:3, 1:4, 1:5, or 1:6
   - k8: Hex/NMP in a ratio (v/v) covered by the range from 6:1 to 1:6, preferably from 4:1 to 1:4, more preferably from 3:1 to 1:3, further more preferably from 1:1 to 1:3, and most preferably the ratio is selected from the group consisting of 6:1, 5:1, 4:1, 3:1, 2:1, 1:1, 1:2, 1:3, 1:4, 1:5, or 1:6
   - k9: Hex/tAmylOH in a ratio (v/v) covered by the range from 6:1 to 1:6, preferably from 4:1 to 1:4, more preferably from 3:1 to 1:3, further more preferably from 1:1 to 1:3, and most preferably the ratio is selected from the group consisting of 6:1, 5:1, 4:1, 3:1, 2:1, 1:1, 1:2, 1:3, 1:4, 1:5, or 1:6
   - k10: Hex/tBuOH in a ratio (v/v) covered by the range from 6:1 to 1:6, preferably from 4:1 to 1:4, more preferably from 3:1 to 1:3, further more preferably from 1:1 to 1:3, and most preferably the ratio is selected from the group consisting of 6:1, 5:1, 4:1, 3:1, 2:1, 1:1, 1:2, 1:3, 1:4, 1:5, or 1:6
   - k11: Hex/cyclopentane in a ratio (v/v) covered by the range from 6:1 to 1:6, preferably from 4:1 to 1:4, more preferably from 3:1 to 1:3, further more preferably from 1:1 to 1:3, and most preferably the ratio is selected from the group consisting of 6:1, 5:1, 4:1, 3:1, 2:1, 1:1, 1:2, 1:3, 1:4, 1:5, or 1:6
   - k12: Hex/diethyl ether in a ratio (v/v) covered by the range from 6:1 to 1:6, preferably from 4:1 to 1:4, more preferably from 3:1 to 1:3, further more preferably from 1:1 to 1:3, and most preferably the ratio is selected from the group consisting of 6:1, 5:1, 4:1, 3:1, 2:1, 1:1, 1:2, 1:3, 1:4, 1:5, or 1:6
l) mixtures with *n*-heptane (hept):
   - l1: Hept/MEK in a ratio (v/v) covered by the range from 6:1 to 1:6, preferably from 4:1 to 1:4, more preferably from 3:1 to 1:3, further more preferably from 1:1 to 1:3, and most preferably the ratio is selected from the group consisting of 6:1, 5:1, 4:1, 3:1, 2:1, 1:1, 1:2, 1:3, 1:4, 1:5, or 1:6
   - l2: Hept/acetone in a ratio (v/v) covered by the range from 6:1 to 1:6, preferably from 4:1 to 1:4, more preferably from 3:1 to 1:3, further more preferably from 1:1 to 1:3, and most preferably the ratio is selected from the group consisting of 6:1, 5:1, 4:1, 3:1, 2:1, 1:1, 1:2, 1:3, 1:4, 1:5, or 1:6
   - l3: Hept/CPME in a ratio (v/v) covered by the range from 6:1 to 1:6, preferably from 4:1 to 1:4, more preferably from 3:1 to 1:3, further more preferably from 1:1 to 1:3, and most preferably the ratio is selected from the group consisting of 6:1, 5:1, 4:1, 3:1, 2:1, 1:1, 1:2, 1:3, 1:4, 1:5, or 1:6
   - 14: Hept/anisole in a ratio (v/v) covered by the range from 6:1 to 1:6, preferably from 4:1 to 1:4, more preferably from 3:1 to 1:3, further more preferably from 1:1 to 1:3, and most preferably the ratio is selected from the group consisting of 6:1, 5:1, 4:1, 3:1, 2:1, 1:1, 1:2, 1:3, 1:4, 1:5, or 1:6
   - l5: Hept/THF in a ratio (v/v) covered by the range from 6:1 to 1:6, preferably from 4:1 to 1:4, more preferably from 3:1 to 1:3, further more preferably from 1:1 to 1:3, and most preferably the ratio is selected from the group consisting of 6:1, 5:1, 4:1, 3:1, 2:1, 1:1, 1:2, 1:3, 1:4, 1:5, or 1:6
   - l6: Hept/Me-THF in a ratio (v/v) covered by the range from 6:1 to 1:6, preferably from 4:1 to 1:4, more preferably from 3:1 to 1:3, further more preferably from 1:1 to 1:3, and most preferably the ratio is selected from the group consisting of 6:1, 5:1, 4:1, 3:1, 2:1, 1:1, 1:2, 1:3, 1:4, 1:5, or 1:6
   - l7: Hept/MIBK in a ratio (v/v) covered by the range from 6:1 to 1:6, preferably from 4:1 to 1:4, more preferably from 3:1 to 1:3, further more preferably from 1:1 to 1:3, and most preferably the ratio is selected from the group consisting of 6:1, 5:1, 4:1, 3:1, 2:1, 1:1, 1:2, 1:3, 1:4, 1:5, or 1:6
   - 18: Hept/NMP in a ratio (v/v) covered by the range from 6:1 to 1:6, preferably from 4:1 to 1:4, more preferably from 3:1 to 1:3, further more preferably from 1:1 to 1:3, and most preferably the ratio is selected from the group consisting of 6:1, 5:1, 4:1, 3:1, 2:1, 1:1, 1:2, 1:3, 1:4, 1:5, or 1:6
   - l9: Hept/tAmylOH in a ratio (v/v) covered by the range from 6:1 to 1:6, preferably from 4:1 to 1:4, more preferably from 3:1 to 1:3, further more preferably from 1:1 to 1:3, and most preferably the ratio is selected from the group consisting of 6:1, 5:1, 4:1, 3:1, 2:1, 1:1, 1:2, 1:3, 1:4, 1:5, or 1:6
   - l10: Hept/tBuOH in a ratio (v/v) covered by the range from 6:1 to 1:6, preferably from 4:1 to 1:4, more preferably from 3:1 to 1:3, further more preferably from 1:1 to 1:3, and most preferably the ratio is selected from the group consisting of 6:1, 5:1, 4:1, 3:1, 2:1, 1:1, 1:2, 1:3, 1:4, 1:5, or 1:6
   - l11: Hept/cyclopentane in a ratio (v/v) covered by the range from 6:1 to 1:6, preferably from 4:1 to 1:4, more preferably from 3:1 to 1:3, further more preferably from 1:1 to 1:3, and most preferably the ratio is selected from the group consisting of 6:1, 5:1, 4:1, 3:1, 2:1, 1:1, 1:2, 1:3, 1:4, 1:5, or 1:6
   - l12: Hept/diethyl ether in a ratio (v/v) covered by the range from 6:1 to 1:6, preferably from 4:1 to 1:4, more preferably from 3:1 to 1:3, further more preferably from 1:1 to 1:3, and most preferably the ratio is selected from the group consisting of 6:1, 5:1, 4:1, 3:1, 2:1, 1:1, 1:2, 1:3, 1:4, 1:5, or 1:6
m) mixtures with *n*-pentane (pent):
   - m1: Pent/MEK in a ratio (v/v) covered by the range from 6:1 to 1:6, preferably from 4:1 to 1:4, more preferably from 3:1 to 1:3, further more preferably from 1:1 to 1:3, and most preferably the ratio is selected from the group consisting of 6:1, 5:1, 4:1, 3:1, 2:1, 1:1, 1:2, 1:3, 1:4, 1:5, or 1:6
   - m2: Pent/acetone in a ratio (v/v) covered by the range from 6:1 to 1:6, preferably from 4:1 to 1:4, more preferably from 3:1 to 1:3, further more preferably from 1:1 to 1:3, and most preferably the ratio is selected from the group consisting of 6:1, 5:1, 4:1, 3:1, 2:1, 1:1, 1:2, 1:3, 1:4, 1:5, or 1:6
   - m3: Pent/CPME in a ratio (v/v) covered by the range from 6:1 to 1:6, preferably from 4:1 to 1:4, more preferably from 3:1 to 1:3, further more preferably from 1:1 to 1:3, and most preferably the ratio is selected from the group consisting of 6:1, 5:1, 4:1, 3:1, 2:1, 1:1, 1:2, 1:3, 1:4, 1:5, or 1:6
   - m4: Pent/anisole in a ratio (v/v) covered by the range from 6:1 to 1:6, preferably from 4:1 to 1:4, more preferably from 3:1 to 1:3, further more preferably from 1:1 to 1:3, and most preferably the ratio is selected from the group consisting of 6:1, 5:1, 4:1, 3:1, 2:1, 1:1, 1:2, 1:3, 1:4, 1:5, or 1:6
   - m5: Pent/THF in a ratio (v/v) covered by the range from 6:1 to 1:6, preferably from 4:1 to 1:4, more preferably from 3:1 to 1:3, further more preferably from 1:1 to 1:3, and most preferably the ratio is selected from the group consisting of 6:1, 5:1, 4:1, 3:1, 2:1, 1:1, 1:2, 1:3, 1:4, 1:5, or 1:6
   - m6: Pent/Me-THF in a ratio (v/v) covered by the range from 6:1 to 1:6, preferably from 4:1 to 1:4, more preferably from 3:1 to 1:3, further more preferably from 1:1 to 1:3, and most preferably the ratio is selected from the group consisting of 6:1, 5:1, 4:1, 3:1, 2:1, 1:1, 1:2, 1:3, 1:4, 1:5, or 1:6
   - m7: Pent/MIBK in a ratio (v/v) covered by the range from 6:1 to 1:6, preferably from 4:1 to 1:4, more preferably from 3:1 to 1:3, further more preferably from 1:1 to 1:3, and most preferably the ratio is selected from the group consisting of 6:1, 5:1, 4:1, 3:1, 2:1, 1:1, 1:2, 1:3, 1:4, 1:5, or 1:6
   - m8: Pent/NMP in a ratio (v/v) covered by the range from 6:1 to 1:6, preferably from 4:1 to 1:4, more preferably from 3:1 to 1:3, further more preferably from 1:1 to 1:3, and most preferably the ratio is selected from the group consisting of 6:1, 5:1, 4:1, 3:1, 2:1, 1:1, 1:2, 1:3, 1:4, 1:5, or 1:6
   - m9: Pent/tAmylOH in a ratio (v/v) covered by the range from 6:1 to 1:6, preferably from 4:1 to 1:4, more preferably from 3:1 to 1:3, further more preferably from 1:1 to 1:3, and most preferably the ratio is selected from the group consisting of 6:1, 5:1, 4:1, 3:1, 2:1, 1:1, 1:2, 1:3, 1:4, 1:5, or 1:6
   - m10: Pent/tBuOH in a ratio (v/v) covered by the range from 6:1 to 1:6, preferably from 4:1 to 1:4, more preferably from 3:1 to 1:3, further more preferably from 1:1 to 1:3, and most preferably the ratio is selected from the group consisting of 6:1, 5:1, 4:1, 3:1, 2:1, 1:1, 1:2, 1:3, 1:4, 1:5, or 1:6
   - m11: Pent/cyclopentane in a ratio (v/v) covered by the range from 6:1 to 1:6, preferably from 4:1 to 1:4, more preferably from 3:1 to 1:3, further more preferably from 1:1 to 1:3, and most preferably the ratio is selected from the group consisting of 6:1, 5:1, 4:1, 3:1, 2:1, 1:1, 1:2, 1:3, 1:4, 1:5, or 1:6
   - m12: Pent/diethyl ether in a ratio (v/v) covered by the range from 6:1 to 1:6, preferably from 4:1 to 1:4, more preferably from 3:1 to 1:3, further more preferably from 1:1 to 1:3, and most preferably the ratio is selected from the group consisting of 6:1, 5:1, 4:1, 3:1, 2:1, 1:1, 1:2, 1:3, 1:4, 1:5, or 1:6
n) mixtures with isooctane:
   - n1: Isooctane/MEK in a ratio (v/v) covered by the range from 6:1 to 1:6, preferably from 4:1 to 1:4, more preferably from 3:1 to 1:3, further more preferably from 1:1 to 1:3, and most preferably the ratio is selected from the group consisting of 6:1, 5:1, 4:1, 3:1, 2:1, 1:1, 1:2, 1:3, 1:4, 1:5, or 1:6
   - n2: Isooctane/acetone in a ratio (v/v) covered by the range from 6:1 to 1:6, preferably from 4:1 to 1:4, more preferably from 3:1 to 1:3, further more preferably from 1:1 to 1:3, and most preferably the ratio is selected from the group consisting of 6:1, 5:1, 4:1, 3:1, 2:1, 1:1, 1:2, 1:3, 1:4, 1:5, or 1:6
   - n3: Isooctane/CPME in a ratio (v/v) covered by the range from 6:1 to 1:6, preferably from 4:1 to 1:4, more preferably from 3:1 to 1:3, further more preferably from 1:1 to 1:3, and most preferably the ratio is selected from the group consisting of 6:1, 5:1, 4:1, 3:1, 2:1, 1:1, 1:2, 1:3, 1:4, 1:5, or 1:6
   - n4: Isooctane/anisole in a ratio (v/v) covered by the range from 6:1 to 1:6, preferably from 4:1 to 1:4, more preferably from 3:1 to 1:3, further more preferably from 1:1 to 1:3, and most preferably the ratio is selected from the group consisting of 6:1, 5:1, 4:1, 3:1, 2:1, 1:1, 1:2, 1:3, 1:4, 1:5, or 1:6
   - n5: Isooctane/THF in a ratio (v/v) covered by the range from 6:1 to 1:6, preferably from 4:1 to 1:4, more preferably from 3:1 to 1:3, further more preferably from 1:1 to 1:3, and most preferably the ratio is selected from the group consisting of 6:1, 5:1, 4:1, 3:1, 2:1, 1:1, 1:2, 1:3, 1:4, 1:5, or 1:6
   - n6: Isooctane/Me-THF in a ratio (v/v) covered by the range from 6:1 to 1:6, preferably from 4:1 to 1:4, more preferably from 3:1 to 1:3, further more preferably from 1:1 to 1:3, and most preferably the ratio is selected from the group consisting of 6:1, 5:1, 4:1, 3:1, 2:1, 1:1, 1:2, 1:3, 1:4, 1:5, or 1:6
   - n7: Isooctane/MIBK in a ratio (v/v) covered by the range from 6:1 to 1:6, preferably from 4:1 to 1:4, more preferably from 3:1 to 1:3, further more preferably from 1:1 to 1:3, and most preferably the ratio is selected from the group consisting of 6:1, 5:1, 4:1, 3:1, 2:1, 1:1, 1:2, 1:3, 1:4, 1:5, or 1:6
   - n8: Isooctane/NMP in a ratio (v/v) covered by the range from 6:1 to 1:6, preferably from 4:1 to 1:4, more preferably from 3:1 to 1:3, further more preferably from 1:1 to 1:3, and most preferably the ratio is selected from the group consisting of 6:1, 5:1, 4:1, 3:1, 2:1, 1:1, 1:2, 1:3, 1:4, 1:5, or 1:6
   - n9: Isooctane/tAmylOH in a ratio (v/v) covered by the range from 6:1 to 1:6, preferably from 4:1 to 1:4, more preferably from 3:1 to 1:3, further more preferably from 1:1 to 1:3, and most preferably the ratio is selected from the group consisting of 6:1, 5:1, 4:1, 3:1, 2:1, 1:1, 1:2, 1:3, 1:4, 1:5, or 1:6
   - n10: Isooctane/tBuOH in a ratio (v/v) covered by the range from 6:1 to 1:6, preferably from 4:1 to 1:4, more preferably from 3:1 to 1:3, further more preferably from 1:1 to 1:3, and most preferably the ratio is selected from the group consisting of 6:1, 5:1, 4:1, 3:1, 2:1, 1:1, 1:2, 1:3, 1:4, 1:5, or 1:6
   - n11: Isooctane/cyclopentane in a ratio (v/v) covered by the range from 6:1 to 1:6, preferably from 4:1 to 1:4, more preferably from 3:1 to 1:3, further more preferably from 1:1 to 1:3, and most preferably the ratio is selected from the group consisting of 6:1, 5:1, 4:1, 3:1, 2:1, 1:1, 1:2, 1:3, 1:4, 1:5, or 1:6
   - n12: Isooctane/diethyl ether in a ratio (v/v) covered by the range from 6:1 to 1:6, preferably from 4:1 to 1:4, more preferably from 3:1 to 1:3, further more preferably from 1:1 to 1:3, and most preferably the ratio is selected from the group consisting of 6:1, 5:1, 4:1, 3:1, 2:1, 1:1, 1:2, 1:3, 1:4, 1:5, or 1:6

The addition of further solvents to form a ternary, or higher order mixtures, wherein said solvent or solvents is
- selected from group (B)
- also suited to facilitate azeotropic distillation
- selected from group (C)
- also suited to ensure lipase activity and a sufficient solubility of the starting materials, or
- a combination of these options
is considered analogously to the mentioned solvent or solvents and is therefore included into the process of the present application.
For example, the use of solvent systems like MTBE/MEK/toluene, MTBE/MEK/Me-THF, MTBE/anisole/acetone or MTBE/Me-THF/benzene is considered analogous and is therefore included into the process of the present application.
The use of ratios in between the mentioned ratios, e.g. ratios like [1.2 : 1], [3.6 : 1.2] or [1.1 : 2.2] is considered analogous and is therefore included into the process of the present application.

Examples 2a, 1 d and 2b show the small impact of the used starting material concentrations (i.e. the ratio between weight of starting material to solvent) in the process of the invention with regard to the yield.

The process of the invention is carried out at a reaction temperature of 0 to 150 °C, preferably at 25 to 130 °C, more preferably at 40 to 110 °C, even more preferably at 50 to 100 °C and most preferably at 60 to 80 °C.

The process of the present invention is carried out under conditions effective to remove water from the reaction mixture in order to allow the reaction to proceed to completion. These conditions include but are not limited to adding dehydrating agents (e.g., molecular sieves, calcium carbonate, magnesium sulfate or sodium sulfate) directly to the reaction mixture, microwave heating, and azeotropic distillation using organic media as described above. The latter is used to conduct the reaction in refluxing organic media using a flask/reactor fitted with a distillation apparatus. The distilled solvent is then wasted and simultaneously replaced by fresh solvent, while maintaining the original volume and ratio of solvents in the vessel. The flask/reactor with the refluxing organic media can also be fitted with a Dean-Stark and condenser assembly or a higher-scale technical equivalent, where after separation of water, the organic media is looped back into the flask/reactor. Since azeotropic distillation is somewhat less effective in smaller scale, the solvent reservoir of the Dean-Stark apparatus may be filled with molecular sieves to finally absorb water transported out of the reaction vessel. It is also possible to use a dropping funnel and condenser assembly in which the dropping funnel (but not the pressure equalizing tube) is filled with a dehydrating agent, e.g. molecular sieves.
Other methods known to those skilled in the art to remove water may also be used. While the removal of water from the reaction vessel is critical for the invention, the method to separate and finally absorb the water is not important as long as the water removal is efficient.

Dicarboxylic acid suitable as bis-acyl donor compounds of the present invention can be but are not limited to free dicarboxy groups (free acids), dicarbonates and anhydrides.

Examples of bis-acyl donors include dicarboxylic acids such as malonic acid, succinic acid, glutaric acid, adipic acid, pimelic acid, suberic acid, azelaic acid, sebacic acid, undecanedioic acid, dodecanedioic acid, 1,12-dodecanedicarboxylic acid, fumaric acid, maleic acid, glutaconic acid, traumatic acid, muconic acid, cis aconitic acid, phthalic acid, isophthalic acid, terephthalic acid, cyclohexan-1,4-dicarboxylic acid, 3-(2-carboxyethyldisulfanyl)propionic acid, diglycolic acid, thio diglycolic acid, 3-(2-carboxyethoxy)propionic acid, 3-[2-(2-carboxyethoxy)ethoxy]propionic acid, 3-{2-[2-(2-carboxyethoxy)ethoxy]ethoxy}propionic acid, 3-(2-{2-[2-(2-carboxy-ethoxy)ethoxy]ethoxy}ethoxy)propionic acid, 3-[2-(2-{2-[2-(2-carboxyethoxy)ethoxy]ethoxy}ethoxy)ethoxy]propionic acid, 3-{2-[2-(2-{2-[2-(2-carboxyethoxy)ethoxy] ethoxy}ethoxy)ethoxy]ethoxy}propionic acid and 3-(2-{2-[2-(2-{2-[2-(2-carboxyethoxy)ethoxy]ethoxy}ethoxy)ethoxy]ethoxy}ethoxy)propionic acid.

Examples of bis-acyl donors include anhydrides such as succinic anhydride, glutaric anhydride, adipic anhydride, pimelic anhydride, suberic anhydride, maleic anhydride, cis aconitic anhydride, phthalic anhydride, diglycolic anhydride and thio diglycolic anhydride.

Weight equivalents of bis-acyl donor with regard to the starting material can vary from substoichiometric ratios over stoichiometric ratios to use of an excess of bis-acyl donor.
As demonstrated by examples 2n-p, the amount of weight equivalents of added glutaric acid with regard to *N*-Fmoc-doxorubicin is an important factor in order to arrive at an optimal yield. While varying the glutaric acid weight equivalents from 2.5 to 12 eq., the yield of *N*-Fmoc-doxorubicin hemiglutarate increased.
Glutaric acid weight equivalents in between the mentioned values, i.e. weight equivalents like e.g. 3.2 wt. eq. or e.g. 7.1 wt. eq. are incorporated in the invention analogous to the mentioned values.

Lipases of the present invention can either be native (wild-type) lipases or genetically-modified lipases engineered using standard methods of molecular biology (Berglund, "Controlling Lipase Enantioselectivity for Organic Synthesis," Biomol. Eng., 18(1): 13-22 (2001); Brocca et al., "Novel Lipases Having Altered Substrate Specificity, Methods for Their Preparation, and Their Use in Biocatalytic Applications," Eur. Pat. Appl. pp.33 (2001); Svendsen, "Lipase Protein Engineering," Biochim. Biophys. Acta, 1543(2): 223-238 (2000); Wong et al., "Lipase Engineering: A Window into Structure-Function Relationships," Methods in Enzymology, 284: 171-84 (1997), which are hereby incorporated in their entirety).

Lipases that can be used for the selective C 14-OH acylation of *N*-Fmoc-doxorubicin include lipases from microbial sources such as *Alicaligenes* sp., *Aspergillus, Aspergillus niger, Aspergillus oryzae, Alcaligenes* sp., *Candida antartica, Candida cylindracea* sp., *Candida rugosa, Fusarium solani pisi, Mucor miehei, Mucor javanicus, Penicillium roqueforti, Pseudomonas cepacia, Pseudomonas fluorescens, Pseudomonas* sp., *Pseudomonas stutzeri, Rhizomucor miehei, Rhizopus arrhizus, Rhizopus delemar, Rhizopus niveus, Rhizopus oryzae, Thermomyces lanuginosus, Bacillus subtilis* and *Porcine pancreas.*

The nonaqueous biocatalysis of the present invention can be carried out where the lipase is immobilized to a solid support. Examples of solid support include diatomaceous earth, silica, ceramic, polypropylene, polystyrene or acrylic resins. The lipase can be immobilized by covalent cross-linking of enzyme aggregates (CLEAs).
Other methods known to those skilled in the art to immobilize a lipase can also be used. The type of solid support or the way the lipase is immobilized to a solid support is not critical for the invention as long as the lipase retains its natural function on the solid support.

Weight equivalents of lipase with regard to the starting material can vary from substoichiometric ratios over stoichiometric ratios to use of an excess of lipase.
Weight equivalents in the context of an immobilized lipase mean the use of a lipase with an activity of 10.000 units/g resin. In the case of a different activity the weight equivalents are corrected accordingly. As demonstrated by examples 2h-m, the amount of weight equivalents of added *Candida Antarctica B* with regard to *N*-Fmoc-doxorubicin is an important factor in order to arrive at an optimal yield. Varying the *Candida Antarctica B* weight equivalents from 0.5 to 6 eq. with regard to the *N*-Fmoc-doxorubicin starting material, increased the yield of *N*-Fmoc-doxorubicin hemiglutarate while the reaction time decreased.
Weight equivalents in between the mentioned values, e.g. weight equivalents like e.g. 1.2 wt. eq. or e.g. 3 wt. eq. are incorporated in the invention as analogous to the mentioned *Candida Antarctica B* weight equivalents values.

### Examples

In order to compare the process of the invention with conditions known from the prior art, reference experiments 5 and 6 are described below.

### Reference experiment 5: Synthesis of N-alloc-doxorubicin-O-hemiglutarate using glutaric acid as bis-acyl donor under lipase conditions

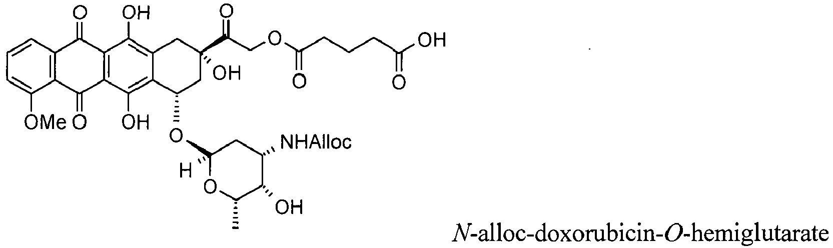

*N*-alloc-doxorubicin (50 mg) was suspended in MTBE/MEK 3:1 (v/v) (10 mL) and to this was added lipase acrylic resin from *Candida Antarctica B* (233 mg, 5 weight equivalents) and glutaric acid (109 mg). The reaction flask was fitted with a dropping funnel and condenser assembly in which the dropping funnel was filled with 3 Å molecular sieves (ca. 10 g). The reaction mixture was stirred vigorously and heated for 25 h at reflux temperature. The progress of the reaction was monitored by HPLC. No work-up was performed.

Yield: not isolated
Purity: 50.9 % (HPLC- see Appendix 4)

### Reference experiment 6: Synthesis of N-trifluoroacetyl-doxorubicin-O-hemiglutarate using glutaric acid as bis-acyl donor under lipase conditions

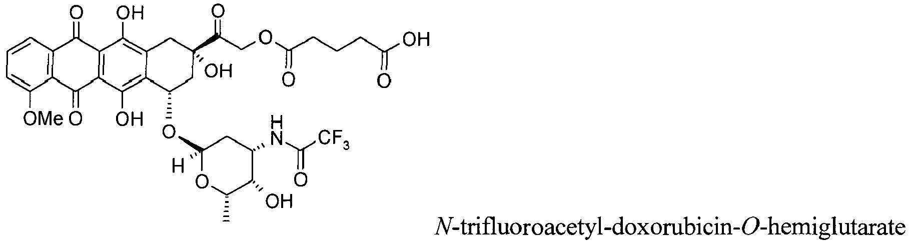

The same reaction conditions as used in reference experiment 5 were applied for the acylation except using *N-*trifluoroacetyl-doxorubicin (Cotterill et al. Organic Process Research and Development 2005, 9, 818) (50 mg) instead of doxorubicin. No work-up was performed.

Yield: not isolated
Purity: 70.2 % (HPLC- see Appendix 5)

In a process typical for the invention, the *N*-Fmoc-doxorubicin derivative (Formula II) is dissolved in suitable organic media, containing 2.5-12 weight equivalents of the desired dicarboxylic acid. To this mixture 1-12 weight equivalents resin bound lipase per g *N*-Fmoc-doxorubicin are added. The reaction flask can be incubated at 25-130 °C, or, alternatively, if an accumulation of the reaction product water inhibits the progress of the reaction, fitted with a condenser assembly and/or an assembly to remove water and stirred at the reflux temperature of the solvent, from 2 hours up to 2 days. Reaction progress is followed by high performance liquid chromatography ("HPLC") or any other suitable analytical method. Upon depletion of the substrate the reaction mixture is filtered to remove the insoluble enzyme powder. The enzyme is washed with an appropriate solvent and the combined solvent is washed with an appropriate aqueous medium, dried and evaporated under reduced pressure. The product is purified using a suitable method, most routinely recrystallization, precipitation, silica gel chromatography, liquid chromatography on other solid adsorbents, or preparative high performance liquid chromatography.

### Inventive Examples

The following examples are provided to illustrate embodiments of the present invention.

### Example 1a: Synthesis of N-Fmoc-doxorubicin-O-hemiglutarate using glutaric acid as bis-acyl donor under lipase conditions in MTBE/MEK

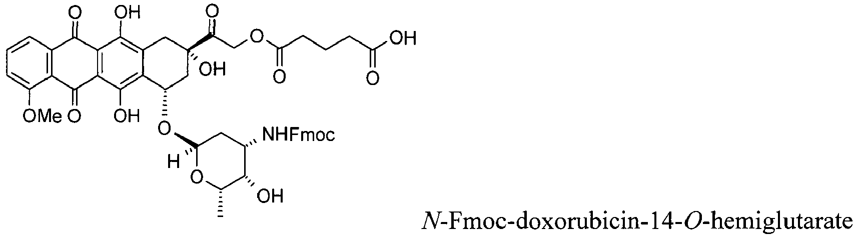

*N*-Fmoc-doxorubicin (1.00 g) was suspended in MTBE/MEK 3:1 (v/v) (80 mL) and to this was added lipase acrylic resin from *Candida Antarctica B* (4.00 g, 4 wt. eq., all weight equivalents with regard to the *N*-Fmoc-doxorubicin starting material) and glutaric acid (1.73 g, 10 eq.). The reaction flask was fitted with a dropping funnel and condenser assembly in which the dropping funnel (but not the pressure equalizing tube) was filled with 3 Å molecular sieves (ca. 60 g). The reaction mixture was stirred vigorously and heated for 25 h at reflux temperature. The progress of the reaction was monitored by HPLC. The enzyme was removed by filtration and washed with hot MTBE (4 x 10 mL). The filtrate was washed with water (5 x 40 mL) and evaporated to dryness under reduced pressure at a temperature of ≤ 50 °C. The residue was redissolved in 5 mL of MEK and added drop wise into 60 mL of a vigorously stirred aqueous acetic acid solution (w/w = 5 %). After stirring for one hour, the product is filtered through a membrane filter (0.2 µm), washed with water and dried under vacuum to constant weight at a temperature of ≤ 50 °C to give crude *N*-Fmoc-doxorubicin-14-*O*-hemiglutarate.

Yield: 1.06 g red solid (92 %)
Purity: 88.2 % (HPLC - see Appendix 6)

### Example 1b: Synthesis of N-Fmoc-doxorubicin-O-hemiglutarate using glutaric anhydride and glutaric acid as bis-acyl donor under lipase conditions in MTBE/PhOMe

*N*-Fmoc-doxorubicin (300 mg) was suspended in MTBE/PhOMe 1:1 (v/v) (24 mL) and to this was added lipase acrylic resin from *Candida Antarctica B* (1200 mg, 4 wt. eq., all weight equivalents with regard to the *N*-Fmoc-doxorubicin starting material), glutaric anhydride (223 mg, 5 eq.) and glutaric acid (258 mg, 5eq.). The reaction mixture was stirred vigorously and heated for 25 h at approx. 80 °C. The progress of the reaction was monitored by HPLC. Following the removal of the enzyme by filtration the reaction was worked-up analogously to Example 1a to give crude
*N*-Fmoc-doxorubicin-14-*O*-hemiglutarate.

Yield: 301 mg red solid (88 %)
Purity: 87.6 % (HPLC - see Appendix 7)

### Example 1c: Synthesis of N-Fmoc-doxorubicin-O-hemiglutarate using glutaric acid as bis-acyl donor under lipase conditions in Me-THF

*N*-Fmoc-doxorubicin (300 mg) was suspended in Me-THF (24 mL) and to this was added lipase acrylic resin from *Candida Antarctica B* (1200 mg, 4 wt. eq., all weight equivalents with regard to the *N*-Fmoc-doxorubicin starting material) and glutaric acid (518 mg, 10 eq.). The reaction flask was fitted with a column and condenser assembly in which the column was filled with 3 Å molecular sieves (ca. 20 g). The reaction mixture was stirred vigorously and heated for 4 h at approx. 100 °C. The progress of the reaction was monitored by HPLC. Following the removal of the enzyme by filtration the reaction was worked-up analogously to Example 1 a to give crude
*N*-Fmoc-doxorubicin-14-*O*-hemiglutarate.

Yield: 254 mg red solid (74 %)
Purity: 74.2 % (HPLC - see Appendix 8)

### Example 1d: Synthesis of N-Fmoc-doxorubicin-O-hemiglutarate using glutaric acid as bis-acyl donor under lipase conditions in MTBE/Me-THF

*N*-Fmoc-doxorubicin (300 mg) was suspended in MTBE/Me-THF 1:2 (v/v) (18 mL) and to this was added lipase acrylic resin from *Candida Antarctica B* (1200 mg, 4 wt. eq., all weight equivalents with regard to the *N*-Fmoc-doxorubicin starting material) and glutaric acid (518 mg, 10 eq.). The reaction flask was fitted with a column and condenser assembly in which the column was filled with 3 Å molecular sieves (ca. 20 g). The reaction mixture was stirred vigorously and heated for 4 h at approx. 90 °C. The progress of the reaction was monitored by HPLC. Following the removal of the enzyme by filtration the reaction was worked-up analogously to Example 1 a to give crude *N*-Fmoc-doxorubicin-14-*O*-hemiglutarate.

Yield: 327 mg red solid (95 %)
Purity: 92.1 % (HPLC - see Appendix 9)

**Example 1e: Synthesis under continuous distillation** *N*-Fmoc-doxorubicin (3 g) was suspended in MTBE/Me-THF 1:2 (v/v) (180 mL) and to this was added lipase acrylic resin from *Candida Antarctica B* (12 g, 4 wt. eq., all weight equivalents with regard to the *N*-Fmoc-doxorubicin starting material) and glutaric acid (5.2 g, 10 eq.). The reaction was performed in a reaction flask which was fitted with a Dean-Stark condenser assembly. The reaction mixture was stirred vigorously and heated for 10 h at approx. 90 °C, while the distillate was collected and removed from the side arm of the Dean-Stark apparatus. Fresh solvent MTBE/Me-THF 1:2 (v/v) was added to replace the removed distillate. The progress of the reaction was monitored by HPLC.

Yield: not isolated
Purity: 75.7 % (HPLC - see Appendix 10)

### Examples 1f-1k: Synthesis of N-Fmoc-doxorubicin-O-hemiglutarate using different lipid organic media under lipase conditions

Examples 1f-1k were performed analogously to Example 1 a applying different liquid organic media. The results are summarized in the following table:

| Ex. | Solvent system (v/v) | T (°C) | Solvent volume (vol./wt.) | Time (h) | *N*-Fmoc-doxorubicin (Formula II) (%, HPLC) | *N*-Fmoc-doxorubicin-O-hemiglutarate (%, HPLC) | Appendix |
|---|---|---|---|---|---|---|---|
| 1f | THF | 61-64 | 80 | 25 | 48.7 | 30.8 | 11 |
| 1g | Me-THF/THF (1 : 1) | 85 | 80 | 25 | 8.2 | 84.6 | 12 |
| 1h | THF/t-Amyl-OH (1:2) | 80 | 80 | 25 | 50.7 | 45.0 | 13 |
| 1i | MTBE/Me-THF (1:4) | 90 | 80 | 16 | 2.5 | 90.5 | 14 |
| 1j | MTBE/Me-THF (1 : 1) | 90 | 80 | 4 | 1.7 | 91.1 | 15 |
| 1k | MTBE/Me-THF (3:1) | 90 | 80 | 8 | 1.9 | 89.1 | 16 |

### Examples 2a-b: Synthesis of N-Fmoc-doxorubicin-O-hemiglutarate using different N-Fmoc-doxorubicin concentrations

Examples 2a-b were performed analogously to Example 1d applying different starting material concentrations, i.e. varying the ratio between solvent volume to weight of *N*-Fmoc-doxorubicin.

| Ex. | Solvent (v/v) | T (°C) | Solvent volume (vol./wt.) | Time (h) | *N*-Fmoc-doxorubicin (Formula II) (%, HPLC) | *N*-Fmoc-doxorubicin-*O-*hemiglutarate (%, HPLC) | Appendix |
|---|---|---|---|---|---|---|---|
| 2a | MTBE/Me-THF (1:2) | 90 | 80 | 4 | 3.7 | 90.6 | 17 |
| 1d | MTBE/Me-THF (1:2) | 90 | 60 | 4 | 2.6 | 92.1 | 9 |
| 2b | MTBE/Me-THF (1:2) | 90 | 40 | 4 | 2.6 | 90.8 | 18 |

### Examples 2c-g: Synthesis of N-Fmoc-doxorubicin-O-hemiglutarate using different water removing conditions

*N*-Fmoc-doxorubicin (300 mg) was suspended in MTBE/Me-THF 1:2 (v/v) (18 mL) and to this was added lipase acrylic resin from *Candida Antarctica B* (1200 mg, 4 wt. eq., all weight equivalents with regard to the *N*-Fmoc-doxorubicin starting material), glutaric acid (518 mg, 10 eq. with regard to the *N*-Fmoc-doxorubicin starting material). The reaction was performed in a reaction flask which was fitted with a column and condenser assembly in which the column was filled with 3 Å molecular sieves or a Dean-Stark condenser assembly or in a sealed tube. The reaction mixture was stirred vigorously and heated at approx. 90 °C. The progress of the reaction was monitored by HPLC. The results are summarized in the following table:

| Example | Drying conditions | Time (h) | *N*-Fmoc-doxorubicin-*O-*hemiglutarate (%, HPLC) |
|---|---|---|---|
| 2c | 60 wt. eq. of molecular sieves (3 Å) | 4 | 91.1 |
| 2d | 20 wt. eq. of molecular sieves (3 Å) | 5 | 91.2 |
| 2e | 5 wt. eq. of molecular sieves (3 Å) | 4 | 93.2 |
| 2f | No molecular sieves, sealed tube | 25 | 52.9 |
| 2g | Dean-Stark apparatus | 4 | 74.0 |

It is shown by these examples, that effective removal of water is an important factor in order to arrive at an optimal yield.

### Examples 2h-m: Synthesis of N-Fmoc-doxorubicin-O-hemiglutarate using different amounts of lipase Candida Antarctica B

*N*-Fmoc-doxorubicin (300 mg) was suspended in MTBE/Me-THF 1:2 (v/v) (18 mL) and to this was added lipase acrylic resin from *Candida Antarctica B* (0.5, 1.0, 2.0 or 4.0 wt. eq., all weight equivalents with regard to the *N*-Fmoc-doxorubicin starting material), glutaric acid (518 mg, 10 eq.). The reaction flask was fitted with a column and condenser assembly in which the column was filled with 3 Å molecular sieves. The reaction mixture was stirred vigorously and heated at approx. 90 °C. The progress of the reaction was monitored by HPLC. The results are summarized in the following table:

| Example | Amount of lipase (*Candida Antarctica B*) | Time (h) | *N*-Fmoc-doxorubicin-*O-*hemiglutarate (%, HPLC) |
|---|---|---|---|
| 2h | 4.0 wt. eq. | 4 | 91 |
| 2i | 3.5 wt. eq. | 6 | 92 |
| 2j | 2.5 wt. eq. | 7 | 90 |
| 2k | 2.0 wt. eq. | 16 | 89 |
| 21 | 1.0 wt. eq. | 48 | 71 |
| 2m | 0.5 wt. eq. | 48 | 43 |

It is shown by these examples, that the amount of weight equivalents of added lipase with regard to *N*-Fmoc-doxorubicin is an important factor in order to arrive at an optimal yield.

### Examples 2n-p: Synthesis of N-Fmoc-doxorubicin-O-hemiglutarate using different amounts of glutaric acid

*N*-Fmoc-doxorubicin (300 mg) was suspended in MTBE/Me-THF 1:2 (v/v) (18 mL) and to this was added lipase acrylic resin from *Candida Antarctica B* (1200 mg, 4 wt. eq., all weight equivalents with regard to the *N*-Fmoc-doxorubicin starting material), glutaric acid (2.5, 5.0 or 10.0 eq.). The reaction flask was fitted with a column and condenser assembly in which the column was filled with 3 Å molecular sieves. The reaction mixture was stirred vigorously and heated at approx. 90 °C. The progress of the reaction was monitored by HPLC. The results are summarized in the following table:

| Example | Amounts of glutaric acid | Time (h) | *N*-Fmoc-doxorubicin (Formula II) (%, HPLC) | *N*-Fmoc-doxorubicin-*O-*hemiglutarate (%, HPLC) |
|---|---|---|---|---|
| 2n | 10.0 eq. | 4 | 2.5 | 91.1 |
| 2o | 5.0 eq. | 4 | 8.1 | 85.3 |
| 2p | 2.5 eq. | 4 | 7.9 | 82.4 |

It is shown by these examples, that the amount of weight equivalents of added glutaric acid with regard to *N*-Fmoc-doxorubicin is an important factor in order to arrive at an optimal yield.

### Examples 2q-r: Synthesis of N-Fmoc-doxorubicin-O-hemiglutarate using different polymorph forms of N-Fmoc-doxorubicin

Amorphous (XRPD pattern see Appendix 19) or crystalline (XRPD pattern see Appendix 20) *N*-Fmoc-doxorubicin (300 mg) was suspended in MTBE/Me-THF 1:2 (v/v) (18 mL) and to this was added lipase acrylic resin from *Candida Antarctica B* (1200 mg, 4 wt. eq., all weight equivalents with regard to the *N*-Fmoc-doxorubicin starting material), glutaric acid (518 mg, 10 eq.). The reaction flask was fitted with a column and condenser assembly in which the column was filled with 3 Å molecular sieves. The reaction mixture was stirred vigorously and heated at approx. 90 °C. The progress of the reaction was monitored by HPLC. The results are summarized in the following table:

| Example | Polymorph form | Time (h) | *N*-Fmoc-doxorubicin-*O-*hemiglutarate (%, HPLC) | Appendix |
|---|---|---|---|---|
| 2q | amorphous | 4 | 91 | 19 |
| 2r | crystalline | 4 | 92 | 20 |

It is shown by these examples, that the solid state of the *N*-Fmoc-doxorubicin is not critical to the process of the invention.

### Further inventive examples

The examples below can be synthesized analogously to Example 1d.

| | | |
|---|---|---|
| **Example 3** | *N-*Fmoc-doxorubicin-*O-*hemisuccinate | 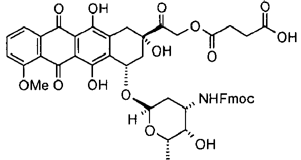 |
| **Example 4** | *N-*Fmoc-doxorubicin-*O-*hemipimelate | 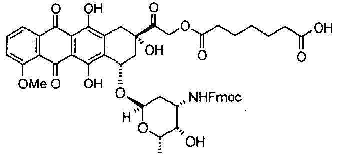 |
| **Example 5** | *N*-Fmoc-doxorubicin-*O-*hemifumarate | 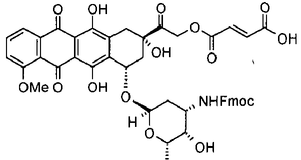 |
| **Example 6** | *N*-Fmoc-doxorubicin-*O*-hemi-3-(2-carboxyethoxy)propionate | 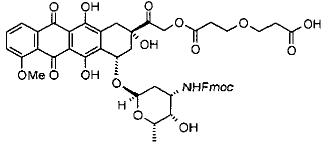 |
| **Example 7** | *N-*Fmoc-doxorubicin-*O*-hemiadipate | 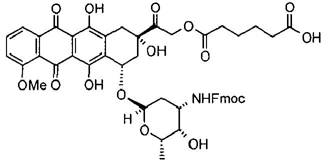 |
| **Example 8** | *N-*Fmoc-doxorubicin-*O-*hemisuberate | 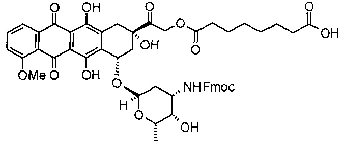 |
| **Example 9** | *N-*Fmoc-doxorubicin-*O*-hemiazelate | 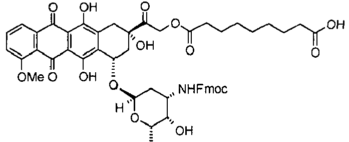 |
| **Example 10** | *N-*Fmoc-doxorubicin-*O-*hemisebacate | 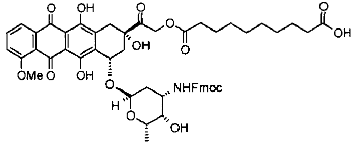 |
| **Example 11** | *N*-Fmoc-doxorubicin-*O-*hemiundecanedioate | 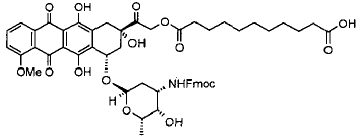 |
| **Example 12** | *N*-Fmoc-doxorubicin-*O-*hemidodecanedioate | 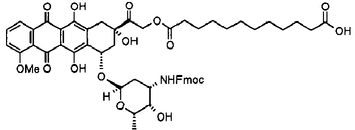 |
| **Example 13** | *N*-Fmoc-doxorubicin-*O*-hemimaleate | 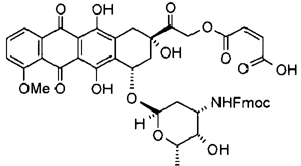 |
| **Example 14** | *N-*Fmoc-doxorubicin-*O-*hemiterephthalate | 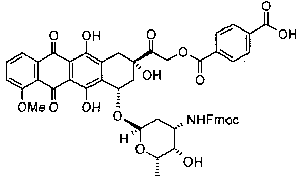 |
| **Example 15** | *N-*Fmoc-doxorubicin-*O*-hemi-(2-carboxyethyldisulfanyl)propionate | 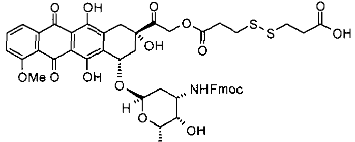 |

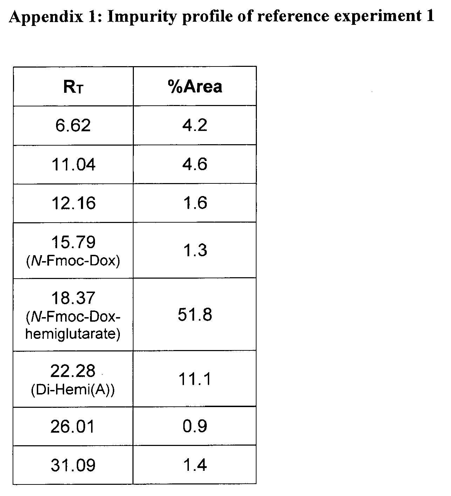

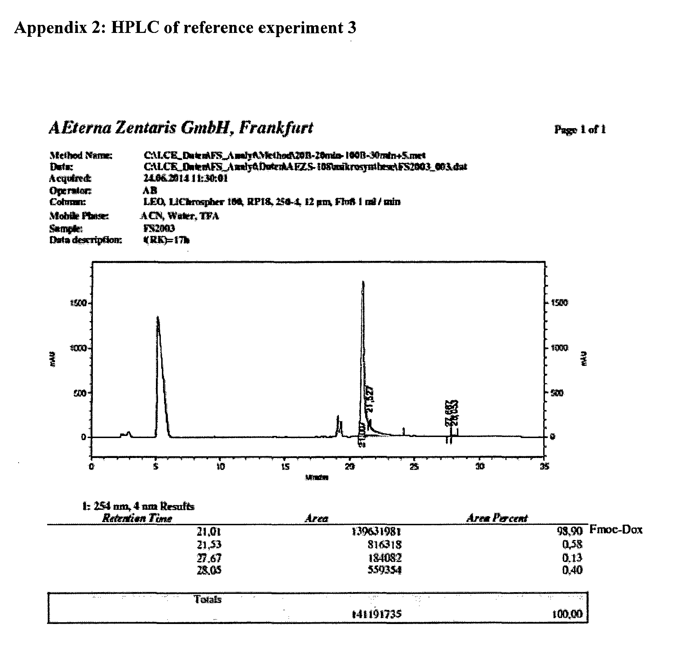

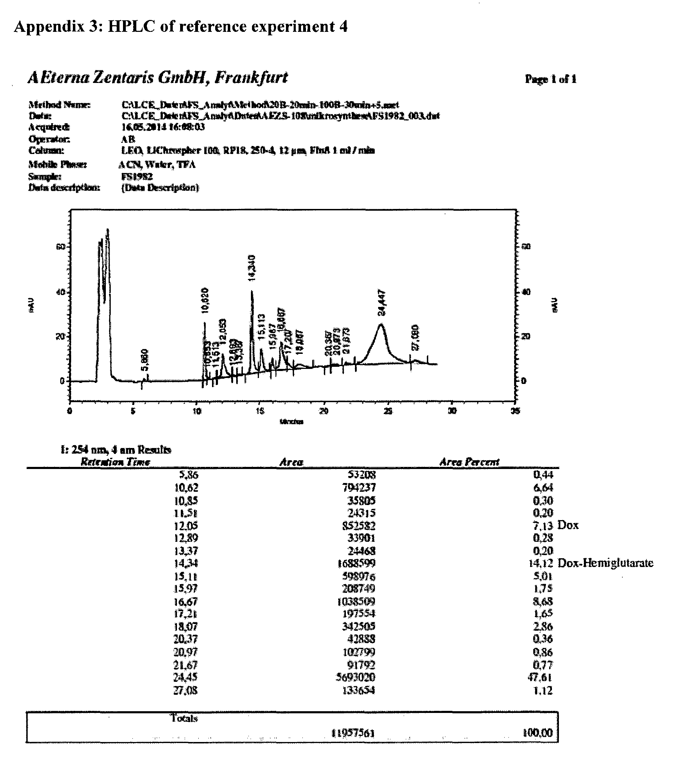

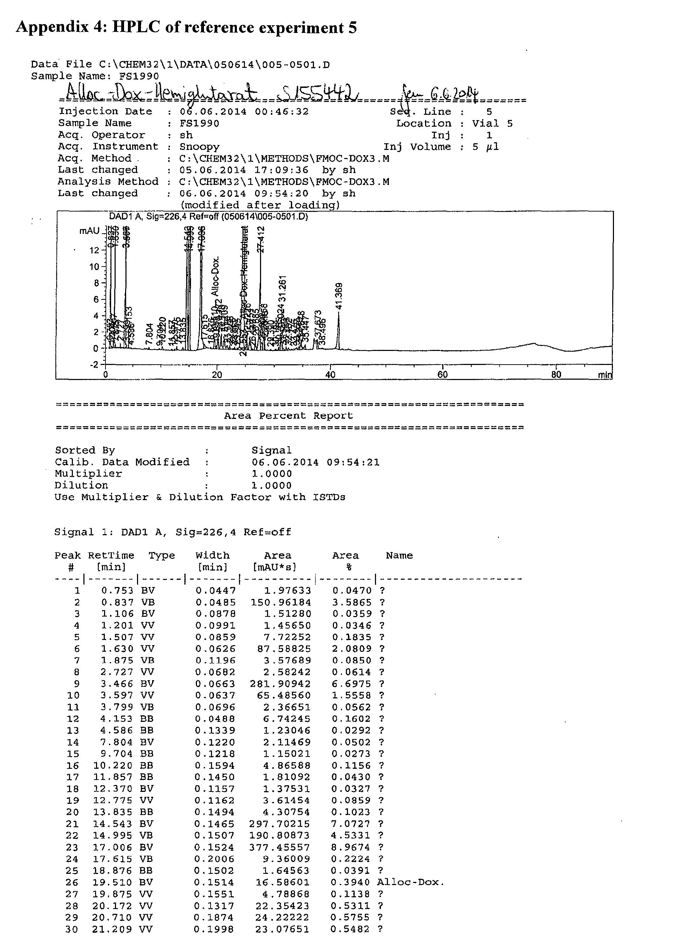

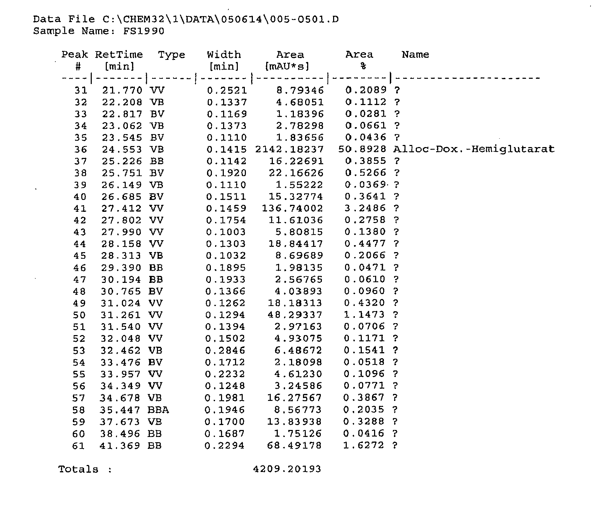

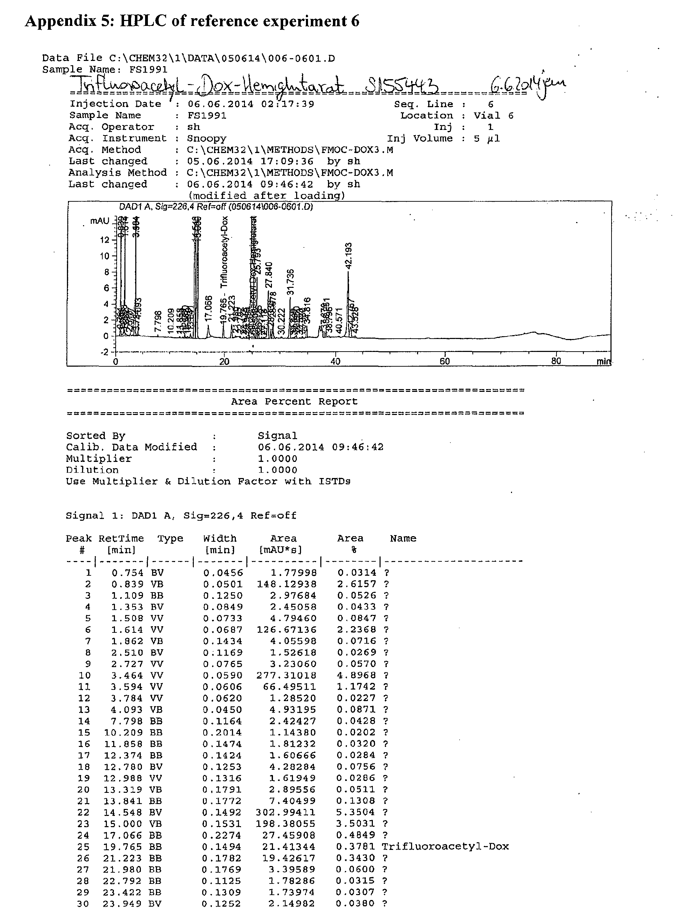

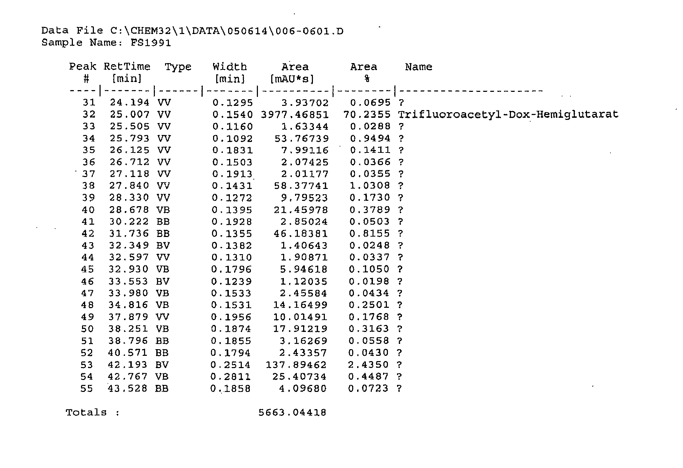

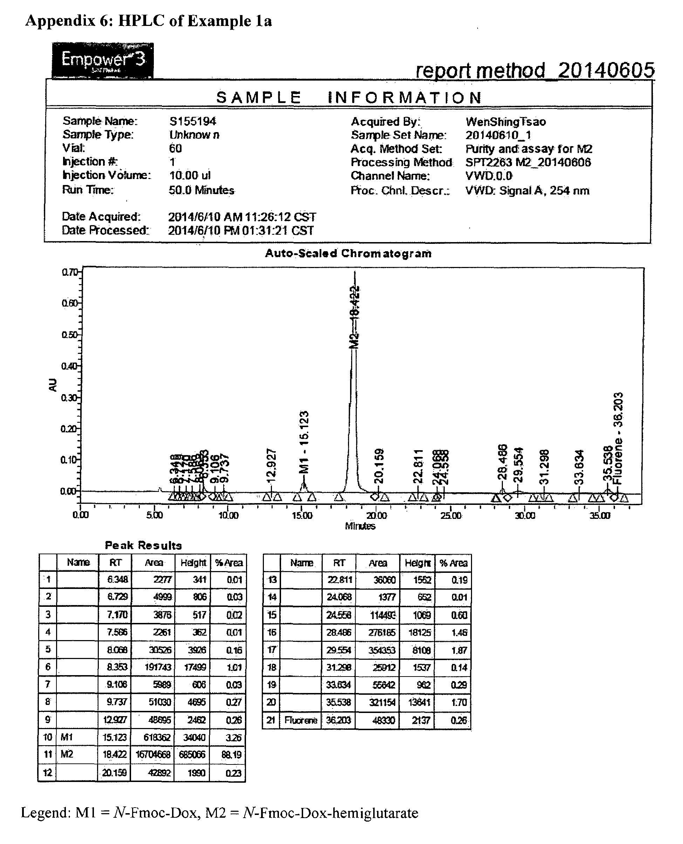

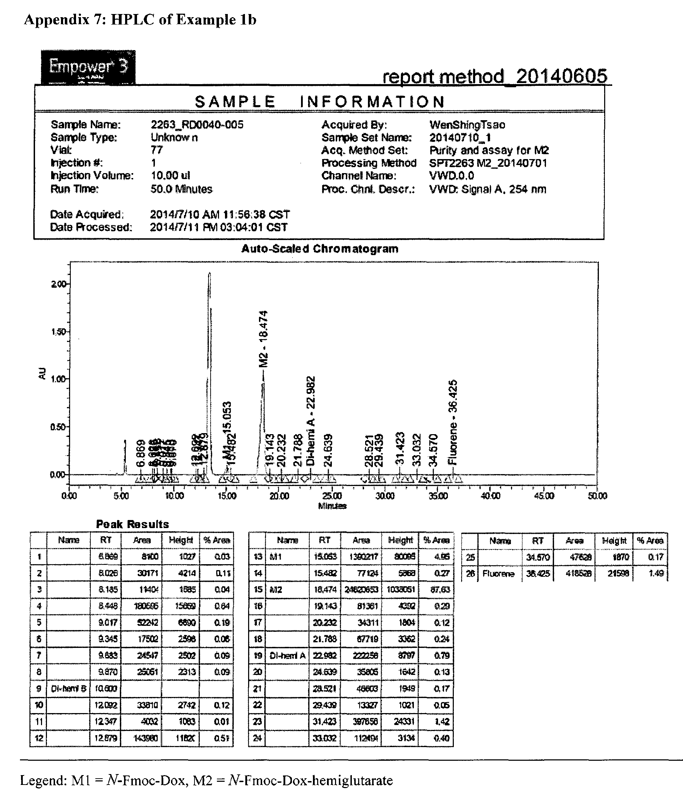

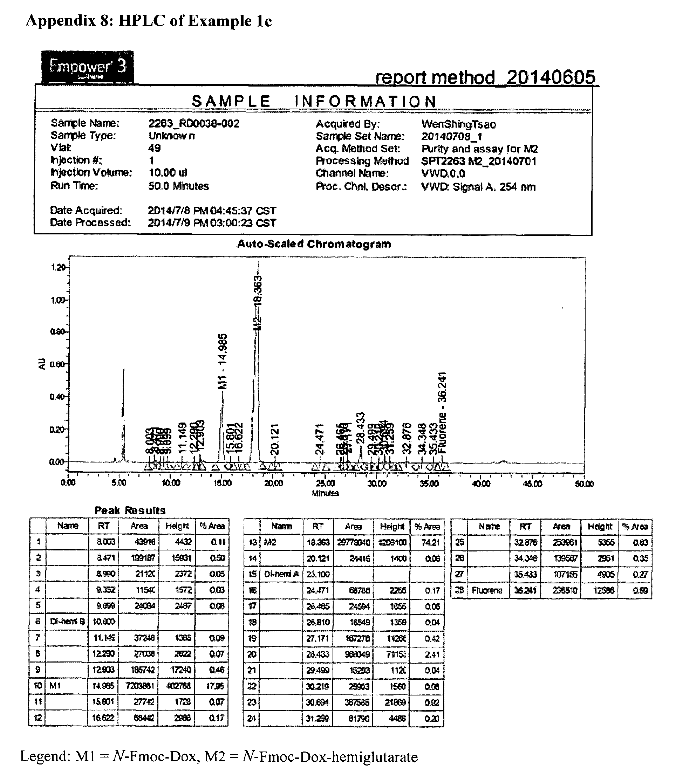

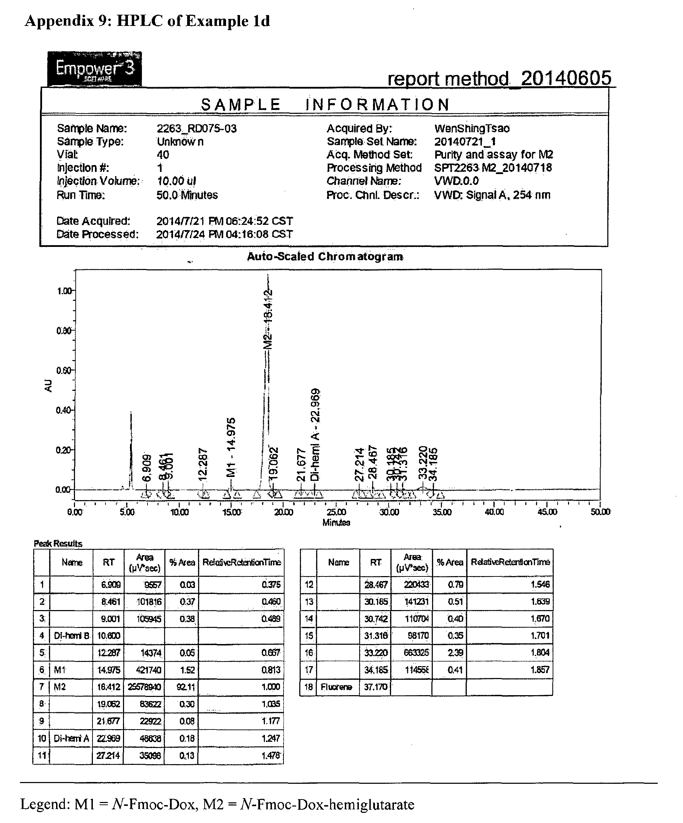

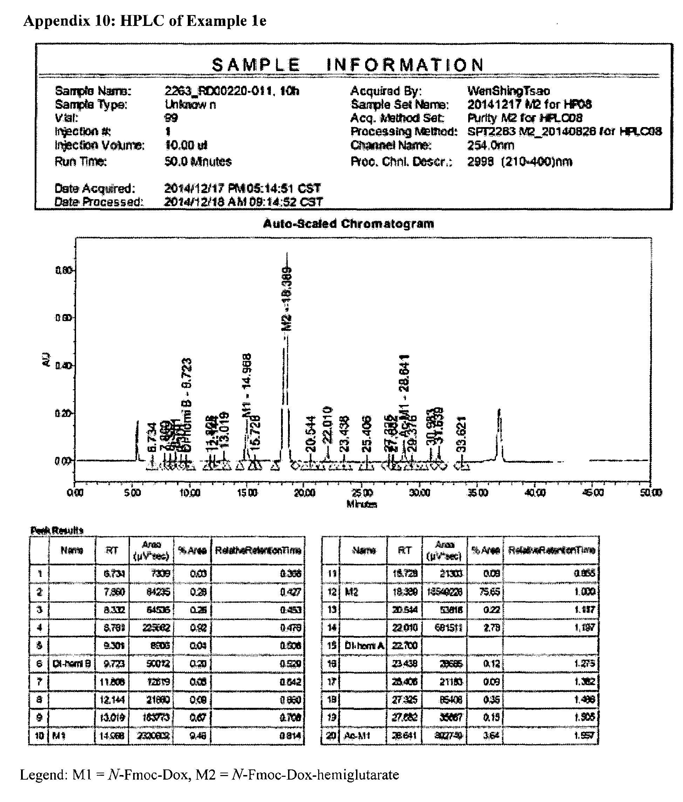

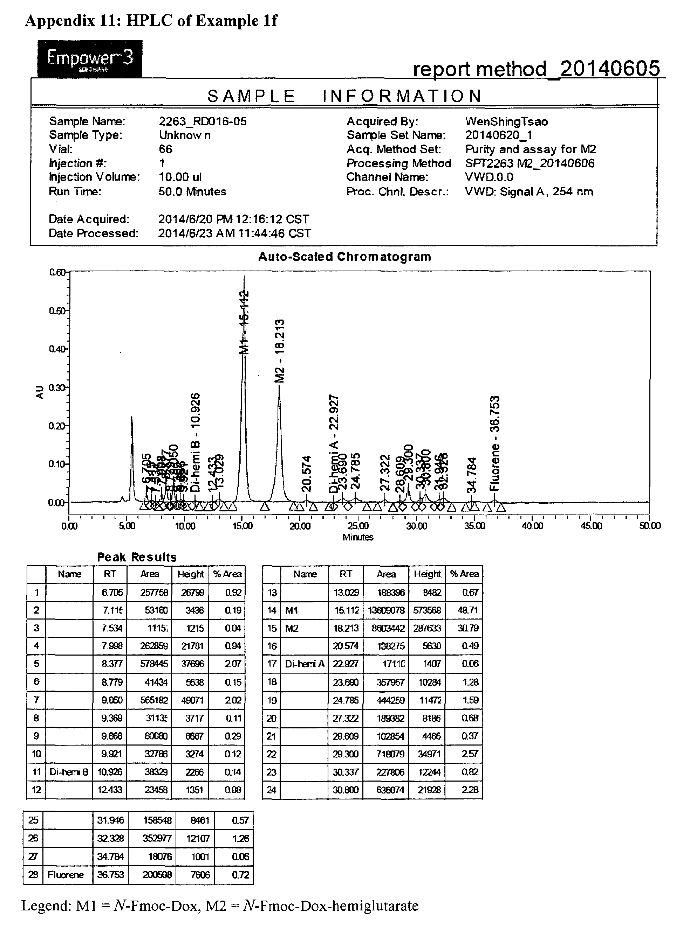

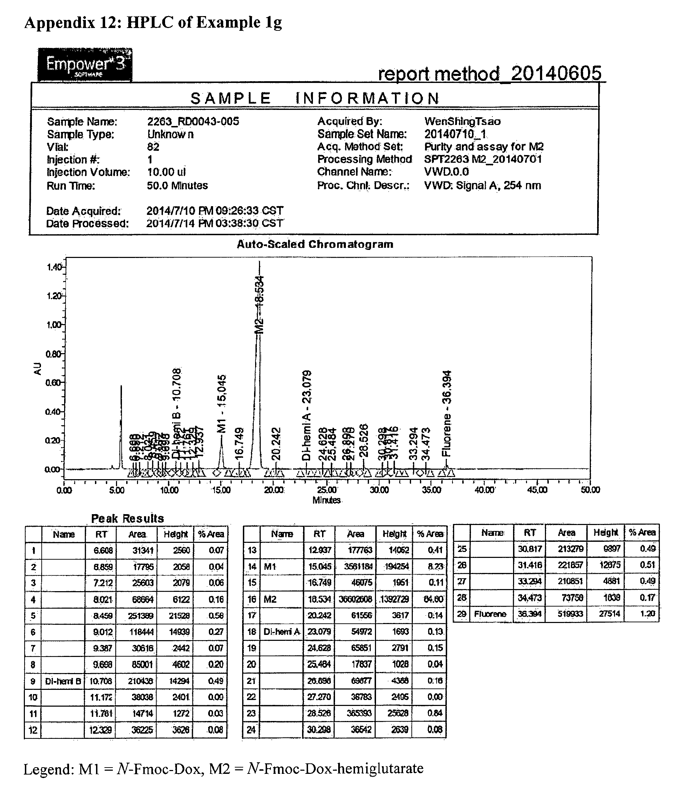

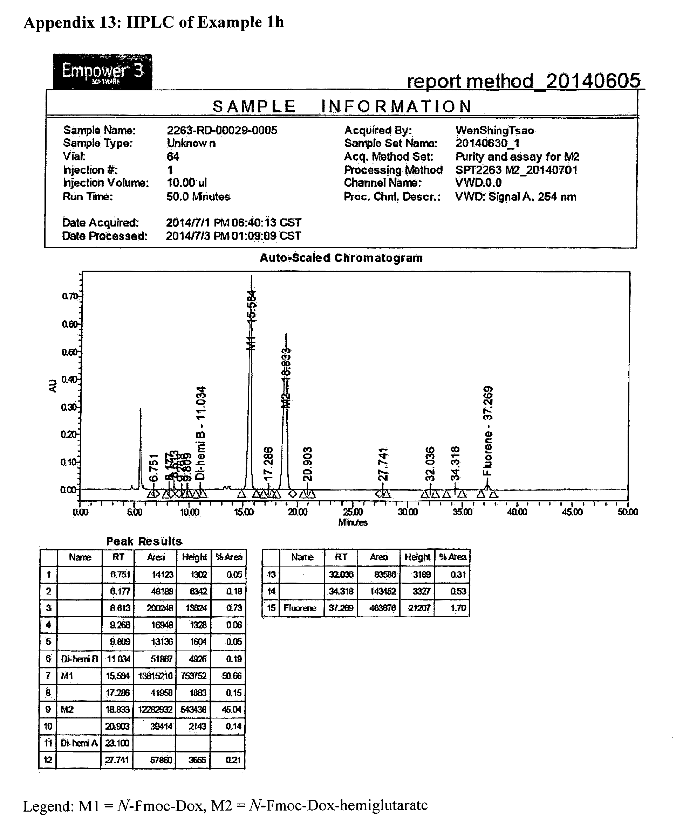

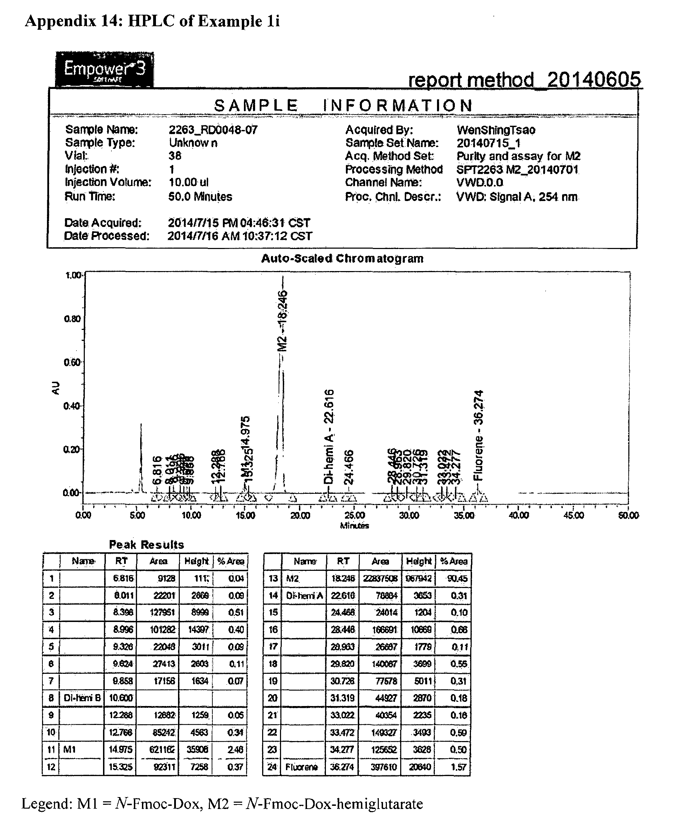

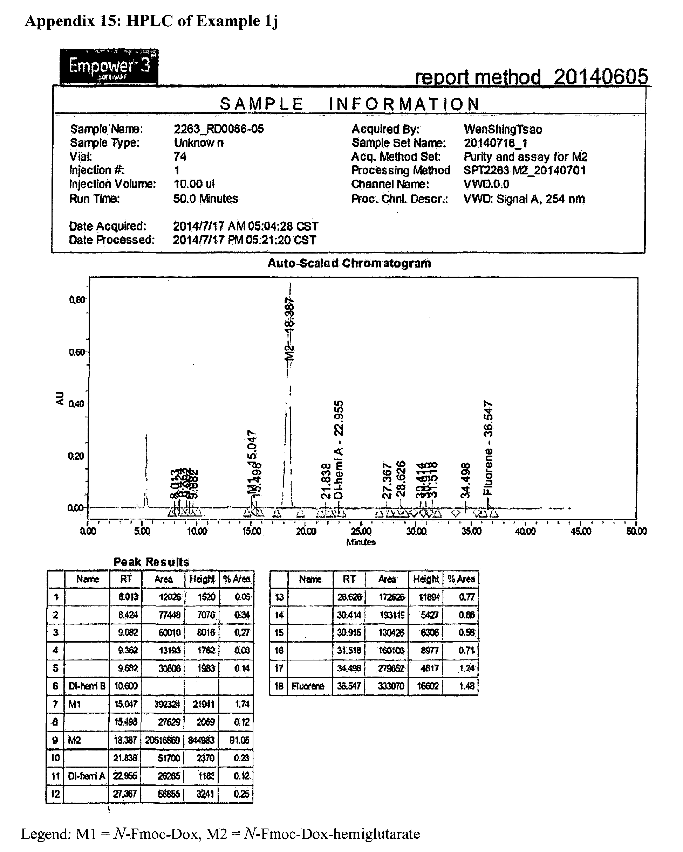

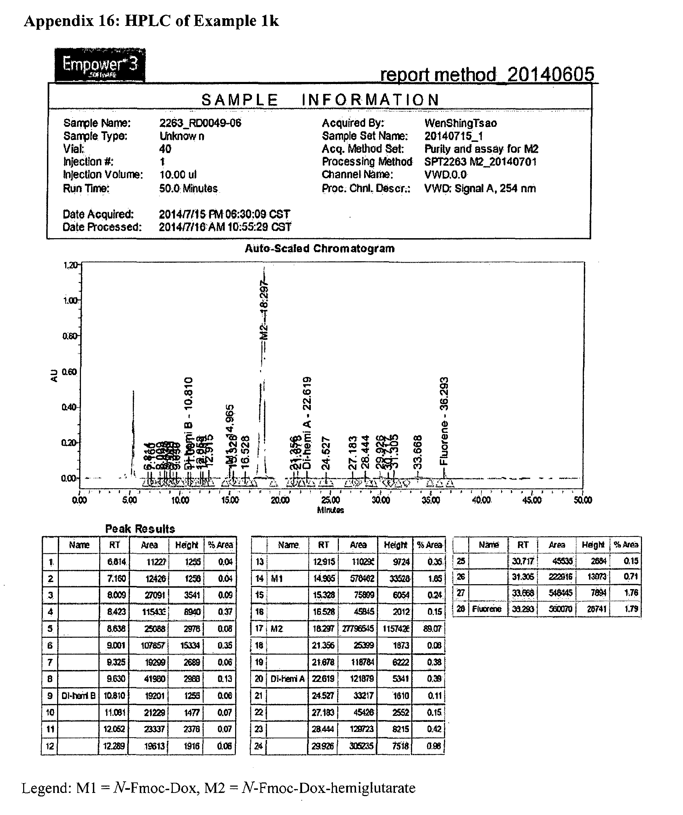

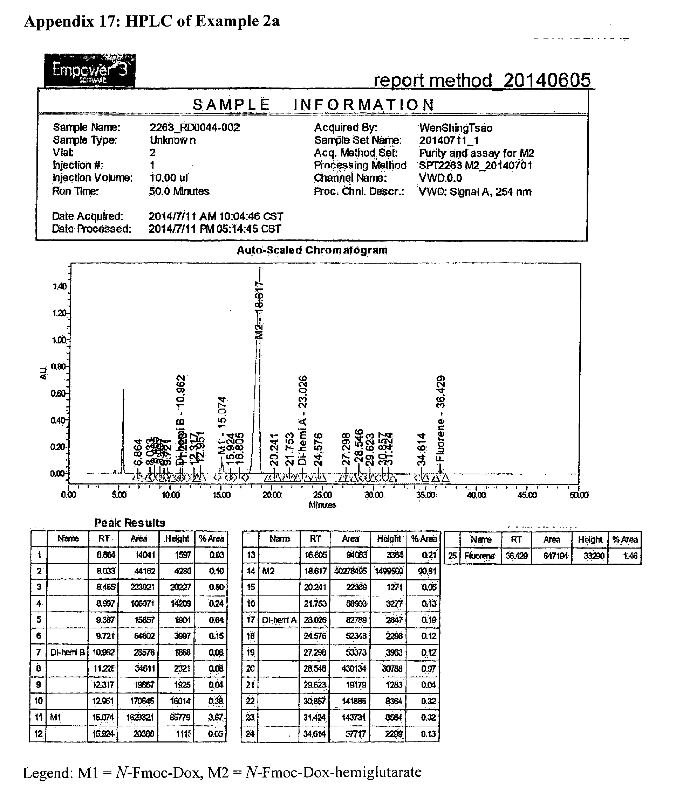

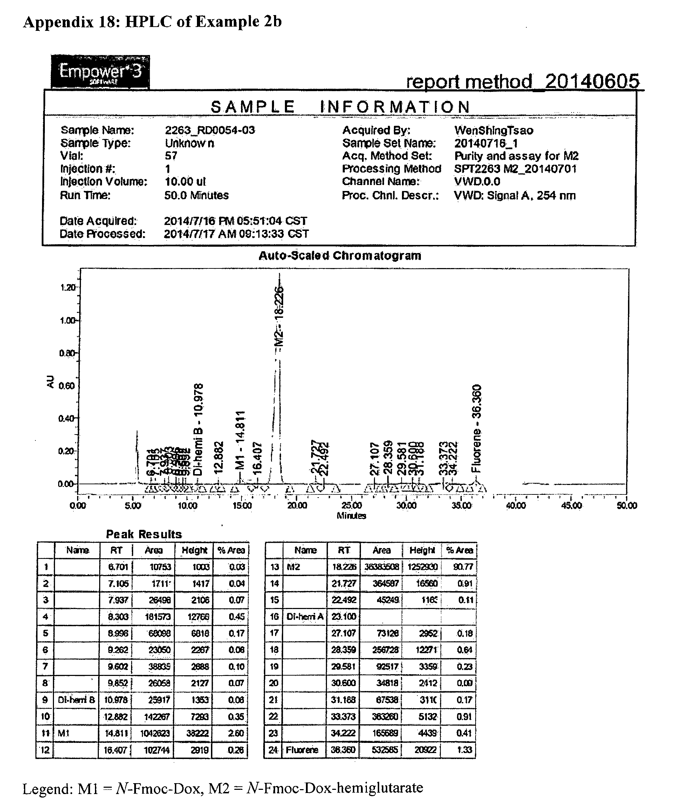

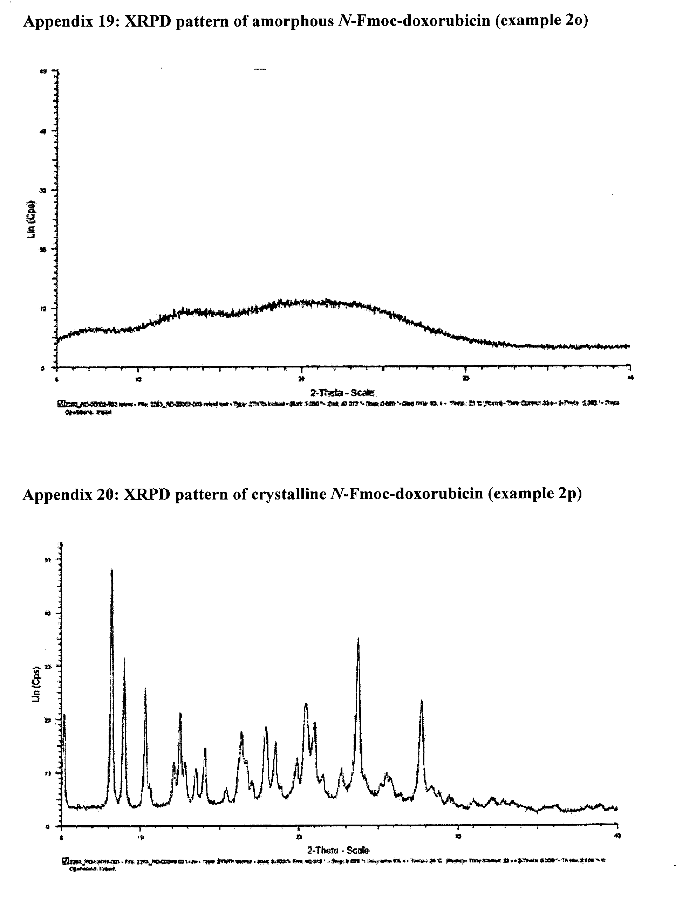

## Claims

1. A process for preparation of an *N-*Fmoc-doxorubicin-14-*O*-dicarboxylic acid mono ester compound according to formula I or a pharmaceutically acceptable salt thereof, wherein R is a dicarboxylic group: 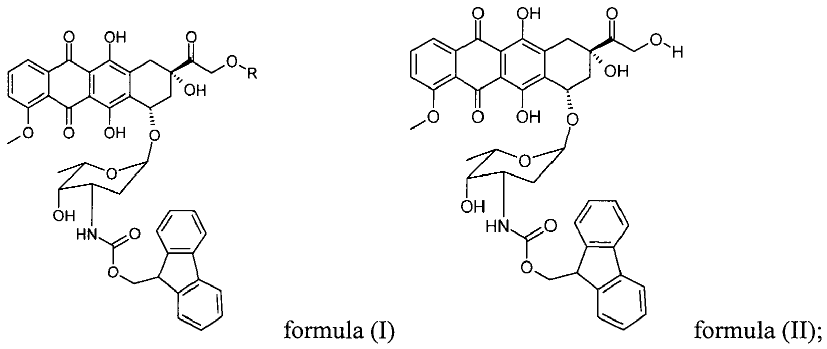 said process comprising reacting *N*-Fmoc-doxorubicin (formula II) with a bis-acyl donor compound in the presence of a lipase in liquid organic media to acylate said *N-*Fmoc-doxorubicin at its C 14-OH group, under conditions effective to remove water from the reaction mixture.

2. The process of claim 1, wherein the organic media is
(i) an organic solvent selected from a group (A) consisting of dichloromethane, chloroform, tetrachloromethane, benzene, toluene, diethyl ether, methyl-*tert-*butyl ether, diisopropyl ether, cyclopentyl methyl ether, acetone, methyl-ethylketone, methyl-isobutylketone, anisole, dimethylsulfoxide, *N*-methyl-2-pyrrolidone, 1,4-dioxanee, pyridine, acetonitrile, *N,N-*dimethyl formamide, tetrahydrofuran, 2-methyltetrahydrofuran, ethyl acetate, *n*-pentane, *n*-hexane, *n-*heptane, 2,2,4-trimethylpentane, cyclopentane, cyclohexane, cycloheptane, *tert-*butanol, *tert*-amyl alcohol;
(ii) a mixture comprising a first organic solvent from group (B) selected from methyl-*tert*-butyl ether, toluene, benzene, cyclopentyl methyl ether, acetonitrile, tetrahydrofuran, 2-methyltetrahydrofuran, methyl-ethylketone, ethyl acetate, 1,4-dioxanee, *n*-hexane, *n*-heptane, *n*-pentane, or 2,2,4-trimethyl pentane; and a second organic solvent from group (C) selected from methyl-ethylketone, acetone, cyclopentyl methyl ether, anisole, tetrahydrofuran, 2-methyltetrahydrofuran, methyl-isobutylketone, *N*-methyl-2-pyrrolidone, *tert*-amyl alcohol, *tert*-butanol, cyclopentane or diethyl ether;
(iii) a mixture comprising a first organic solvent from group (B), a second organic solvent from group (C) and a third organic solvent from group (B) or group (C); or
(vi) a higher order mixture, comprising of solvents selected from groups (A), (B) or (C).

3. The process of claim 1, wherein the organic media is a solvent system selected from
a) mixtures with MTBE:
a1 MTBE/MEK in a ratio (v/v) covered by the range from 6:1 to 1:6, preferably from 4:1 to 1:4, more preferably from 3:1 to 1:3, further more preferably from 1:1 to 1:3, and most preferably the ratio is selected from the group consisting of 6:1, 5:1, 4:1, 3:1, 2:1, 1:1, 1:2, 1:3, 1:4, 1:5, or 1:6
a2 MTBE/acetone in a ratio (v/v) covered by the range from 6:1 to 1:6, preferably from 4:1 to 1:4, more preferably from 3:1 to 1:3, further more preferably from 1:1 to 1:3, and most preferably the ratio is selected from the group consisting of 6:1, 5:1, 4:1, 3:1, 2:1, 1:1, 1:2, 1:3, 1:4, 1:5, or 1:6
a3 MTBE/CPME in a ratio (v/v) covered by the range from 6:1 to 1:6, preferably from 4:1 to 1:4, more preferably from 3:1 to 1:3, further more preferably from 1:1 to 1:3, and most preferably the ratio is selected from the group consisting of 6:1, 5:1, 4:1, 3:1, 2:1, 1:1, 1:2, 1:3, 1:4, 1:5, or 1:6
a4 MTBE/anisole in a ratio (v/v) covered by the range from 6:1 to 1:6, preferably from 4:1 to 1:4, more preferably from 3:1 to 1:3, further more preferably from 1:1 to 1:3, and most preferably the ratio is selected from the group consisting of 6:1, 5:1, 4:1, 3:1, 2:1, 1:1, 1:2, 1:3, 1:4, 1:5, or 1:6
a5 MTBE/THF in a ratio (v/v) covered by the range from 6:1 to 1:6, preferably from 4:1 to 1:4, more preferably from 3:1 to 1:3, further more preferably from 1:1 to 1:3, and most preferably the ratio is selected from the group consisting of 6:1, 5:1, 4:1, 3:1, 2:1, 1:1, 1:2, 1:3, 1:4, 1:5, or 1:6
a6 MTBE/Me-THF in a ratio (v/v) covered by the range from 6:1 to 1:6, preferably from 4:1 to 1:4, more preferably from 3:1 to 1:3, further more preferably from 1:1 to 1:3, and most preferably the ratio is selected from the group consisting of 6:1, 5:1, 4:1, 3:1, 2:1, 1:1, 1:2, 1:3, 1:4, 1:5, or 1:6
a7 MTBE/MIBK in a ratio (v/v) covered by the range from 6:1 to 1:6, preferably from 4:1 to 1:4, more preferably from 3:1 to 1:3, further more preferably from 1:1 to 1:3, and most preferably the ratio is selected from the group consisting of 6:1, 5:1, 4:1, 3:1, 2:1, 1:1, 1:2, 1:3, 1:4, 1:5, or 1:6
a8 MTBE/NMP in a ratio (v/v) covered by the range from 6:1 to 1:6, preferably from 4:1 to 1:4, more preferably from 3:1 to 1:3, further more preferably from 1:1 to 1:3, and most preferably the ratio is selected from the group consisting of 6:1, 5:1, 4:1, 3:1, 2:1, 1:1, 1:2, 1:3, 1:4, 1:5, or 1:6
a9 MTBE/tAmylOH in a ratio (v/v) covered by the range from 6:1 to 1:6, preferably from 4:1 to 1:4, more preferably from 3:1 to 1:3, further more preferably from 1:1 to 1:3, and most preferably the ratio is selected from the group consisting of 6:1, 5:1, 4:1, 3:1, 2:1, 1:1, 1:2, 1:3, 1:4, 1:5, or 1:6
a10 MTBE/tBuOH in a ratio (v/v) covered by the range from 6:1 to 1:6, preferably from 4:1 to 1:4, more preferably from 3:1 to 1:3, further more preferably from 1:1 to 1:3, and most preferably the ratio is selected from the group consisting of 6:1, 5:1, 4:1, 3:1, 2:1, 1:1, 1:2, 1:3, 1:4, 1:5, or 1:6
a11 MTBE/cyclopentane in a ratio (v/v) covered by the range from 6:1 to 1:6, preferably from 4:1 to 1:4, more preferably from 3:1 to 1:3, further more preferably from 1:1 to 1:3, and most preferably the ratio is selected from the group consisting of 6:1, 5:1, 4:1, 3:1, 2:1, 1:1, 1:2, 1:3, 1:4, 1:5, or 1:6
a12 MTBE/diethyl ether in a ratio (v/v) covered by the range from 6:1 to 1:6, preferably from 4:1 to 1:4, more preferably from 3:1 to 1:3, further more preferably from 1:1 to 1:3, and most preferably the ratio is selected from the group consisting of 6:1, 5:1, 4:1, 3:1, 2:1, 1:1, 1:2, 1:3, 1:4, 1:5, or 1:6
b) mixtures with toluene:
b1 Toluene/MEK in a ratio (v/v) covered by the range from 6:1 to 1:6, preferably from 4:1 to 1:4, more preferably from 3:1 to 1:3, further more preferably from 1:1 to 1:3, and most preferably the ratio is selected from the group consisting of 6:1, 5:1, 4:1, 3:1, 2:1, 1:1, 1:2, 1:3, 1:4, 1:5, or 1:6
b2 Toluene/acetone in a ratio (v/v) covered by the range from 6:1 to 1:6, preferably from 4:1 to 1:4, more preferably from 3:1 to 1:3, further more preferably from 1:1 to 1:3, and most preferably the ratio is selected from the group consisting of 6:1, 5:1, 4:1, 3:1, 2:1, 1:1, 1:2, 1:3, 1:4, 1:5, or 1:6
b3 Toluene/CPME in a ratio (v/v) covered by the range from 6:1 to 1:6, preferably from 4:1 to 1:4, more preferably from 3:1 to 1:3, further more preferably from 1:1 to 1:3, and most preferably the ratio is selected from the group consisting of 6:1, 5:1, 4:1, 3:1, 2:1, 1:1, 1:2, 1:3, 1:4, 1:5, or 1:6
b4 Toluene/anisole in a ratio (v/v) covered by the range from 6:1 to 1:6, preferably from 4:1 to 1:4, more preferably from 3:1 to 1:3, further more preferably from 1:1 to 1:3, and most preferably the ratio is selected from the group consisting of 6:1, 5:1, 4:1, 3:1, 2:1, 1:1, 1:2, 1:3, 1:4, 1:5, or 1:6
b5 Toluene/THF in a ratio (v/v) covered by the range from 6:1 to 1:6, preferably from 4:1 to 1:4, more preferably from 3:1 to 1:3, further more preferably from 1:1 to 1:3, and most preferably the ratio is selected from the group consisting of 6:1, 5:1, 4:1, 3:1, 2:1, 1:1, 1:2, 1:3, 1:4, 1:5, or 1:6
b6 Toluene/Me-THF in a ratio (v/v) covered by the range from 6:1 to 1:6, preferably from 4:1 to 1:4, more preferably from 3:1 to 1:3, further more preferably from 1:1 to 1:3, and most preferably the ratio is selected from the group consisting of 6:1, 5:1, 4:1, 3:1, 2:1, 1:1, 1:2, 1:3, 1:4, 1:5, or 1:6
b7 Toluene/MIBK in a ratio (v/v) covered by the range from 6:1 to 1:6, preferably from 4:1 to 1:4, more preferably from 3:1 to 1:3, further more preferably from 1:1 to 1:3, and most preferably the ratio is selected from the group consisting of 6:1, 5:1, 4:1, 3:1, 2:1, 1:1, 1:2, 1:3, 1:4, 1:5, or 1:6
b8 Toluene/NMP in a ratio (v/v) covered by the range from 6:1 to 1:6, preferably from 4:1 to 1:4, more preferably from 3:1 to 1:3, further more preferably from 1:1 to 1:3, and most preferably the ratio is selected from the group consisting of 6:1, 5:1, 4:1, 3:1, 2:1, 1:1, 1:2, 1:3, 1:4, 1:5, or 1:6
b9 Toluene/tAmylOH in a ratio (v/v) covered by the range from 6:1 to 1:6, preferably from 4:1 to 1:4, more preferably from 3:1 to 1:3, further more preferably from 1:1 to 1:3, and most preferably the ratio is selected from the group consisting of 6:1, 5:1, 4:1, 3:1, 2:1, 1:1, 1:2, 1:3, 1:4, 1:5, or 1:6
b10 Toluene/tBuOH in a ratio (v/v) covered by the range from 6:1 to 1:6, preferably from 4:1 to 1:4, more preferably from 3:1 to 1:3, further more preferably from 1:1 to 1:3, and most preferably the ratio is selected from the group consisting of 6:1, 5:1, 4:1, 3:1, 2:1, 1:1, 1:2, 1:3, 1:4, 1:5, or 1:6
b11 Toluene/cyclopentane in a ratio (v/v) covered by the range from 6:1 to 1:6, preferably from 4:1 to 1:4, more preferably from 3:1 to 1:3, further more preferably from 1:1 to 1:3, and most preferably the ratio is selected from the group consisting of 6:1, 5:1, 4:1, 3:1, 2:1, 1:1, 1:2, 1:3, 1:4, 1:5, or 1:6
b12 Toluene/diethyl ether in a ratio (v/v) covered by the range from 6:1 to 1:6, preferably from 4:1 to 1:4, more preferably from 3:1 to 1:3, further more preferably from 1:1 to 1:3, and most preferably the ratio is selected from the group consisting of 6:1, 5:1, 4:1, 3:1, 2:1, 1:1, 1:2, 1:3, 1:4, 1:5, or 1:6
c) mixtures with benzene:
c1 Benzene/MEK in a ratio (v/v) covered by the range from 6:1 to 1:6, preferably from 4:1 to 1:4, more preferably from 3:1 to 1:3, further more preferably from 1:1 to 1:3, and most preferably the ratio is selected from the group consisting of 6:1, 5:1, 4:1, 3:1, 2:1, 1:1, 1:2, 1:3, 1:4, 1:5, or 1:6
c2 Benzene/acetone in a ratio (v/v) covered by the range from 6:1 to 1:6, preferably from 4:1 to 1:4, more preferably from 3:1 to 1:3, further more preferably from 1:1 to 1:3, and most preferably the ratio is selected from the group consisting of 6:1, 5:1, 4:1, 3:1, 2:1, 1:1, 1:2, 1:3, 1:4, 1:5, or 1:6
c3 Benzene/CPME in a ratio (v/v) covered by the range from 6:1 to 1:6, preferably from 4:1 to 1:4, more preferably from 3:1 to 1:3, further more preferably from 1:1 to 1:3, and most preferably the ratio is selected from the group consisting of 6:1, 5:1, 4:1, 3:1, 2:1, 1:1, 1:2, 1:3, 1:4, 1:5, or 1:6
c4 Benzene/anisole in a ratio (v/v) covered by the range from 6:1 to 1:6, preferably from 4:1 to 1:4, more preferably from 3:1 to 1:3, further more preferably from 1:1 to 1:3, and most preferably the ratio is selected from the group consisting of 6:1, 5:1, 4:1, 3:1, 2:1, 1:1, 1:2, 1:3, 1:4, 1:5, or 1:6
c5 Benzene/THF in a ratio (v/v) covered by the range from 6:1 to 1:6, preferably from 4:1 to 1:4, more preferably from 3:1 to 1:3, further more preferably from 1:1 to 1:3, and most preferably the ratio is selected from the group consisting of 6:1, 5:1, 4:1, 3:1, 2:1, 1:1, 1:2, 1:3, 1:4, 1:5, or 1:6
c6 Benzene/Me-THF in a ratio (v/v) covered by the range from 6:1 to 1:6, preferably from 4:1 to 1:4, more preferably from 3:1 to 1:3, further more preferably from 1:1 to 1:3, and most preferably the ratio is selected from the group consisting of 6:1, 5:1, 4:1, 3:1, 2:1, 1:1, 1:2, 1:3, 1:4, 1:5, or 1:6
c7 Benzene/MIBK in a ratio (v/v) covered by the range from 6:1 to 1:6, preferably from 4:1 to 1:4, more preferably from 3:1 to 1:3, further more preferably from 1:1 to 1:3, and most preferably the ratio is selected from the group consisting of 6:1, 5:1, 4:1, 3:1, 2:1, 1:1, 1:2, 1:3, 1:4, 1:5, or 1:6
c8 Benzene/NMP in a ratio (v/v) covered by the range from 6:1 to 1:6, preferably from 4:1 to 1:4, more preferably from 3:1 to 1:3, further more preferably from 1:1 to 1:3, and most preferably the ratio is selected from the group consisting of 6:1, 5:1, 4:1, 3:1, 2:1, 1:1, 1:2, 1:3, 1:4, 1:5, or 1:6
c9 Benzene/tAmylOH in a ratio (v/v) covered by the range from 6:1 to 1:6, preferably from 4:1 to 1:4, more preferably from 3:1 to 1:3, further more preferably from 1:1 to 1:3, and most preferably the ratio is selected from the group consisting of 6:1, 5:1, 4:1, 3:1, 2:1, 1:1, 1:2, 1:3, 1:4, 1:5, or 1:6
c10 Benzene/tBuOH in a ratio (v/v) covered by the range from 6:1 to 1:6, preferably from 4:1 to 1:4, more preferably from 3:1 to 1:3, further more preferably from 1:1 to 1:3, and most preferably the ratio is selected from the group consisting of 6:1, 5:1, 4:1, 3:1, 2:1, 1:1, 1:2, 1:3, 1:4, 1:5, or 1:6
c11 Benzene/cyclopentane in a ratio (v/v) covered by the range from 6:1 to 1:6, preferably from 4:1 to 1:4, more preferably from 3:1 to 1:3, further more preferably from 1:1 to 1:3, and most preferably the ratio is selected from the group consisting of 6:1, 5:1, 4:1, 3:1, 2:1, 1:1, 1:2, 1:3, 1:4, 1:5, or 1:6
c12 Benzene/diethyl ether in a ratio (v/v) covered by the range from 6:1 to 1:6, preferably from 4:1 to 1:4, more preferably from 3:1 to 1:3, further more preferably from 1:1 to 1:3, and most preferably the ratio is selected from the group consisting of 6:1, 5:1, 4:1, 3:1, 2:1, 1:1, 1:2, 1:3, 1:4, 1:5, or 1:6
d) mixtures with CPME:
d1 CPME/MEK in a ratio (v/v) covered by the range from 6:1 to 1:6, preferably from 4:1 to 1:4, more preferably from 3:1 to 1:3, further more preferably from 1:1 to 1:3, and most preferably the ratio is selected from the group consisting of 6:1, 5:1, 4:1, 3:1, 2:1, 1:1, 1:2, 1:3, 1:4, 1:5, or 1:6
d2 CPME/acetone in a ratio (v/v) covered by the range from 6:1 to 1:6, preferably from 4:1 to 1:4, more preferably from 3:1 to 1:3, further more preferably from 1:1 to 1:3, and most preferably the ratio is selected from the group consisting of 6:1, 5:1, 4:1, 3:1, 2:1, 1:1, 1:2, 1:3, 1:4, 1:5, or 1:6
d3 CPME/anisole in a ratio (v/v) covered by the range from 6:1 to 1:6, preferably from 4:1 to 1:4, more preferably from 3:1 to 1:3, further more preferably from 1:1 to 1:3, and most preferably the ratio is selected from the group consisting of 6:1, 5:1, 4:1, 3:1, 2:1, 1:1, 1:2, 1:3, 1:4, 1:5, or 1:6
d4 CPME/THF in a ratio (v/v) covered by the range from 6:1 to 1:6, preferably from 4:1 to 1:4, more preferably from 3:1 to 1:3, further more preferably from 1:1 to 1:3, and most preferably the ratio is selected from the group consisting of 6:1, 5:1, 4:1, 3:1, 2:1, 1:1, 1:2, 1:3, 1:4, 1:5, or 1:6
d5 CPME/Me-THF in a ratio (v/v) covered by the range from 6:1 to 1:6, preferably from 4:1 to 1:4, more preferably from 3:1 to 1:3, further more preferably from 1:1 to 1:3, and most preferably the ratio is selected from the group consisting of 6:1, 5:1, 4:1, 3:1, 2:1, 1:1, 1:2, 1:3, 1:4, 1:5, or 1:6
d6 CPME/MIBK in a ratio (v/v) covered by the range from 6:1 to 1:6, preferably from 4:1 to 1:4, more preferably from 3:1 to 1:3, further more preferably from 1:1 to 1:3, and most preferably the ratio is selected from the group consisting of 6:1, 5:1, 4:1, 3:1, 2:1, 1:1, 1:2, 1:3, 1:4, 1:5, or 1:6
d7 CPME/NMP in a ratio (v/v) covered by the range from 6:1 to 1:6, preferably from 4:1 to 1:4, more preferably from 3:1 to 1:3, further more preferably from 1:1 to 1:3, and most preferably the ratio is selected from the group consisting of 6:1, 5:1, 4:1, 3:1, 2:1, 1:1, 1:2, 1:3, 1:4, 1:5, or 1:6
d8 CPME/tAmylOH in a ratio (v/v) covered by the range from 6:1 to 1:6, preferably from 4:1 to 1:4, more preferably from 3:1 to 1:3, further more preferably from 1:1 to 1:3, and most preferably the ratio is selected from the group consisting of 6:1, 5:1, 4:1, 3:1, 2:1, 1:1, 1:2, 1:3, 1:4, 1:5, or 1:6
d9 CPME/tBuOH in a ratio (v/v) covered by the range from 6:1 to 1:6, preferably from 4:1 to 1:4, more preferably from 3:1 to 1:3, further more preferably from 1:1 to 1:3, and most preferably the ratio is selected from the group consisting of 6:1, 5:1, 4:1, 3:1, 2:1, 1:1, 1:2, 1:3, 1:4, 1:5, or 1:6
d10 CPME/cyclopentane in a ratio (v/v) covered by the range from 6:1 to 1:6, preferably from 4:1 to 1:4, more preferably from 3:1 to 1:3, further more preferably from 1:1 to 1:3, and most preferably the ratio is selected from the group consisting of 6:1, 5:1, 4:1, 3:1, 2:1, 1:1, 1:2, 1:3, 1:4, 1:5, or 1:6
d11 CPME/diethyl ether in a ratio (v/v) covered by the range from 6:1 to 1:6, preferably from 4:1 to 1:4, more preferably from 3:1 to 1:3, further more preferably from 1:1 to 1:3, and most preferably the ratio is selected from the group consisting of 6:1, 5:1, 4:1, 3:1, 2:1, 1:1, 1:2, 1:3, 1:4, 1:5, or 1:6
e) mixtures with MeCN:
e1 MeCN/MEK in a ratio (v/v) covered by the range from 6:1 to 1:6, preferably from 4:1 to 1:4, more preferably from 3:1 to 1:3, further more preferably from 1:1 to 1:3, and most preferably the ratio is selected from the group consisting of 6:1, 5:1, 4:1, 3:1, 2:1, 1:1, 1:2, 1:3, 1:4, 1:5, or 1:6
e2 MeCN/acetone in a ratio (v/v) covered by the range from 6:1 to 1:6, preferably from 4:1 to 1:4, more preferably from 3:1 to 1:3, further more preferably from 1:1 to 1:3, and most preferably the ratio is selected from the group consisting of 6:1, 5:1, 4:1, 3:1, 2:1, 1:1, 1:2, 1:3, 1:4, 1:5, or 1:6
e3 MeCN/CPME in a ratio (v/v) covered by the range from 6:1 to 1:6, preferably from 4:1 to 1:4, more preferably from 3:1 to 1:3, further more preferably from 1:1 to 1:3, and most preferably the ratio is selected from the group consisting of 6:1, 5:1, 4:1, 3:1, 2:1, 1:1, 1:2, 1:3, 1:4, 1:5, or 1:6
e4 MeCN/anisole in a ratio (v/v) covered by the range from 6:1 to 1:6, preferably from 4:1 to 1:4, more preferably from 3:1 to 1:3, further more preferably from 1:1 to 1:3, and most preferably the ratio is selected from the group consisting of 6:1, 5:1, 4:1, 3:1, 2:1, 1:1, 1:2, 1:3, 1:4, 1:5, or 1:6
e5 MeCN/THF in a ratio (v/v) covered by the range from 6:1 to 1:6, preferably from 4:1 to 1:4, more preferably from 3:1 to 1:3, further more preferably from 1:1 to 1:3, and most preferably the ratio is selected from the group consisting of 6:1, 5:1, 4:1, 3:1, 2:1, 1:1, 1:2, 1:3, 1:4, 1:5, or 1:6
e6 MeCN/Me-THF in a ratio (v/v) covered by the range from 6:1 to 1:6, preferably from 4:1 to 1:4, more preferably from 3:1 to 1:3, further more preferably from 1:1 to 1:3, and most preferably the ratio is selected from the group consisting of 6:1, 5:1, 4:1, 3:1, 2:1, 1:1, 1:2, 1:3, 1:4, 1:5, or 1:6
e7 MeCN/MIBK in a ratio (v/v) covered by the range from 6:1 to 1:6, preferably from 4:1 to 1:4, more preferably from 3:1 to 1:3, further more preferably from 1:1 to 1:3, and most preferably the ratio is selected from the group consisting of 6:1, 5:1, 4:1, 3:1, 2:1, 1:1, 1:2, 1:3, 1:4, 1:5, or 1:6
e8 MeCN/NMP in a ratio (v/v) covered by the range from 6:1 to 1:6, preferably from 4:1 to 1:4, more preferably from 3:1 to 1:3, further more preferably from 1:1 to 1:3, and most preferably the ratio is selected from the group consisting of 6:1, 5:1, 4:1, 3:1, 2:1, 1:1, 1:2, 1:3, 1:4, 1:5, or 1:6
e9 MeCN/tAmylOH in a ratio (v/v) covered by the range from 6:1 to 1:6, preferably from 4:1 to 1:4, more preferably from 3:1 to 1:3, further more preferably from 1:1 to 1:3, and most preferably the ratio is selected from the group consisting of 6:1, 5:1, 4:1, 3:1, 2:1, 1:1, 1:2, 1:3, 1:4, 1:5, or 1:6
e10 MeCN/tBuOH in a ratio (v/v) covered by the range from 6:1 to 1:6, preferably from 4:1 to 1:4, more preferably from 3:1 to 1:3, further more preferably from 1:1 to 1:3, and most preferably the ratio is selected from the group consisting of 6:1, 5:1, 4:1, 3:1, 2:1, 1:1, 1:2, 1:3, 1:4, 1:5, or 1:6
e11 MeCN/cyclopentane in a ratio (v/v) covered by the range from 6:1 to 1:6, preferably from 4:1 to 1:4, more preferably from 3:1 to 1:3, further more preferably from 1:1 to 1:3, and most preferably the ratio is selected from the group consisting of 6:1, 5:1, 4:1, 3:1, 2:1, 1:1, 1:2, 1:3, 1:4, 1:5, or 1:6
e12 MeCN/diethyl ether in a ratio (v/v) covered by the range from 6:1 to 1:6, preferably from 4:1 to 1:4, more preferably from 3:1 to 1:3, further more preferably from 1:1 to 1:3, and most preferably the ratio is selected from the group consisting of 6:1, 5:1, 4:1, 3:1, 2:1, 1:1, 1:2, 1:3, 1:4, 1:5, or 1:6
f) mixtures with THF:
f1 THF/MEK in a ratio (v/v) covered by the range from 6:1 to 1:6, preferably from 4:1 to 1:4, more preferably from 3:1 to 1:3, further more preferably from 1:1 to 1:3, and most preferably the ratio is selected from the group consisting of 6:1, 5:1, 4:1, 3:1, 2:1, 1:1, 1:2, 1:3, 1:4, 1:5, or 1:6
f2 THF/acetone in a ratio (v/v) covered by the range from 6:1 to 1:6, preferably from 4:1 to 1:4, more preferably from 3:1 to 1:3, further more preferably from 1:1 to 1:3, and most preferably the ratio is selected from the group consisting of 6:1, 5:1, 4:1, 3:1, 2:1, 1:1, 1:2, 1:3, 1:4, 1:5, or 1:6
f3 THF/CPME in a ratio (v/v) covered by the range from 6:1 to 1:6, preferably from 4:1 to 1:4, more preferably from 3:1 to 1:3, further more preferably from 1:1 to 1:3, and most preferably the ratio is selected from the group consisting of 6:1, 5:1, 4:1, 3:1, 2:1, 1:1, 1:2, 1:3, 1:4, 1:5, or 1:6
f4 THF/anisole in a ratio (v/v) covered by the range from 6:1 to 1:6, preferably from 4:1 to 1:4, more preferably from 3:1 to 1:3, further more preferably from 1:1 to 1:3, and most preferably the ratio is selected from the group consisting of 6:1, 5:1, 4:1, 3:1, 2:1, 1:1, 1:2, 1:3, 1:4, 1:5, or 1:6
f5 THF/Me-THF in a ratio (v/v) covered by the range from 6:1 to 1:6, preferably from 4:1 to 1:4, more preferably from 3:1 to 1:3, further more preferably from 1:1 to 1:3, and most preferably the ratio is selected from the group consisting of 6:1, 5:1, 4:1, 3:1, 2:1, 1:1, 1:2, 1:3, 1:4, 1:5, or 1:6
f6 THF/MIBK in a ratio (v/v) covered by the range from 6:1 to 1:6, preferably from 4:1 to 1:4, more preferably from 3:1 to 1:3, further more preferably from 1:1 to 1:3, and most preferably the ratio is selected from the group consisting of 6:1, 5:1, 4:1, 3:1, 2:1, 1:1, 1:2, 1:3, 1:4, 1:5, or 1:6
f7 THF/NMP in a ratio (v/v) covered by the range from 6:1 to 1:6, preferably from 4:1 to 1:4, more preferably from 3:1 to 1:3, further more preferably from 1:1 to 1:3, and most preferably the ratio is selected from the group consisting of 6:1, 5:1, 4:1, 3:1, 2:1, 1:1, 1:2, 1:3, 1:4, 1:5, or 1:6
f8 THF/tAmylOH in a ratio (v/v) covered by the range from 6:1 to 1:6, preferably from 4:1 to 1:4, more preferably from 3:1 to 1:3, further more preferably from 1:1 to 1:3, and most preferably the ratio is selected from the group consisting of 6:1, 5:1, 4:1, 3:1, 2:1, 1:1, 1:2, 1:3, 1:4, 1:5, or 1:6
f9 THF/tBuOH in a ratio (v/v) covered by the range from 6:1 to 1:6, preferably from 4:1 to 1:4, more preferably from 3:1 to 1:3, further more preferably from 1:1 to 1:3, and most preferably the ratio is selected from the group consisting of 6:1, 5:1, 4:1, 3:1, 2:1, 1:1, 1:2, 1:3, 1:4, 1:5, or 1:6
f10 THF/cyclopentane in a ratio (v/v) covered by the range from 6:1 to 1:6, preferably from 4:1 to 1:4, more preferably from 3:1 to 1:3, further more preferably from 1:1 to 1:3, and most preferably the ratio is selected from the group consisting of 6:1, 5:1, 4:1, 3:1, 2:1, 1:1, 1:2, 1:3, 1:4, 1:5, or 1:6
f11 THF/diethyl ether in a ratio (v/v) covered by the range from 6:1 to 1:6, preferably from 4:1 to 1:4, more preferably from 3:1 to 1:3, further more preferably from 1:1 to 1:3, and most preferably the ratio is selected from the group consisting of 6:1, 5:1, 4:1, 3:1, 2:1, 1:1, 1:2, 1:3, 1:4, 1:5, or 1:6
g) mixtures with Me-THF:
g1 Me-THF/MEK in a ratio (v/v) covered by the range from 6:1 to 1:6, preferably from 4:1 to 1:4, more preferably from 3:1 to 1:3, further more preferably from 1:1 to 1:3, and most preferably the ratio is selected from the group consisting of 6:1, 5:1, 4:1, 3:1, 2:1, 1:1, 1:2, 1:3, 1:4, 1:5, or 1:6
g2 Me-THF/acetone in a ratio (v/v) covered by the range from 6:1 to 1:6, preferably from 4:1 to 1:4, more preferably from 3:1 to 1:3, further more preferably from 1:1 to 1:3, and most preferably the ratio is selected from the group consisting of 6:1, 5:1, 4:1, 3:1, 2:1, 1:1, 1:2, 1:3, 1:4, 1:5, or 1:6
g3 Me-THF/CPME in a ratio (v/v) covered by the range from 6:1 to 1:6, preferably from 4:1 to 1:4, more preferably from 3:1 to 1:3, further more preferably from 1:1 to 1:3, and most preferably the ratio is selected from the group consisting of 6:1, 5:1, 4:1, 3:1, 2:1, 1:1, 1:2, 1:3, 1:4, 1:5, or 1:6
g4 Me-THF/anisole in a ratio (v/v) covered by the range from 6:1 to 1:6, preferably from 4:1 to 1:4, more preferably from 3:1 to 1:3, further more preferably from 1:1 to 1:3, and most preferably the ratio is selected from the group consisting of 6:1, 5:1, 4:1, 3:1, 2:1, 1:1, 1:2, 1:3, 1:4, 1:5, or 1:6
g5 Me-THF/THF in a ratio (v/v) covered by the range from 6:1 to 1:6, preferably from 4:1 to 1:4, more preferably from 3:1 to 1:3, further more preferably from 1:1 to 1:3, and most preferably the ratio is selected from the group consisting of 6:1, 5:1, 4:1, 3:1, 2:1, 1:1, 1:2, 1:3, 1:4, 1:5, or 1:6
g6 Me-THF/MIBK in a ratio (v/v) covered by the range from 6:1 to 1:6, preferably from 4:1 to 1:4, more preferably from 3:1 to 1:3, further more preferably from 1:1 to 1:3, and most preferably the ratio is selected from the group consisting of 6:1, 5:1, 4:1, 3:1, 2:1, 1:1, 1:2, 1:3, 1:4, 1:5, or 1:6
g7 Me-THF/NMP in a ratio (v/v) covered by the range from 6:1 to 1:6, preferably from 4:1 to 1:4, more preferably from 3:1 to 1:3, further more preferably from 1:1 to 1:3, and most preferably the ratio is selected from the group consisting of 6:1, 5:1, 4:1, 3:1, 2:1, 1:1, 1:2, 1:3, 1:4, 1:5, or 1:6
g8 Me-THF/tAmylOH in a ratio (v/v) covered by the range from 6:1 to 1:6, preferably from 4:1 to 1:4, more preferably from 3:1 to 1:3, further more preferably from 1:1 to 1:3, and most preferably the ratio is selected from the group consisting of 6:1, 5:1, 4:1, 3:1, 2:1, 1:1, 1:2, 1:3, 1:4, 1:5, or 1:6
g9 Me-THF/tBuOH in a ratio (v/v) covered by the range from 6:1 to 1:6, preferably from 4:1 to 1:4, more preferably from 3:1 to 1:3, further more preferably from 1:1 to 1:3, and most preferably the ratio is selected from the group consisting of 6:1, 5:1, 4:1, 3:1, 2:1, 1:1, 1:2, 1:3, 1:4, 1:5, or 1:6
g10 Me-THF/cyclopentane in a ratio (v/v) covered by the range from 6:1 to 1:6, preferably from 4:1 to 1:4, more preferably from 3:1 to 1:3, further more preferably from 1:1 to 1:3, and most preferably the ratio is selected from the group consisting of 6:1, 5:1, 4:1, 3:1, 2:1, 1:1, 1:2, 1:3, 1:4, 1:5, or 1:6
g11 Me-THF/diethyl ether in a ratio (v/v) covered by the range from 6:1 to 1:6, preferably from 4:1 to 1:4, more preferably from 3:1 to 1:3, further more preferably from 1:1 to 1:3, and most preferably the ratio is selected from the group consisting of 6:1, 5:1, 4:1, 3:1, 2:1, 1:1, 1:2, 1:3, 1:4, 1:5, or 1:6
h) mixtures with MEK:
h1 MEK/acetone in a ratio (v/v) covered by the range from 6:1 to 1:6, preferably from 4:1 to 1:4, more preferably from 3:1 to 1:3, further more preferably from 1:1 to 1:3, and most preferably the ratio is selected from the group consisting of 6:1, 5:1, 4:1, 3:1, 2:1, 1:1, 1:2, 1:3, 1:4, 1:5, or 1:6
h2 MEK/CPME in a ratio (v/v) covered by the range from 6:1 to 1:6, preferably from 4:1 to 1:4, more preferably from 3:1 to 1:3, further more preferably from 1:1 to 1:3, and most preferably the ratio is selected from the group consisting of 6:1, 5:1, 4:1, 3:1, 2:1, 1:1, 1:2, 1:3, 1:4, 1:5, or 1:6
h3 MEK/anisole in a ratio (v/v) covered by the range from 6:1 to 1:6, preferably from 4:1 to 1:4, more preferably from 3:1 to 1:3, further more preferably from 1:1 to 1:3, and most preferably the ratio is selected from the group consisting of 6:1, 5:1, 4:1, 3:1, 2:1, 1:1, 1:2, 1:3, 1:4, 1:5, or 1:6
h4 MEK/THF in a ratio (v/v) covered by the range from 6:1 to 1:6, preferably from 4:1 to 1:4, more preferably from 3:1 to 1:3, further more preferably from 1:1 to 1:3, and most preferably the ratio is selected from the group consisting of 6:1, 5:1, 4:1, 3:1, 2:1, 1:1, 1:2, 1:3, 1:4, 1:5, or 1:6
h5 MEK/Me-THF in a ratio (v/v) covered by the range from 6:1 to 1:6, preferably from 4:1 to 1:4, more preferably from 3:1 to 1:3, further more preferably from 1:1 to 1:3, and most preferably the ratio is selected from the group consisting of 6:1, 5:1, 4:1, 3:1, 2:1, 1:1, 1:2, 1:3, 1:4, 1:5, or 1:6
h6 MEK/MIBK in a ratio (v/v) covered by the range from 6:1 to 1:6, preferably from 4:1 to 1:4, more preferably from 3:1 to 1:3, further more preferably from 1:1 to 1:3, and most preferably the ratio is selected from the group consisting of 6:1, 5:1, 4:1, 3:1, 2:1, 1:1, 1:2, 1:3, 1:4, 1:5, or 1:6
h7 MEK/NMP in a ratio (v/v) covered by the range from 6:1 to 1:6, preferably from 4:1 to 1:4, more preferably from 3:1 to 1:3, further more preferably from 1:1 to 1:3, and most preferably the ratio is selected from the group consisting of 6:1, 5:1, 4:1, 3:1, 2:1, 1:1, 1:2, 1:3, 1:4, 1:5, or 1:6
h8 MEK/tAmylOH in a ratio (v/v) covered by the range from 6:1 to 1:6, preferably from 4:1 to 1:4, more preferably from 3:1 to 1:3, further more preferably from 1:1 to 1:3, and most preferably the ratio is selected from the group consisting of 6:1, 5:1, 4:1, 3:1, 2:1, 1:1, 1:2, 1:3, 1:4, 1:5, or 1:6
h9 MEK/tBuOH in a ratio (v/v) covered by the range from 6:1 to 1:6, preferably from 4:1 to 1:4, more preferably from 3:1 to 1:3, further more preferably from 1:1 to 1:3, and most preferably the ratio is selected from the group consisting of 6:1, 5:1, 4:1, 3:1, 2:1, 1:1, 1:2, 1:3, 1:4, 1:5, or 1:6
h10 MEK/cyclopentane in a ratio (v/v) covered by the range from 6:1 to 1:6, preferably from 4:1 to 1:4, more preferably from 3:1 to 1:3, further more preferably from 1:1 to 1:3, and most preferably the ratio is selected from the group consisting of 6:1, 5:1, 4:1, 3:1, 2:1, 1:1, 1:2, 1:3, 1:4, 1:5, or 1:6
h11 MEK/diethyl ether in a ratio (v/v) covered by the range from 6:1 to 1:6, preferably from 4:1 to 1:4, more preferably from 3:1 to 1:3, further more preferably from 1:1 to 1:3, and most preferably the ratio is selected from the group consisting of 6:1, 5:1, 4:1, 3:1, 2:1, 1:1, 1:2, 1:3, 1:4, 1:5, or 1:6
i) mixtures with EtOAc:
i1 EtOAc/MEK in a ratio (v/v) covered by the range from 6:1 to 1:6, preferably from 4:1 to 1:4, more preferably from 3:1 to 1:3, further more preferably from 1:1 to 1:3, and most preferably the ratio is selected from the group consisting of 6:1, 5:1, 4:1, 3:1, 2:1, 1:1, 1:2, 1:3, 1:4, 1:5, or 1:6
i2 EtOAc/acetone in a ratio (v/v) covered by the range from 6:1 to 1:6, preferably from 4:1 to 1:4, more preferably from 3:1 to 1:3, further more preferably from 1:1 to 1:3, and most preferably the ratio is selected from the group consisting of 6:1, 5:1, 4:1, 3:1, 2:1, 1:1, 1:2, 1:3, 1:4, 1:5, or 1:6
i3 EtOAc/CPME in a ratio (v/v) covered by the range from 6:1 to 1:6, preferably from 4:1 to 1:4, more preferably from 3:1 to 1:3, further more preferably from 1:1 to 1:3, and most preferably the ratio is selected from the group consisting of 6:1, 5:1, 4:1, 3:1, 2:1, 1:1, 1:2, 1:3, 1:4, 1:5, or 1:6
i4 EtOAc/anisole in a ratio (v/v) covered by the range from 6:1 to 1:6, preferably from 4:1 to 1:4, more preferably from 3:1 to 1:3, further more preferably from 1:1 to 1:3, and most preferably the ratio is selected from the group consisting of 6:1, 5:1, 4:1, 3:1, 2:1, 1:1, 1:2, 1:3, 1:4, 1:5, or 1:6
i5 EtOAc/THF in a ratio (v/v) covered by the range from 6:1 to 1:6, preferably from 4:1 to 1:4, more preferably from 3:1 to 1:3, further more preferably from 1:1 to 1:3, and most preferably the ratio is selected from the group consisting of 6:1, 5:1, 4:1, 3:1, 2:1, 1:1, 1:2, 1:3, 1:4, 1:5, or 1:6
i6 EtOAc/Me-THF in a ratio (v/v) covered by the range from 6:1 to 1:6, preferably from 4:1 to 1:4, more preferably from 3:1 to 1:3, further more preferably from 1:1 to 1:3, and most preferably the ratio is selected from the group consisting of 6:1, 5:1, 4:1, 3:1, 2:1, 1:1, 1:2, 1:3, 1:4, 1:5, or 1:6
i7 EtOAc/MIBK in a ratio (v/v) covered by the range from 6:1 to 1:6, preferably from 4:1 to 1:4, more preferably from 3:1 to 1:3, further more preferably from 1:1 to 1:3, and most preferably the ratio is selected from the group consisting of 6:1, 5:1, 4:1, 3:1, 2:1, 1:1, 1:2, 1:3, 1:4, 1:5, or 1:6
i8 EtOAc/NMP in a ratio (v/v) covered by the range from 6:1 to 1:6, preferably from 4:1 to 1:4, more preferably from 3:1 to 1:3, further more preferably from 1:1 to 1:3, and most preferably the ratio is selected from the group consisting of 6:1, 5:1, 4:1, 3:1, 2:1, 1:1, 1:2, 1:3, 1:4, 1:5, or 1:6
i9 EtOAc/tAmylOH in a ratio (v/v) covered by the range from 6:1 to 1:6, preferably from 4:1 to 1:4, more preferably from 3:1 to 1:3, further more preferably from 1:1 to 1:3, and most preferably the ratio is selected from the group consisting of 6:1, 5:1, 4:1, 3:1, 2:1, 1:1, 1:2, 1:3, 1:4, 1:5, or 1:6
i10 EtOAc/tBuOH in a ratio (v/v) covered by the range from 6:1 to 1:6, preferably from 4:1 to 1:4, more preferably from 3:1 to 1:3, further more preferably from 1:1 to 1:3, and most preferably the ratio is selected from the group consisting of 6:1, 5:1, 4:1, 3:1, 2:1, 1:1, 1:2, 1:3, 1:4, 1:5, or 1:6
i11 EtOAc/cyclopentane in a ratio (v/v) covered by the range from 6:1 to 1:6, preferably from 4:1 to 1:4, more preferably from 3:1 to 1:3, further more preferably from 1:1 to 1:3, and most preferably the ratio is selected from the group consisting of 6:1, 5:1, 4:1, 3:1, 2:1, 1:1, 1:2, 1:3, 1:4, 1:5, or 1:6
i12 EtOAc/diethyl ether in a ratio (v/v) covered by the range from 6:1 to 1:6, preferably from 4:1 to 1:4, more preferably from 3:1 to 1:3, further more preferably from 1:1 to 1:3, and most preferably the ratio is selected from the group consisting of 6:1, 5:1, 4:1, 3:1, 2:1, 1:1, 1:2, 1:3, 1:4, 1:5, or 1:6
j) mixtures with 1,4-dioxane:
j1 1,4-dioxane/MEK in a ratio (v/v) covered by the range from 6:1 to 1:6, preferably from 4:1 to 1:4, more preferably from 3:1 to 1:3, further more preferably from 1:1 to 1:3, and most preferably the ratio is selected from the group consisting of 6:1, 5:1, 4:1, 3:1, 2:1, 1:1, 1:2, 1:3, 1:4, 1:5, or 1:6
j2 1,4-dioxane/acetone in a ratio (v/v) covered by the range from 6:1 to 1:6, preferably from 4:1 to 1:4, more preferably from 3:1 to 1:3, further more preferably from 1:1 to 1:3, and most preferably the ratio is selected from the group consisting of 6:1, 5:1, 4:1, 3:1, 2:1, 1:1, 1:2, 1:3, 1:4, 1:5, or 1:6
j3 1,4-dioxane/CPME in a ratio (v/v) covered by the range from 6:1 to 1:6, preferably from 4:1 to 1:4, more preferably from 3:1 to 1:3, further more preferably from 1:1 to 1:3, and most preferably the ratio is selected from the group consisting of 6:1, 5:1, 4:1, 3:1, 2:1, 1:1, 1:2, 1:3, 1:4, 1:5, or 1:6
j4 1,4-dioxane/anisole in a ratio (v/v) covered by the range from 6:1 to 1:6, preferably from 4:1 to 1:4, more preferably from 3:1 to 1:3, further more preferably from 1:1 to 1:3, and most preferably the ratio is selected from the group consisting of 6:1, 5:1, 4:1, 3:1, 2:1, 1:1, 1:2, 1:3, 1:4, 1:5, or 1:6
j5 1,4-dioxane/THF in a ratio (v/v) covered by the range from 6:1 to 1:6, preferably from 4:1 to 1:4, more preferably from 3:1 to 1:3, further more preferably from 1:1 to 1:3, and most preferably the ratio is selected from the group consisting of 6:1, 5:1, 4:1, 3:1, 2:1, 1:1, 1:2, 1:3, 1:4, 1:5, or 1:6
j6 1,4-dioxane/Me-THF in a ratio (v/v) covered by the range from 6:1 to 1:6, preferably from 4:1 to 1:4, more preferably from 3:1 to 1:3, further more preferably from 1:1 to 1:3, and most preferably the ratio is selected from the group consisting of 6:1, 5:1, 4:1, 3:1, 2:1, 1:1, 1:2, 1:3, 1:4, 1:5, or 1:6
j7 1,4-dioxane/MIBK in a ratio (v/v) covered by the range from 6:1 to 1:6, preferably from 4:1 to 1:4, more preferably from 3:1 to 1:3, further more preferably from 1:1 to 1:3, and most preferably the ratio is selected from the group consisting of 6:1, 5:1, 4:1, 3:1, 2:1, 1:1, 1:2, 1:3, 1:4, 1:5, or 1:6
j8 1,4-dioxane/NMP in a ratio (v/v) covered by the range from 6:1 to 1:6, preferably from 4:1 to 1:4, more preferably from 3:1 to 1:3, further more preferably from 1:1 to 1:3, and most preferably the ratio is selected from the group consisting of 6:1, 5:1, 4:1, 3:1, 2:1, 1:1, 1:2, 1:3, 1:4, 1:5, or 1:6
j9 1,4-dioxane/tAmylOH in a ratio (v/v) covered by the range from 6:1 to 1:6, preferably from 4:1 to 1:4, more preferably from 3:1 to 1:3, further more preferably from 1:1 to 1:3, and most preferably the ratio is selected from the group consisting of 6:1, 5:1, 4:1, 3:1, 2:1, 1:1, 1:2, 1:3, 1:4, 1:5, or 1:6
j10 1,4-dioxane/tBuOH in a ratio (v/v) covered by the range from 6:1 to 1:6, preferably from 4:1 to 1:4, more preferably from 3:1 to 1:3, further more preferably from 1:1 to 1:3, and most preferably the ratio is selected from the group consisting of 6:1, 5:1, 4:1, 3:1, 2:1, 1:1, 1:2, 1:3, 1:4, 1:5, or 1:6
j11 1,4-dioxane/cyclopentane in a ratio (v/v) covered by the range from 6:1 to 1:6, preferably from 4:1 to 1:4, more preferably from 3:1 to 1:3, further more preferably from 1:1 to 1:3, and most preferably the ratio is selected from the group consisting of 6:1, 5:1, 4:1, 3:1, 2:1, 1:1, 1:2, 1:3, 1:4, 1:5, or 1:6
j12 1,4-dioxane/diethyl ether in a ratio (v/v) covered by the range from 6:1 to 1:6, preferably from 4:1 to 1:4, more preferably from 3:1 to 1:3, further more preferably from 1:1 to 1:3, and most preferably the ratio is selected from the group consisting of 6:1, 5:1, 4:1, 3:1, 2:1, 1:1, 1:2, 1:3, 1:4, 1:5, or 1:6
k) mixtures with *n*-hexane (hex):
k1 Hex/MEK in a ratio (v/v) covered by the range from 6:1 to 1:6, preferably from 4:1 to 1:4, more preferably from 3:1 to 1:3, further more preferably from 1:1 to 1:3, and most preferably the ratio is selected from the group consisting of 6:1, 5:1, 4:1, 3:1, 2:1, 1:1, 1:2, 1:3, 1:4, 1:5, or 1:6
k2 Hex/acetone in a ratio (v/v) covered by the range from 6:1 to 1:6, preferably from 4:1 to 1:4, more preferably from 3:1 to 1:3, further more preferably from 1:1 to 1:3, and most preferably the ratio is selected from the group consisting of 6:1, 5:1, 4:1, 3:1, 2:1, 1:1, 1:2, 1:3, 1:4, 1:5, or 1:6
k3 Hex/CPME in a ratio (v/v) covered by the range from 6:1 to 1:6, preferably from 4:1 to 1:4, more preferably from 3:1 to 1:3, further more preferably from 1:1 to 1:3, and most preferably the ratio is selected from the group consisting of 6:1, 5:1, 4:1, 3:1, 2:1, 1:1, 1:2, 1:3, 1:4, 1:5, or 1:6
k4 Hex/anisole in a ratio (v/v) covered by the range from 6:1 to 1:6, preferably from 4:1 to 1:4, more preferably from 3:1 to 1:3, further more preferably from 1:1 to 1:3, and most preferably the ratio is selected from the group consisting of 6:1, 5:1, 4:1, 3:1, 2:1, 1:1, 1:2, 1:3, 1:4, 1:5, or 1:6
k5 Hex/THF in a ratio (v/v) covered by the range from 6:1 to 1:6, preferably from 4:1 to 1:4, more preferably from 3:1 to 1:3, further more preferably from 1:1 to 1:3, and most preferably the ratio is selected from the group consisting of 6:1, 5:1, 4:1, 3:1, 2:1, 1:1, 1:2, 1:3, 1:4, 1:5, or 1:6
k6 Hex/Me-THF in a ratio (v/v) covered by the range from 6:1 to 1:6, preferably from 4:1 to 1:4, more preferably from 3:1 to 1:3, further more preferably from 1:1 to 1:3, and most preferably the ratio is selected from the group consisting of 6:1, 5:1, 4:1, 3:1, 2:1, 1:1, 1:2, 1:3, 1:4, 1:5, or 1:6
k7 Hex/MIBK in a ratio (v/v) covered by the range from 6:1 to 1:6, preferably from 4:1 to 1:4, more preferably from 3:1 to 1:3, further more preferably from 1:1 to 1:3, and most preferably the ratio is selected from the group consisting of 6:1, 5:1, 4:1, 3:1, 2:1, 1:1, 1:2, 1:3, 1:4, 1:5, or 1:6
k8 Hex/NMP in a ratio (v/v) covered by the range from 6:1 to 1:6, preferably from 4:1 to 1:4, more preferably from 3:1 to 1:3, further more preferably from 1:1 to 1:3, and most preferably the ratio is selected from the group consisting of 6:1, 5:1, 4:1, 3:1, 2:1, 1:1, 1:2, 1:3, 1:4, 1:5, or 1:6
k9 Hex/tAmylOH in a ratio (v/v) covered by the range from 6:1 to 1:6, preferably from 4:1 to 1:4, more preferably from 3:1 to 1:3, further more preferably from 1:1 to 1:3, and most preferably the ratio is selected from the group consisting of 6:1, 5:1, 4:1, 3:1, 2:1, 1:1, 1:2, 1:3, 1:4, 1:5, or 1:6
k10 Hex/tBuOH in a ratio (v/v) covered by the range from 6:1 to 1:6, preferably from 4:1 to 1:4, more preferably from 3:1 to 1:3, further more preferably from 1:1 to 1:3, and most preferably the ratio is selected from the group consisting of 6:1, 5:1, 4:1, 3:1, 2:1, 1:1, 1:2, 1:3, 1:4, 1:5, or 1:6
k11 Hex/cyclopentane in a ratio (v/v) covered by the range from 6:1 to 1:6, preferably from 4:1 to 1:4, more preferably from 3:1 to 1:3, further more preferably from 1:1 to 1:3, and most preferably the ratio is selected from the group consisting of 6:1, 5:1, 4:1, 3:1, 2:1, 1:1, 1:2, 1:3, 1:4, 1:5, or 1:6
k12 Hex/diethyl ether in a ratio (v/v) covered by the range from 6:1 to 1:6, preferably from 4:1 to 1:4, more preferably from 3:1 to 1:3, further more preferably from 1:1 to 1:3, and most preferably the ratio is selected from the group consisting of 6:1, 5:1, 4:1, 3:1, 2:1, 1:1, 1:2, 1:3, 1:4, 1:5, or 1:6
l) mixtures with *n*-heptane (Hept):
l1 Hept/MEK in a ratio (v/v) covered by the range from 6:1 to 1:6, preferably from 4:1 to 1:4, more preferably from 3:1 to 1:3, further more preferably from 1:1 to 1:3, and most preferably the ratio is selected from the group consisting of 6:1, 5:1, 4:1, 3:1, 2:1, l:1, 1:2, 1:3, 1:4, 1:5, or 1:6
l2 Hept/acetone in a ratio (v/v) covered by the range from 6:1 to 1:6, preferably from 4:1 to 1:4, more preferably from 3:1 to 1:3, further more preferably from 1:1 to 1:3, and most preferably the ratio is selected from the group consisting of 6:1, 5:1, 4:1, 3:1, 2:1, 1:1, 1:2, 1:3, 1:4, 1:5, or 1:6
l3 Hept/CPME in a ratio (v/v) covered by the range from 6:1 to 1:6, preferably from 4:1 to 1:4, more preferably from 3:1 to 1:3, further more preferably from 1:1 to 1:3, and most preferably the ratio is selected from the group consisting of 6:1, 5:1, 4:1, 3:1, 2:1, 1:1, 1:2, 1:3, 1:4, 1:5, or 1:6
l4 Hept/anisole in a ratio (v/v) covered by the range from 6:1 to 1:6, preferably from 4:1 to 1:4, more preferably from 3:1 to 1:3, further more preferably from 1:1 to 1:3, and most preferably the ratio is selected from the group consisting of 6:1, 5:1, 4:1, 3:1, 2:1, 1:1, 1:2, 1:3, 1:4, 1:5, or 1:6
l5 Hept/THF in a ratio (v/v) covered by the range from 6:1 to 1:6, preferably from 4:1 to 1:4, more preferably from 3:1 to 1:3, further more preferably from 1:1 to 1:3, and most preferably the ratio is selected from the group consisting of 6:1, 5:1, 4:1, 3:1, 2:1, 1:1, 1:2, 1:3, 1:4, 1:5, or 1:6
l6 Hept/Me-THF in a ratio (v/v) covered by the range from 6:1 to 1:6, preferably from 4:1 to 1:4, more preferably from 3:1 to 1:3, further more preferably from 1:1 to 1:3, and most preferably the ratio is selected from the group consisting of 6:1, 5:1, 4:1, 3:1, 2:1, 1:1, 1:2, 1:3, 1:4, 1:5, or 1:6
l7 Hept/MIBK in a ratio (v/v) covered by the range from 6:1 to 1:6, preferably from 4:1 to 1:4, more preferably from 3:1 to 1:3, further more preferably from 1:1 to 1:3, and most preferably the ratio is selected from the group consisting of 6:1, 5:1, 4:1, 3:1, 2:1, 1:1, 1:2, 1:3, 1:4, 1:5, or 1:6
l8 Hept/NMP in a ratio (v/v) covered by the range from 6:1 to 1:6, preferably from 4:1 to 1:4, more preferably from 3:1 to 1:3, further more preferably from 1:1 to 1:3, and most preferably the ratio is selected from the group consisting of 6:1, 5:1, 4:1, 3:1, 2:1, 1:1, 1:2, 1:3, 1:4, 1:5, or 1:6
l9 Hept/tAmylOH in a ratio (v/v) covered by the range from 6:1 to 1:6, preferably from 4:1 to 1:4, more preferably from 3:1 to 1:3, further more preferably from 1:1 to 1:3, and most preferably the ratio is selected from the group consisting of 6:1, 5:1, 4:1, 3:1, 2:1, 1:1, 1:2, 1:3, 1:4, 1:5, or 1:6
l10 Hept/tBuOH in a ratio (v/v) covered by the range from 6:1 to 1:6, preferably from 4:1 to 1:4, more preferably from 3:1 to 1:3, further more preferably from 1:1 to 1:3, and most preferably the ratio is selected from the group consisting of 6:1, 5:1, 4:1, 3:1, 2:1, 1:1, 1:2, 1:3, 1:4, 1:5, or 1:6
l11 Hept/cyclopentane in a ratio (v/v) covered by the range from 6:1 to 1:6, preferably from 4:1 to 1:4, more preferably from 3:1 to 1:3, further more preferably from 1:1 to 1:3, and most preferably the ratio is selected from the group consisting of 6:1, 5:1, 4:1, 3:1, 2:1, 1:1, 1:2, 1:3, 1:4, 1:5, or 1:6
l12 Hept/diethyl ether in a ratio (v/v) covered by the range from 6:1 to 1:6, preferably from 4:1 to 1:4, more preferably from 3:1 to 1:3, further more preferably from 1:1 to 1:3, and most preferably the ratio is selected from the group consisting of 6:1, 5:1, 4:1, 3:1, 2:1, 1:1, 1:2, 1:3, 1:4, 1:5, or 1:6
m) mixtures with *n*-pentane (Pent):
m1 Pent/MEK in a ratio (v/v) covered by the range from 6:1 to 1:6, preferably from 4:1 to 1:4, more preferably from 3:1 to 1:3, further more preferably from 1:1 to 1:3, and most preferably the ratio is selected from the group consisting of 6:1, 5:1, 4:1, 3:1, 2:1, 1:1, 1:2, 1:3, 1:4, 1:5, or 1:6
m2 Pent/acetone in a ratio (v/v) covered by the range from 6:1 to 1:6, preferably from 4:1 to 1:4, more preferably from 3:1 to 1:3, further more preferably from 1:1 to 1:3, and most preferably the ratio is selected from the group consisting of 6:1, 5:1, 4:1, 3:1, 2:1, 1:1, 1:2, 1:3, 1:4, 1:5, or 1:6
m3 Pent/CPME in a ratio (v/v) covered by the range from 6:1 to 1:6, preferably from 4:1 to 1:4, more preferably from 3:1 to 1:3, further more preferably from 1:1 to 1:3, and most preferably the ratio is selected from the group consisting of 6:1, 5:1, 4:1, 3:1, 2:1, 1:1, 1:2, 1:3, 1:4, 1:5, or 1:6
m4 Pent/anisole in a ratio (v/v) covered by the range from 6:1 to 1:6, preferably from 4:1 to 1:4, more preferably from 3:1 to 1:3, further more preferably from 1:1 to 1:3, and most preferably the ratio is selected from the group consisting of 6:1, 5:1, 4:1, 3:1, 2:1, 1:1, 1:2, 1:3, 1:4, 1:5, or 1:6
m5 Pent/THF in a ratio (v/v) covered by the range from 6:1 to 1:6, preferably from 4:1 to 1:4, more preferably from 3:1 to 1:3, further more preferably from 1:1 to 1:3, and most preferably the ratio is selected from the group consisting of 6:1, 5:1, 4:1, 3:1, 2:1, 1:1, 1:2, 1:3, 1:4, 1:5, or 1:6
m6 Pent/Me-THF in a ratio (v/v) covered by the range from 6:1 to 1:6, preferably from 4:1 to 1:4, more preferably from 3:1 to 1:3, further more preferably from 1:1 to 1:3, and most preferably the ratio is selected from the group consisting of 6:1, 5:1, 4:1, 3:1, 2:1, 1:1, 1:2, 1:3, 1:4, 1:5, or 1:6
m7 Pent/MIBK in a ratio (v/v) covered by the range from 6:1 to 1:6, preferably from 4:1 to 1:4, more preferably from 3:1 to 1:3, further more preferably from 1:1 to 1:3, and most preferably the ratio is selected from the group consisting of 6:1, 5:1, 4:1, 3:1, 2:1, 1:1, 1:2, 1:3, 1:4, 1:5, or 1:6
m8 Pent/NMP in a ratio (v/v) covered by the range from 6:1 to 1:6, preferably from 4:1 to 1:4, more preferably from 3:1 to 1:3, further more preferably from 1:1 to 1:3, and most preferably the ratio is selected from the group consisting of 6:1, 5:1, 4:1, 3:1, 2:1, 1:1, 1:2, 1:3, 1:4, 1:5, or 1:6
m9 Pent/tAmylOH in a ratio (v/v) covered by the range from 6:1 to 1:6, preferably from 4:1 to 1:4, more preferably from 3:1 to 1:3, further more preferably from 1:1 to 1:3, and most preferably the ratio is selected from the group consisting of 6:1, 5:1, 4:1, 3:1, 2:1, 1:1, 1:2, 1:3, 1:4, 1:5, or 1:6
m10 Pent/tBuOH in a ratio (v/v) covered by the range from 6:1 to 1:6, preferably from 4:1 to 1:4, more preferably from 3:1 to 1:3, further more preferably from 1:1 to 1:3, and most preferably the ratio is selected from the group consisting of 6:1, 5:1, 4:1, 3:1, 2:1, 1:1, 1:2, 1:3, 1:4, 1:5, or 1:6
m11 Pent/cyclopentane in a ratio (v/v) covered by the range from 6:1 to 1:6, preferably from 4:1 to 1:4, more preferably from 3:1 to 1:3, further more preferably from 1:1 to 1:3, and most preferably the ratio is selected from the group consisting of 6:1, 5:1, 4:1, 3:1, 2:1, 1:1, 1:2, 1:3, 1:4, 1:5, or 1:6
m12 Pent/diethyl ether in a ratio (v/v) covered by the range from 6:1 to 1:6, preferably from 4:1 to 1:4, more preferably from 3:1 to 1:3, further more preferably from 1:1 to 1:3, and most preferably the ratio is selected from the group consisting of 6:1, 5:1, 4:1, 3:1, 2:1, 1:1, 1:2, 1:3, 1:4, 1:5, or 1:6
n) mixtures with isooctane:
n1 Isooctane/MEK in a ratio (v/v) covered by the range from 6:1 to 1:6, preferably from 4:1 to 1:4, more preferably from 3:1 to 1:3, further more preferably from 1:1 to 1:3, and most preferably the ratio is selected from the group consisting of 6:1, 5:1, 4:1, 3:1, 2:1, 1:1, 1:2, 1:3, 1:4, 1:5, or 1:6
n2 Isooctane/acetone in a ratio (v/v) covered by the range from 6:1 to 1:6, preferably from 4:1 to 1:4, more preferably from 3:1 to 1:3, further more preferably from 1:1 to 1:3, and most preferably the ratio is selected from the group consisting of 6:1, 5:1, 4:1, 3:1, 2:1, 1:1, 1:2, 1:3, 1:4, 1:5, or 1:6
n3 Isooctane/CPME in a ratio (v/v) covered by the range from 6:1 to 1:6, preferably from 4:1 to 1:4, more preferably from 3:1 to 1:3, further more preferably from 1:1 to 1:3, and most preferably the ratio is selected from the group consisting of 6:1, 5:1, 4:1, 3:1, 2:1, 1:1, 1:2, 1:3, 1:4, 1:5, or 1:6
n4 Isooctane/anisole in a ratio (v/v) covered by the range from 6:1 to 1:6, preferably from 4:1 to 1:4, more preferably from 3:1 to 1:3, further more preferably from 1:1 to 1:3, and most preferably the ratio is selected from the group consisting of 6:1, 5:1, 4:1, 3:1, 2:1, 1:1, 1:2, 1:3, 1:4, 1:5, or 1:6
n5 Isooctane/THF in a ratio (v/v) covered by the range from 6:1 to 1:6, preferably from 4:1 to 1:4, more preferably from 3:1 to 1:3, further more preferably from 1:1 to 1:3, and most preferably the ratio is selected from the group consisting of 6:1, 5:1, 4:1, 3:1, 2:1, 1:1, 1:2, 1:3, 1:4, 1:5, or 1:6
n6 Isooctane/Me-THF in a ratio (v/v) covered by the range from 6:1 to 1:6, preferably from 4:1 to 1:4, more preferably from 3:1 to 1:3, further more preferably from 1:1 to 1:3, and most preferably the ratio is selected from the group consisting of 6:1, 5:1, 4:1, 3:1, 2:1, 1:1, 1:2, 1:3, 1:4, 1:5, or 1:6
n7 Isooctane/MIBK in a ratio (v/v) covered by the range from 6:1 to 1:6, preferably from 4:1 to 1:4, more preferably from 3:1 to 1:3, further more preferably from 1:1 to 1:3, and most preferably the ratio is selected from the group consisting of 6:1, 5:1, 4:1, 3:1, 2:1, 1:1, 1:2, 1:3, 1:4, 1:5, or 1:6
n8 Isooctane/NMP in a ratio (v/v) covered by the range from 6:1 to 1:6, preferably from 4:1 to 1:4, more preferably from 3:1 to 1:3, further more preferably from 1:1 to 1:3, and most preferably the ratio is selected from the group consisting of 6:1, 5:1, 4:1, 3:1, 2:1, 1:1, 1:2, 1:3, 1:4, 1:5, or 1:6
n9 Isooctane/tAmylOH in a ratio (v/v) covered by the range from 6:1 to 1:6, preferably from 4:1 to 1:4, more preferably from 3:1 to 1:3, further more preferably from 1:1 to 1:3, and most preferably the ratio is selected from the group consisting of 6:1, 5:1, 4:1, 3:1, 2:1, 1:1, 1:2, 1:3, 1:4, 1:5, or 1:6
n10 Isooctane/tBuOH in a ratio (v/v) covered by the range from 6:1 to 1:6, preferably from 4:1 to 1:4, more preferably from 3:1 to 1:3, further more preferably from 1:1 to 1:3, and most preferably the ratio is selected from the group consisting of 6:1, 5:1, 4:1, 3:1, 2:1, 1:1, 1:2, 1:3, 1:4, 1:5, or 1:6
n11 Isooctane/cyclopentane in a ratio (v/v) covered by the range from 6:1 to 1:6, preferably from 4:1 to 1:4, more preferably from 3:1 to 1:3, further more preferably from 1:1 to 1:3, and most preferably the ratio is selected from the group consisting of 6:1, 5:1, 4:1, 3:1, 2:1, 1:1, 1:2, 1:3, 1:4, 1:5, or 1:6
n 12 Isooctane/diethyl ether in a ratio (v/v) covered by the range from 6:1 to 1:6, preferably from 4:1 to 1:4, more preferably from 3:1 to 1:3, further more preferably from 1:1 to 1:3, and most preferably the ratio is selected from the group consisting of 6:1, 5:1, 4:1, 3:1, 2:1, 1:1, 1:2, 1:3, 1:4, 1:5, or 1:6

4. The process of claim 1, wherein the bis-acyl donor compound
(i) is a dicarboxylic acid selected from the group malonic acid, succinic acid, glutaric acid, adipic acid, pimelic acid, suberic acid, azelaic acid, sebacic acid, undecanedioic acid, dodecanedioic acid, 1,12-dodecanedicarboxylic acid, fumaric acid, maleic acid, glutaconic acid, traumatic acid, muconic acid, cis aconitic acid, phthalic acid, isophthalic acid, terephthalic acid, cyclohexan-1,4-dicarboxylic acid, 3-(2-carboxyethyldisulfanyl)propionic acid, diglycolic acid, thio diglycolic acid, 3-(2-carboxyethoxy)propionic acid, 3-[2-(2-carboxyethoxy)ethoxy]propionic acid, 3-{2-[2-(2-carboxyethoxy)ethoxy]ethoxy}propionic acid, 3-(2-{2-[2-(2-carboxy-ethoxy)ethoxy]ethoxy}ethoxy)propionic acid, 3-[2-(2-{2-[2-(2-carboxyethoxy)ethoxy]ethoxy}ethoxy)ethoxy]propionic acid, 3-{2-[2-(2-{2-[2-(2-carboxyethoxy)ethoxy] ethoxy}ethoxy)ethoxy]ethoxy}propionic acid and 3-(2-{2-[2-(2-{2-[2-(2-carboxyethoxy)ethoxy]ethoxy}ethoxy)ethoxy]ethoxy}ethoxy)-propionic acid; or
(ii) is an anhydride selected from the group of succinic anhydride, glutaric anhydride, adipic anhydride, pimelic anhydride, suberic anhydride, maleic anhydride, cis aconitic anhydride, phthalic anhydride, diglycolic anhydride and thio diglycolic anhydride.

5. The process of claim 1, wherein the lipase
(i) is a lipases from microbial sources selected from the group of *Alicaligenes* sp., *Aspergillus, Aspergillus niger, Aspergillus oryzae, Alcaligenes* sp., *Candida antartica A, Candida antartica B, Candida cylindracea* sp., *Candida rugosa, Fusarium solani pisi, Mucor miehei, Mucor javanicus, Penicillium roqueforti, Pseudomonas cepacia, Pseudomonas fluorescens, Pseudomonas* sp., *Pseudomonas stutzeri, Rhizomucor miehei, Rhizopus arrhizus, Rhizopus delemar, Rhizopus niveus, Rhizopus oryzae, Thermomyces lanuginosus, Bacillus subtilis* and *Porcine pancreas;*
(ii) is a genetically modified lipase selected from the group of *Alicaligenes* sp., *Aspergillus, Aspergillus niger, Aspergillus oryzae, Alcaligenes* sp., *Candida antartica A, Candida antartica B, Candida cylindracea* sp., *Candida rugosa, Fusarium solani pisi, Mucor miehei, Mucor javanicus, Penicillium roqueforti, Pseudomonas cepacia, Pseudomonas fluorescens, Pseudomonas* sp., *Pseudomonas stutzeri, Rhizomucor miehei, Rhizopus arrhizus, Rhizopus delemar, Rhizopus niveus, Rhizopus oryzae, Thermomyces lanuginosus, Bacillus subtilis* and *Porcine pancreas;*
wherein the lipase is immobilized to a solid support selected from the group of diatomaceous earth, silica, ceramic, polypropylene, polystyrene or acrylic resins or is immobilized by covalent cross-linking of enzyme aggregates.

6. The process of claim 1, wherein the lipase is added in 1 to 6 weight equivalents (wt. eq.), preferably in 1, 2, 3, 4, 5 or 6 wt. eq. with respect to the *N*-Fmoc-doxorubicin starting material

7. The process of claim 1, wherein the bis-acyl donor is added in 1 to 12 weight equivalents (wt. eq.), preferably 1, 2, 3, 4, 5, 6, 7, 8, 9, 10, 11, or 12 wt. eq. with respect to the *N*-Fmoc-doxorubicin starting material.

8. The process of claim 1, wherein the solvent volume is between 40 and 80 times, preferably 60 or 80 times as high as the weight of the *N*-Fmoc-doxorubicin starting material.

9. The process of claim 1, wherein said *N-*Fmoc-doxorubicin-14-*O*-dicarboxylic acid mono ester compound is *N*-Fmoc-doxorubicin-14-*O*-hemiglutarate,
wherein the lipase is *Candida antartica B* immobilized to a solid acrylic support, added in 4 weight equivalents with respect to the *N*-Fmoc-doxorubicin starting material;
wherein the solvent is a mixture comprising a first organic solvent selected from the group of methyl-*tert*-butyl ether, tetrahydrofuran, 1,4-dioxanee or acetonitrile; and a second organic solvent selected from the group of methyl-ethylketone, acetone, CPME, anisole, 2-methyltetrahydrofuran, or methyl-isobutylketone;
wherein the bis-acyl donor compound is glutaric acid, added in 10 weight equivalents with respect to the Fmoc-doxorubicin starting material;
wherein the ratio between said first solvent and said second solvent is covered by the range from 6:1 to 1:6, preferably from 4:1 to 1:4, more preferably from 3:1 to 1:3, further more preferably from 1:1 to 1:3, and most preferably the ratio is selected from the group consisting of 6:1, 5:1, 4:1, 3:1, 2:1, 1:1, 1:2, 1:3, 1:4, 1:5, or 1:6;
under conditions effective to remove water from the reaction mixture.

10. The process of claim 9, wherein the organic media is a solvent system selected from
a) mixtures with MTBE:
a1 MTBE/MEK in a ratio (v/v) covered by the range from 6:1 to 1:6, preferably from 4:1 to 1:4, more preferably from 3:1 to 1:3, further more preferably from 1:1 to 1:3, and most preferably the ratio is selected from the group consisting of 6:1, 5:1, 4:1, 3:1, 2:1, 1:1, 1:2, 1:3, 1:4, 1:5, or 1:6
a2 MTBE/acetone in a ratio (v/v) covered by the range from 6:1 to 1:6, preferably from 4:1 to 1:4, more preferably from 3:1 to 1:3, further more preferably from 1:1 to 1:3, and most preferably the ratio is selected from the group consisting of 6:1, 5:1, 4:1, 3:1, 2:1, 1:1, 1:2, 1:3, 1:4, 1:5, or 1:6
a3 MTBE/CPME in a ratio (v/v) covered by the range from 6:1 to 1:6, preferably from 4:1 to 1:4, more preferably from 3:1 to 1:3, further more preferably from 1:1 to 1:3, and most preferably the ratio is selected from the group consisting of 6:1, 5:1, 4:1, 3:1, 2:1, 1:1, 1:2, 1:3, 1:4, 1:5, or 1:6
a4 MTBE/anisole in a ratio (v/v) covered by the range from 6:1 to 1:6, preferably from 4:1 to 1:4, more preferably from 3:1 to 1:3, further more preferably from 1:1 to 1:3, and most preferably the ratio is selected from the group consisting of 6:1, 5:1, 4:1, 3:1, 2:1, 1:1, 1:2, 1:3, 1:4, 1:5, or 1:6
a5 MTBE/Me-THF in a ratio (v/v) covered by the range from 6:1 to 1:6, preferably from 4:1 to 1:4, more preferably from 3:1 to 1:3, further more preferably from 1:1 to 1:3, and most preferably the ratio is selected from the group consisting of 6:1, 5:1, 4:1, 3:1, 2:1, 1:1, 1:2, 1:3, 1:4, 1:5, or 1:6
a6 MTBE/MIBK in a ratio (v/v) covered by the range from 6:1 to 1:6, preferably from 4:1 to 1:4, more preferably from 3:1 to 1:3, further more preferably from 1:1 to 1:3, and most preferably the ratio is selected from the group consisting of 6:1, 5:1, 4:1, 3:1, 2:1, 1:1, 1:2, 1:3, 1:4, 1:5, or 1:6
b) mixtures with THF:
b1 THF/MEK in a ratio (v/v) covered by the range from 6:1 to 1:6, preferably from 4:1 to 1:4, more preferably from 3:1 to 1:3, further more preferably from 1:1 to 1:3, and most preferably the ratio is selected from the group consisting of 6:1, 5:1, 4:1, 3:1, 2:1, 1:1, 1:2, 1:3, 1:4, 1:5, or 1:6
b2 THF/acetone in a ratio (v/v) covered by the range from 6:1 to 1:6, preferably from 4:1 to 1:4, more preferably from 3:1 to 1:3, further more preferably from 1:1 to 1:3, and most preferably the ratio is selected from the group consisting of 6:1, 5:1, 4:1, 3:1, 2:1, 1:1, 1:2, 1:3, 1:4, 1:5, or 1:6
b3 THF/CPME in a ratio (v/v) covered by the range from 6:1 to 1:6, preferably from 4:1 to 1:4, more preferably from 3:1 to 1:3, further more preferably from 1:1 to 1:3, and most preferably the ratio is selected from the group consisting of 6:1, 5:1, 4:1, 3:1, 2:1, 1:1, 1:2, 1:3, 1:4, 1:5, or 1:6
b4 THF/anisole in a ratio (v/v) covered by the range from 6:1 to 1:6, preferably from 4:1 to 1:4, more preferably from 3:1 to 1:3, further more preferably from 1:1 to 1:3, and most preferably the ratio is selected from the group consisting of 6:1, 5:1, 4:1, 3:1, 2:1, 1:1, 1:2, 1:3, 1:4, 1:5, or 1:6
b5 THF/Me-THF in a ratio (v/v) covered by the range from 6:1 to 1:6, preferably from 4:1 to 1:4, more preferably from 3:1 to 1:3, further more preferably from 1:1 to 1:3, and most preferably the ratio is selected from the group consisting of 6:1, 5:1, 4:1, 3:1, 2:1, 1:1, 1:2, 1:3, 1:4, 1:5, or 1:6
b6 THF/MIBK in a ratio (v/v) covered by the range from 6:1 to 1:6, preferably from 4:1 to 1:4, more preferably from 3:1 to 1:3, further more preferably from 1:1 to 1:3, and most preferably the ratio is selected from the group consisting of 6:1, 5:1, 4:1, 3:1, 2:1, 1:1, 1:2, 1:3, 1:4, 1:5, or 1:6
c) mixtures with 1,4-dioxane:
c1 1,4-dioxane/MEK in a ratio (v/v) covered by the range from 6:1 to 1:6, preferably from 4:1 to 1:4, more preferably from 3:1 to 1:3, further more preferably from 1:1 to 1:3, and most preferably the ratio is selected from the group consisting of 6:1, 5:1, 4:1, 3:1, 2:1, 1:1, 1:2, 1:3, 1:4, 1:5, or 1:6
c2 1,4-dioxane/acetone in a ratio (v/v) covered by the range from 6:1 to 1:6, preferably from 4:1 to 1:4, more preferably from 3:1 to 1:3, further more preferably from 1:1 to 1:3, and most preferably the ratio is selected from the group consisting of 6:1, 5:1, 4:1, 3:1, 2:1, 1:1, 1:2, 1:3, 1:4, 1:5, or 1:6
c3 1,4-dioxane/CPME in a ratio (v/v) covered by the range from 6:1 to 1:6, preferably from 4:1 to 1:4, more preferably from 3:1 to 1:3, further more preferably from 1:1 to 1:3, and most preferably the ratio is selected from the group consisting of 6:1, 5:1, 4:1, 3:1, 2:1, 1:1, 1:2, 1:3, 1:4, 1:5, or 1:6
c4 1,4-dioxane/anisole in a ratio (v/v) covered by the range from 6:1 to 1:6, preferably from 4:1 to 1:4, more preferably from 3:1 to 1:3, further more preferably from 1:1 to 1:3, and most preferably the ratio is selected from the group consisting of 6:1, 5:1, 4:1, 3:1, 2:1, 1:1, 1:2, 1:3, 1:4, 1:5, or 1:6
c5 1,4-dioxane/Me-THF in a ratio (v/v) covered by the range from 6:1 to 1:6, preferably from 4:1 to 1:4, more preferably from 3:1 to 1:3, further more preferably from 1:1 to 1:3, and most preferably the ratio is selected from the group consisting of 6:1, 5:1, 4:1, 3:1, 2:1, 1:1, 1:2, 1:3, 1:4, 1:5, or 1:6
c6 1,4-dioxane/MIBK in a ratio (v/v) covered by the range from 6:1 to 1:6, preferably from 4:1 to 1:4, more preferably from 3:1 to 1:3, further more preferably from 1:1 to 1:3, and most preferably the ratio is selected from the group consisting of 6:1, 5:1, 4:1, 3:1, 2:1, 1:1, 1:2, 1:3, 1:4, 1:5, or 1:6
d) mixtures with MeCN:
d1 MeCN/MEK in a ratio (v/v) covered by the range from 6:1 to 1:6, preferably from 4:1 to 1:4, more preferably from 3:1 to 1:3, further more preferably from 1:1 to 1:3, and most preferably the ratio is selected from the group consisting of 6:1, 5:1, 4:1, 3:1, 2:1, 1:1, 1:2, 1:3, 1:4, 1:5, or 1:6
d2 MeCN/acetone in a ratio (v/v) covered by the range from 6:1 to 1:6, preferably from 4:1 to 1:4, more preferably from 3:1 to 1:3, further more preferably from 1:1 to 1:3, and most preferably the ratio is selected from the group consisting of 6:1, 5:1, 4:1, 3:1, 2:1, 1:1, 1:2, 1:3, 1:4, 1:5, or 1:6
d3 MeCN/CPME in a ratio (v/v) covered by the range from 6:1 to 1:6, preferably from 4:1 to 1:4, more preferably from 3:1 to 1:3, further more preferably from 1:1 to 1:3, and most preferably the ratio is selected from the group consisting of 6:1, 5:1, 4:1, 3:1, 2:1, 1:1, 1:2, 1:3, 1:4, 1:5, or 1:6
d4 MeCN/anisole in a ratio (v/v) covered by the range from 6:1 to 1:6, preferably from 4:1 to 1:4, more preferably from 3:1 to 1:3, further more preferably from 1:1 to 1:3, and most preferably the ratio is selected from the group consisting of 6:1, 5:1, 4:1, 3:1, 2:1, 1:1, 1:2, 1:3, 1:4, 1:5, or 1:6
d5 MeCN/Me-THF in a ratio (v/v) covered by the range from 6:1 to 1:6, preferably from 4:1 to 1:4, more preferably from 3:1 to 1:3, further more preferably from 1:1 to 1:3, and most preferably the ratio is selected from the group consisting of 6:1, 5:1, 4:1, 3:1, 2:1, 1:1, 1:2, 1:3, 1:4, 1:5, or 1:6
d6 MeCN/MIBK in a ratio (v/v) covered by the range from 6:1 to 1:6, preferably from 4:1 to 1:4, more preferably from 3:1 to 1:3, further more preferably from 1:1 to 1:3, and most preferably the ratio is selected from the group consisting of 6:1, 5:1, 4:1, 3:1, 2:1, 1:1, 1:2, 1:3, 1:4, 1:5, or 1:6.

11. The process of claim 1, wherein said *N-*Fmoc-doxorubicin-14-*O*-dicarboxylic acid mono ester compound is *N*-Fmoc-doxorubicin-14-*O*-hemiglutarate,
wherein the lipase is *Candida antartica B* immobilized to a solid acrylic support, added in 4 weight equivalents with respect to the *N*-Fmoc-doxorubicin starting material;
wherein the bis-acyl donor compound is glutaric acid, added in 10 weight equivalents with respect to the Fmoc-doxorubicin starting material
wherein the solvent is a mixture comprising methyl-*tert*-butyl ether and methyl-ethylketone in a ratio of 3:1;
wherein the solvent volume is 60 times as high as the weight of *N*-Fmoc-doxorubicin;
under conditions effective to remove water from the reaction mixture;
wherein the reaction is completed after 25 h to give an isolated yield of ∼88% (as analyzed by HPLC).

12. The process of claim 1, wherein said *N-*Fmoc-doxorubicin-14-*O*-dicarboxylic acid mono ester compound is *N*-Fmoc-doxorubicin-14-*O*-hemiglutarate,
wherein the lipase is *Candida antartica B* immobilized to a solid acrylic support, added in 4 weight equivalents with respect to the *N*-Fmoc-doxorubicin starting material;
wherein the solvent is a mixture comprising methyl-*tert*-butyl ether and anisole in a ratio of 1:1;
wherein the bis-acyl donor compound is glutaric acid, added in 5 weight equivalents with respect to the *N*-Fmoc-doxorubicin starting material, and glutaric anhydride, added in 5 weight equivalents with respect to the *N*-Fmoc-doxorubicin starting material;
wherein the solvent volume is 60 times as high as the weight of *N*-Fmoc-doxorubicin;
under conditions effective to remove water from the reaction mixture;
wherein the reaction is completed after 25 h to give an isolated yield of ∼88% (as analyzed by HPLC).

13. The process of claim 1, wherein said *N-*Fmoc-doxorubicin-14-*O*-dicarboxylic acid mono ester compound is *N*-Fmoc-doxorubicin-14-*O*-hemiglutarate,
wherein the lipase is *Candida antartica B* immobilized to a solid acrylic support, added in 4 weight equivalents with respect to the *N*-Fmoc-doxorubicin starting material;
wherein the solvent is a mixture comprising methyl-*tert*-butyl ether and Me-THF in a ratio of 1:1;
wherein the bis-acyl donor compound is glutaric acid, added in 10 weight equivalents with respect to the *N*-Fmoc-doxorubicin starting material;
wherein the solvent volume is 60 times as high as the weight of *N*-Fmoc-doxorubicin;
under conditions effective to remove water from the reaction mixture;
wherein the reaction is completed after 4 h to give an isolated yield of ∼74%.

14. The process of claim 1, wherein said *N-*Fmoc-doxorubicin-14-*O*-dicarboxylic acid mono ester compound is *N*-Fmoc-doxorubicin-14-*O*-hemiglutarate,
wherein the lipase is *Candida antartica B* immobilized to a solid acrylic support, added in 4 weight equivalents with respect to the *N*-Fmoc-doxorubicin starting material;
wherein the bis-acyl donor compound is glutaric acid, added in 10 weight equivalents with respect to the *N*-Fmoc-doxorubicin starting material;
wherein the solvent is a mixture comprising methyl-*tert*-butyl ether and Me-THF in a ratio of from 6:1 to 1:6, preferably from 4:1 to 1:4;
wherein the solvent volume is 60 times as high as the weight of Fmox-doxorubicin;
under conditions effective to remove water from the reaction mixture.

15. The process of claim 1, wherein said *N-*Fmoc-doxorubicin-14-*O*-dicarboxylic acid mono ester compound is *N*-Fmoc-doxorubicin-14-*O*-hemiglutarate,
wherein the lipase is *Candida antartica B* immobilized to a solid acrylic support, added in 4 weight equivalents with respect to the *N*-Fmoc-doxorubicin starting material;
wherein the bis-acyl donor compound is glutaric acid, added in 10 weight equivalents with respect to the *N*-Fmoc-doxorubicin starting material
wherein the solvent is a mixture comprising methyl-*tert*-butyl ether and Me-THF in a ratio of from 6:1 to 1:6, preferably from 3:1 to 1:3, more preferably from 1:1 to 1:3, further more preferred from 1:1,5 to 1:2,5 and most preferred 1:2;
wherein the solvent volume is 60 times as high as the weight of *N*-Fmoc-doxorubicin;
under conditions effective to remove water from the reaction mixture.

16. The process of claim 1, wherein said *N-*Fmoc-doxorubicin-14-*O*-dicarboxylic acid mono ester compound is *N*-Fmoc-doxorubicin-14-*O*-hemiglutarate,
wherein the lipase is *Candida antartica B* immobilized to a solid acrylic support, added in 4 weight equivalents with respect to the *N*-Fmoc-doxorubicin starting material;
wherein the solvent is a mixture comprising methyl-*tert*-butyl ether and Me-THF in a ratio of 1:2;
wherein the bis-acyl donor compound is glutaric acid, added in 10 weight equivalents with respect to the Fmoc-doxorubicin starting material;
wherein the solvent volume is 60 times as high as the weight of *N*-Fmoc-doxorubicin;
under conditions effective to remove water from the reaction mixture;
wherein the reaction is completed after 4 h to give an isolated yield of ∼91%.

17. The process of claim 1, wherein said *N*-Fmoc-doxorubicin-14-*O*-dicarboxylic acid mono ester compound is *N*-Fmoc-doxorubicin-*O*-hemisuccinate, *N*-Fmoc-doxorubicin-*O*-hemipimelate, *N-*Fmoc-doxorubicin-*O*-hemifumarate, *N-*Fmoc-doxorubicin-*O-*hemi-3-(2-carboxyethoxy)propionate, *N-*Fmoc-doxorubicin-*O*-hemiadipate, *N-*Fmoc-doxorubicin-O-hemisuberate, *N-*Fmoc-doxorubicin-*O*-hemiazelate, *N-*Fmoc-doxorubicin-O-hemisebacate, *N-*Fmoc-doxorubicin-*O*-hemiundecanedioate, *N-*Fmoc-doxorubicin-O-hemidodecanedioate, *N-*Fmoc-doxorubicin-*O*-hemimaleate, *N-*Fmoc-doxorubicin-O-hemiterephthalate, or *N*-Fmoc-doxorubicin-O-hemi-(2-carboxyethyldisulfanyl)propionate;
wherein the lipase is *Candida antartica B* immobilized to a solid acrylic support, added in 4 weight equivalents with respect to the *N*-Fmoc-doxorubicin starting material;
wherein the solvent is a mixture comprising a first organic solvent selected from the group of methyl-*tert*-butyl ether, CPME, tetrahydrofuran, 1,4-dioxanee or acetonitrile; and a second organic solvent selected from the group of acetone, methyl-ethylketone, methyl-isobutylketone, anisole, or 2-methyltetrahydrofuran;
wherein the ratio between said first solvent and said second solvent is covered by the range from 6:1 to 1:6, preferably from 4:1 to 1:4, more preferably from 3:1 to 1:3, further more preferably from 1:1 to 1:3, and most preferably may be 1:1, 2:1, 3:1, 4:1; 5:1, 6:1, 1:2, 1:3, 1:4, 1:5, or 1:6;
wherein benzene or toluene or both may be added to remove water from the reaction mixture;
wherein the bis-acyl donor compound is glutaric acid;
wherein said glutaric acid is added in 10 weight equivalents with respect to the *N-*Fmoc-doxorubicin starting material;
wherein the solvent volume is 60 times as high as the weight of *N*-Fmoc-doxorubicin;
under conditions effective to remove water from the reaction mixture.
